# EUROPEAN PATENT APPLICATION

(11) **EP 2 223 929 A1**
(43) Date of publication of application: **01.09.2010**
(21) Application number: 08801009.5
(22) Date of filing: 26.09.2008
(51) Int. Cl.: C07H 17/04, A61K 31/7048, A61P 35/00

(54) **GAMBOGIC GLYCOSIDE DERIVATIVES AND ANALOGS, THE PREPARATION AND THE APPLICATION THEREOF**

(30) Priority: 29.09.2007 CN 200710157223
(71) Applicant: Liaoning Lifeng Scientific&Technology Development Company Ltd., Huanggu District Shenyang, Liaoning 110036 (CN)
(72) Inventor: XU, Lifeng, Shenyang Liaoning 110036 (CN)
(74) Representative: Lambrinos, Matthew Franklin
(86) International application number: PCT/CN2008/072540
(87) International publication number: WO 2009/043296

(57) **Abstract**

This invention relates with the gambogic acid glycoside derivatives and analogs of formula I: 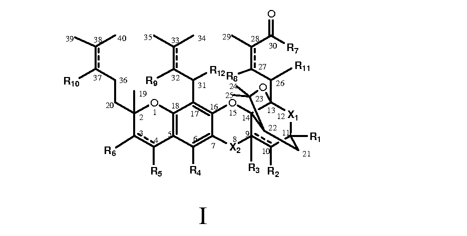 or stereoisomers, tautoers, prodrugs, pharmaceutically acceptable salts, complex salts or solvates thereof, wherein:
X₁, X₂, R₁, R₂, R₃, R₄, R₅, R₆, R₇, R₈, R₉, R₁₀, R₁₁ or/and R₁₂ is, independently at each occurrence, optionally substituted glycosyl, optionally substituted multi-hydroxyl, optional substituent oxy, optional substituent containing carbon, oxygen, sulfur, nitrogen or phosphorus element. Compounds of the present invention are useful as therapeutically effective agents of anti-cancer, anti-virus and anti-bacterial.

This invention also relates with their preparative methods and applications.

## Description

### THE FIELD OF INVENTION

This invention relates with anti-tumor activities, medicinal chemistry research and preparative methods of new gambogic acid glycoside derivatives and analogs thereof. The invention also relates with the medication applications of anti-tumor and other diseases by this kind of compounds.

### BACKGROUND OF THE INVENTION

This invention is in the field of medicinal chemistry. In particular, the invention relates with gambogic acid derivatives and analogs, and the discovery of these compounds is therapeutically effective anti-cancer agents.

### DESCRIPTION OF BACKGROUND ART

Medicinal plant Gamboge is a gel resin from trees of Hai teng, Yu huang, Yue huang and La huang, in India, Thailand, the islands of Southeast Asia, Cambodia, Thailand, Viet Nam and China (Ref. **1:** Wang Ming, Feng Xu, Zhao Youyi, Fu Hui, Studies and Application of Gamboge, Chinese Medicine Research and Chinese Wild Plant Resources, 2003, 22 (1), 1-3; Ref. **2:** Wenmember Liu, Feng Feng, Yousheng Chen, Qi-Dong You, Shou Xun Zhao, The Study on the structures of gambogic acid and alkaline degradation products, Chinese Natural Medicine Resource, 2004, 2 (2), 75-77).

Gambogic resin contains gambogic acid 23% - 37%, neogambogic acid, allogambogic acid and other ingredients. In 1965 W. Dollis confirmed the gambogic acid structure from the extract of Gamboge (Ref. **3:** Jun A, Kazuhiro C B, Masahiro T.D. et al., 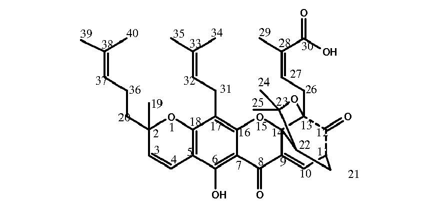 Cytotoxic xanthones from gamboge, J. Phytochemistry, 1996, 41(3):815-820; Ref. **4:** Lin L. J, Lin L.Z. John M.P. et al, Isogambogic acid and isomorellinol from gamboge hanburyi, Magn Reson Chem, 1993, 31: 340-347; Ref. **5:** Cao, S.G. Valerie, H S, Wu X., et al, Novel cytotoxic multi-prenylated xanthonoids from gamboge gaudichaudi (Guttiferae). Tetrahedron, 1998, 54: 10915-10924; Ref. **6:** Cao, S.G. Wu X., Sim KY, et al, Cytotoxic caged tetraprenylated xanthonoids from gambogyl gaudichaudi (Guttiferae), Tetrahedron, 1998, 39 :3353-3356).

Traditional Chinese Medicines believes that gamboge treats swollen ulcer, ulcers and tumors. Gamboge was widely used in treatment of cancer, sores and folliculitis. (Ref. **7:** Jiangsu New Medical College Compiled, "TCM Dictionary" (second volume) Shanghai Science and Technology Press, 19771, 26951). Anti-cancer effects of gambogic acid was proved by the experiments of Chinese research group (Ref. **8:** Xiang S.R, Chen TK, Huang, Y.C. et al, Effect on tumor S180 and ascites by gambogic acid, J. Acta Acad Med Jiang xi, 1981, (1), 172211).

Gamboge inhibited significantly growth of S₃₇, S₁₈₀, ARA₄, W₂₅₆, ECA and liver ascite tumor strains (Ref. **9:** Xiang Huan Qiu, Shao-Bai Xue, Jiangxi Medicine, 1984, (5), 1-4; Ref. **10:** Qiumo Lei, Jin Mei Liui, Jiangxi and Medicine, 1982, (3), 1-5.13, 20; Ref. **11:** Hongyan Gu, Qing Long Guo, Chinese natural medicines, 2005, 3 (3), 168-172). The alcohol extract showed inhibition effect of reticular cell sarcoma of mice (Ref. **12:** Qiu yuxing, Effect of 7361 on reticular cell sarcoma of mice, Jiangxi and Medicine, 1984, (1), 1-9). Recent study showed the effect of gambogic acid on pancreatic cancer cells (Qidong You, et al, Chinese patent CN1309125A).

Qidong You, et al. made gambogic acid salt as an anticancer agent with water soluble complex, Jin Biao, et al (Ref. **13:** Jin Biao, Dong Hui, Qiao-lin, Gambogic acids complexes as active ingredients and their preparation, Chinese patent application (CN1390839A). Shu Long, Wang reported a prodrug of gambogic acid reacted with multi-ethyleneglycol (Ref. **14:** Shulong, Wang, A new gambogic acid derivative, Chinese Patent Application CN 1563014A). While Wen-Hu, Duan, et al, reported the structural modifications of C-4 and C-30 of gambogic acid [CN 1715283].

The patents, WO 06/44216, US7176,234, US 7138,620, US 7138,428, US 6613,762, US 6462,041, US 2005/00040206, US 2004/0082066, US 2003/0078292 and US 2002/0076733 reported the structural modification of gambogic acid, including chemical synthesis, preparation and study of anti-tumor activity at sites of C-10 and C-30.

To date there has been no report related with structural modification of gambogic acid with glycosyl, multi-hydroxyl, amino acids, phosphate, acyloxy at C-6 or C-11 site to form gambogic acid glycoside derivatives and analogs, nor the introduction of polar substituents to solve the poor water-soluble, low bioavailability and low anti-tumor activity problems of gambogic acid from all literature reviewed.

### BRIEF DESCRIPTION OF TH DRAWINGS

Figure. 1 is a representation of the inhibition effects of colorectal cancer cell, HT₂₉ (Example compounds of 3, 18, 25, 27, cisplatin and 5-Fu).

Figure. 2 is a representation of the inhibition effects of colorectal cancer cell, HT₂₉ (Example compounds of 3, 18, 25, 27 and cisplatin).

Figure. 3 is a representation of the inhibition effects of pancreatic cancer cell, Panc-1 (Example compounds of 3, 18, 25, 27 and cisplatin).

Figure. 4 is a representation of the inhibition effects of lung cancer cell, NIH-H₄₆₀. (Example compounds of 3, 18, 25, 27, cisplatin and 5-Fu).

Figure. 5-1 is a representation of the long-term toxicity of control by heart biopsy examination.

Figure. 5-2 is a representation of the long-term toxicity of compound 26 (8 mg /kg) by heart biopsy examination.

Figure. 5-3 is a representation of the long-term toxicity of compound 26 (4 mg /kg) by heart biopsy examination.

Figure. 5-4 is a representation of the long-term toxicity of control by liver biopsy examination.

Figure. 5-5 is a representation of the long-term toxicity of compound 26 (8 mg /kg) by liver biopsy examination.

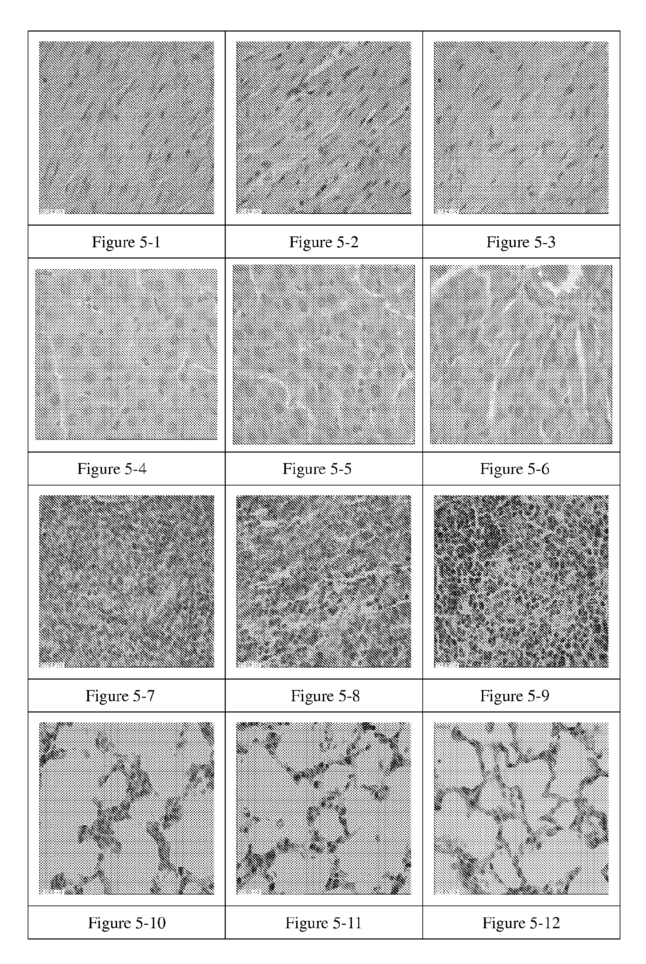

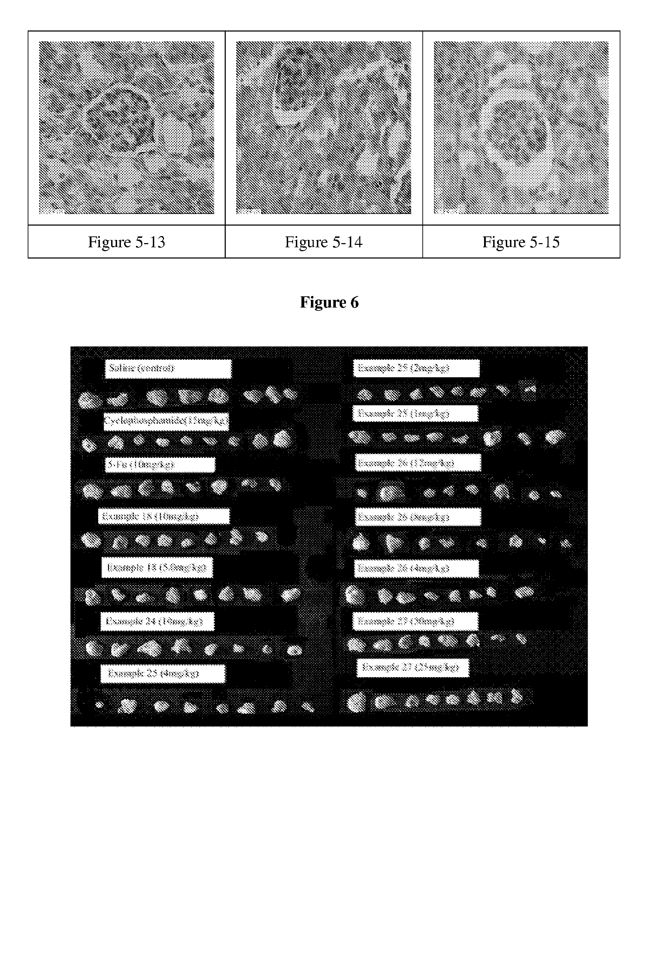

Figure. 5-6 is a representation of the long-term toxicity of compound 26 (4 mg /kg) by liver biopsy examination.

Figure. 5-7 is a representation of the long-term toxicity of control by spleen biopsy examination.

Figure. 5-8 is a representation of the long-term toxicity of compound 26 (8 mg /kg) by spleen biopsy examination.

Figure. 5-9 is a representation of the long-term toxicity of compound 26 (4 mg /kg) by spleen biopsy examination.

Figure. 5-10 is a representation of the long-term toxicity of control by lung biopsy examination.

Figure. 5-11 is a representation of the long-term toxicity of compound 26 (8 mg /kg) by lung biopsy examination.

Figure. 5-12 is a representation of the long-term toxicity of compound 26 (4 mg /kg) by lung biopsy examination.

Figure. 5-13 is a representation of the long-term toxicity of control by renal biopsy examination.

Figure. 5-14 is a representation of the long-term toxicity of compound 26 (8 mg /kg) by renal biopsy examination.

Figure. 5-15 is a representation of the long-term toxicity of compound 26 (4 mg /kg) by renal biopsy examination.

Figure. 6 is a representation of the inhibition of tumor S180 (In vivo, Example compounds of 18, 24, 25, 26, 27, cisplatin and 5-Fu).

### SUMMARY OF THE INVENTION

This invention relates with the gambogic acid glycoside derivatives and analogs of formula I, 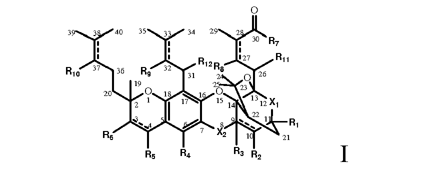 or stereoisomers, tautoers, prodrugs, pharmaceutically acceptable salts, complex salts or solvates thereof, wherein:

R₁, R₂, R₃, R₄, R₅, R₆, R₇, R₈, R₉, R₁₀, R₁₁ or/and R₁₂ is, independently at each occurrence, optionally substituted glycosyl, optionally substituted multi-hydroxyl, optional substituent oxy, optional substituent containing oxygen, sulfur, nitrogen or phosphorus element;

Glycosyl is D- or L-configuration and its glycoside bond is C-C or C-hetero bond connection, including 1-8 optionally substituted glycosyl or optional substituent glycosyl;

Multi-hydroxyl is, independently at each occurrence, 1-10 optionally substituted hydroxyl group of alkyl, aryl, cyclic or heterocyclic, where contains optionally substituted one or combination of amino acid, acyloxy, phosphoric acid oxy, alkoxy, alkyl, alicyclic, aryl ring, aliphatic heterocyclic or aryl heterocyclic;

Substituent oxy is, independently at each occurrence, optionally substituted acyloxy, 1-4 optionally substituted phosphoryloxy, optionally substituted alkoxy, optionally substituted aryloxy or optionally substituted heterocyclic oxy;

Substituent containing oxygen, sulfur, nitrogen or phosphorus element is, independently at each occurrence, optionally substituted saturated, optionally substituted unsaturated C₁₋₁₀ alkyl, 1-4 optionally substituted double bond, optionally substituted triple bond, optional substituent of saturated or unsaturated C₁₋₁₀ alicyclic, arylcyclic and heterocyclic group, where contains cyclic one or combination of oxygen, sulfur, nitrogen or phosphorus element, saturated or unsaturated 3-10 membered alicyclic, aryl cyclic, multi-cyclic, aliphatic heterocyclic, aryl heterocyclic or fused heterocyclic;

Substituent is, independently at each occurrence, optionally substituted one or combination of saturated or unsaturated C₁₋₁₀ alkyl, 1-4 double bond, 1-4 triple bond, saturated or unsaturated C₁₋₁₀ alicyclic, C₁₋₁₀ aryl and C₁₋₁₀ heterocyclic group;

R₄ is, independently at each occurrence, optional substituent of 1-8 glycosyl, multi-hydroxyl, substituted multi-hydroxyl, 1-5 amino acid, 1-4 phosphate, acyloxy, phosphoric, sulfonyloxy, alkoxy, aryloxy, heterocyclic oxy, alkyl, alicyclic, aryl cyclic, aliphatic heterocyclic or aryl heterocyclic containing oxygen, sulfur, nitrogen or phosphorus element.

This invention is to provide for the use of these novel compounds for treating, preventing or slowing the progression of neoplasia and cancer, and infectious diseases by virus, bacterial or fungi, wherein said compounds is administered together with at least one known cancer chemotherapeutic agent. The administration may be by oral route, parenteral, subcutaneous, intravenous, intramuscular, intra-peritoneal, transdermal, buccal, intrathecal, intracranial, intranasal or topical routes.

This invention also relates with their preparative methods and applications.

### DETAILED DESCRIPTION OF THE INVENTION

This invention relates with the gambogic acid glycoside derivatives and analogs of formula I, 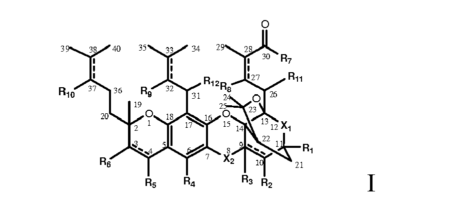 or stereoisomers, tautoers, prodrug, pharmaceutically acceptable salts, complex salts or solvates thereof, wherein:

The dotted lines are optionally substituted single bonds, optionally substituted double bond or a optionally substituted heterocyclic group containing carbon, oxygen, sulfur or nitrogen element;

R₁, R₂, R₃, R₄, R₅, R₆, R₇, R₈, R₉, R₁₀, R₁₁ or/and R₁₂ is, independently at each occurrence, optionally substituted glycosyl, optionally substituted multi-hydroxyl, optional substituent oxy, optional substituent containing oxygen, sulfur, nitrogen or phosphorus element;

Glycosyl is D- or L-configuration and its glycoside bond is C-C or C-hetero bond connection, including 1-8 optionally substituted glycosyl or optional substituent glycosyl;

Multi-hydroxyl is, independently at each occurrence, 1-10 optionally substituted hydroxyl group of alkyl, aryl, cyclic or heterocyclic, where contains optionally substituted one or combination of amino acid, acyloxy, phosphoric acid oxy, alkoxy, alkyl, alicyclic, aryl ring, aliphatic heterocyclic or aryl heterocyclic;

Substituent oxy is, independently at each occurrence, optionally substituted acyloxy, 1-4 optionally substituted phosphoryloxy, optionally substituted alkoxy, optionally substituted aryloxy or optionally substituted heterocyclic oxy;

Substituent containing oxygen, sulfur, nitrogen or phosphorus element is, independently at each occurrence, optionally substituted saturated, optionally substituted unsaturated C₁₋₁₀ alkyl, 1-4 optionally substituted double bond, optionally substituted 1-4 triple bond, optional substituent of saturated or unsaturated C₁₋₁₀ alicyclic, arylcyclic and heterocyclic group, where contains cyclic one or combination of oxygen, sulfur, nitrogen or phosphorus element, saturated or unsaturated 3-10 membered alicyclic, aryl cyclic, multi-cyclic, aliphatic heterocyclic, aryl heterocyclic or fused heterocyclic;

Substituent is, independently at each occurrence, optionally substituted one or combination of saturated or unsaturated C₁₋₁₀ alkyl, 1-4 double bond, 1-4 triple bond, saturated or unsaturated C₁₋₁₀ alicyclic, C₁₋₁₀ aryl and C₁₋₁₀ heterocyclic group; wherein:

X₁ and X₂ are, independently at each occurrence, C = O, C = Rb-Ra, CHOH, CHORb, CHRb or substituent, where Rb contains, independently at each occurrence, C, N or P element; Ra is H, H₂, optionally substituted straight-alkyl, optionally substituted branched-alkyl, C₁₋₁₀ optionally substituted saturated alkyl, optionally substituted 1-4 double bond, optionally substituted 1-4 triple bond, optionally substituted unsaturated alkyl, optionally substituted saturated or unsaturated alicyclic, optionally substituted arylcyclic, optionally substituted aryl or optionally substituted heterocyclic, where contains hydroxyl, halogen, oxygen, nitrogen, sulfur or phosphorus element;
wherein:

Substituent is, independently at each occurrence, C₁₋₁₀ optionally substituted saturated or unsaturated alkyl, 1-4 optionally substituted double bond, 1-4 optionally substituted triple bond, optionally substituted or saturated, unsaturated C₁₋₁₀ alicyclic, C₁₋₁₀ optionally substituted aryl group or C₁₋₁₀ optionally substituted heterocyclic, where contains optionally substituted one or combination of oxygen, sulfur, nitrogen, phosphorus element, halogen, saturated or unsaturated 3-10 membered alicyclic, aryl cyclic, multi-cyclic, aliphatic heterocyclic, aryl heterocyclic, fused heterocyclic, 1-8 glycosyl, substituent glycosyl, amino acid, acyloxy, phosphoryloxy, alkoxy, heterocyclic oxy and multi-hydroxyl of alkyl, ring, aryl or heterocyclic.

A compound according to the gambogic acid glycoside derivatives and analogs of formula I, wherein:

R₁, R₂, R₅, R₆, R₈, R₉, R₁₀, R₁₁ or/and R₁₂ is H, halogen or XRa; where XRa is unsubstituted or substituted group containing C, O, S, Se, N, and / or the P element.

A compound according to the gambogic acid glycoside derivatives and analogs of formula I, wherein:

R₃ is XaRa electrophilic substituent, where Xa is unsubstituted or substituted group containing C, S, P, and / or Si element.

A compound according to the gambogic acid glycoside derivatives and analogs of formula I, wherein:

R₄ is, independently at each occurrence, optional substituent of 1-8 glycosyl, multi-hydroxyl, substituted multi-hydroxyl, 1-5 amino acid, 1-4 phosphate, acyloxy, phosphoric, sulfonyloxy, alkoxy, aryloxy, heterocyclic oxy, alkyl, alicyclic, aryl cyclic, aliphatic heterocyclic or aryl heterocyclic containing oxygen, sulfur, nitrogen or phosphorus element, where glycosyl is D-and L-configuration with C-C or C-hetero bond of glycoside;
wherein:

Multi-hydroxyl is, independently at each occurrence, optionally substituted one or combination of 1-20 hydroxyl alkyl of cyclic alkyl, aryl, heterocyclic, amino acid, acryl oxy, phosphoryloxy, alkoxy or heterocyclic oxy, where contains alkyl, alicyclic, arylcyclic, aliphatic heterocyclic or aryl heterocyclic;

Substituted multi-hydroxyl is, independently at each occurrence, optionally substituted multi-hydroxyl substituted by saturated or unsaturated C₁₋₁₀ alkyl, 1-4 double bond or triple bond, saturated or unsaturated C₁₋₁₀ alicyclic, alkyl and aryl, where contains optionally substituted one or combination of oxygen, sulfur, nitrogen, phosphorus element, halogen, amino acid, acyloxy, phosphoryloxy, alkoxy, saturated or unsaturated 3-10 membered alicyclic, aryl cyclic, multi-cyclic, aliphatic heterocyclic, aryl heterocyclic or fused heterocyclic

A compound according to the gambogic acid glycoside derivatives and analogs of formula I, wherein:
1-8 glycosyl is, independently at each occurrence, optionally substituted C₃₋₈ saccharide, optionally substituted monosaccharide, optionally substituted disaccharide, optionally substituted trisaccharide and / or optionally substituted polysaccharide

A compound according to said saccharide, wherein:
C₃₋₈ saccharide, monosaccharide, disaccharide, trisaccharide, and / or polysaccharide is, independently at each occurrence, optionally substituted hydroxyl saccharide, optionally substituted amino saccharide, optionally substituted deoxy saccharide, optionally substituted sulfuric acid saccharide, optionally substituted hetero-element saccharide and / or its glycoside.

A compound according to the gambogic acid glycoside derivatives and analogs of formula I, wherein:

R₇ is H or XbRa; Xb is, independently at each occurrence, optional substituent containing H, C, O or N element.

A compound according to the gambogic acid glycoside derivatives and analogs of formula I, wherein:

When X₁ and / or X₂ is C = O, C = Rb-Ra, CHOH, CHORb or CHRb, X₁ and X₂ are the same or different substituents; when Rb is C, N or P element, Ra is, independently at each occurrence, optionally substituted formation of olefin, alkane, halogenated hydrocarbon, alcohol, ether, oxime, hydrazone or substituted said groups.

A compound according to the gambogic acid glycoside derivatives and analogs of formula I, wherein:

A bromo-compound at 11-position is selected, independently at each occurrence, from: gambogic acid, methyl gambogate, ethyl gambogate, gambogyl morpholine, gambogyl piperidine, gambogyl 4-methyl pyrazine, 9,10-dihydro-10-morpholinyl gambogic acid, ethyl-9,10-dihydro-10-morpholinylgambogate, 9,10-dihydro-10-morpholinyl gambogyl piperidine, methyl-9,10-dihydro-10- (nitro-methane) gambogate, 9,10-dihydro-10-(1-aminopiperidinyl)gambogyl (1-amino piperidine), benzyl alanine-9,10-dihydro-10-(benzyl-L-alaninyl) gambogyl, 9,10-dihydro-10-(N-methyl-1-naphthyl amino)gambogyl (N-methyl-1-naphthalene methylamine);

A compound introduced methyl amino at 11-position is selected, independently at each occurrence, from: gambogic acid, methyl gambogate, ethyl gambogate, gambogyl morpholine, gambogyl piperidine, gambogyl-4-methyl-pyrazine, 9,10-dihydro-10-morpholinyl gambogic acid, methyl-9,10-dihydro-10-morpholinyl gambogate;

A compound introduced benzoyloxy at 11-position is selected, independently at each occurrence, from: gambogic acid, methyl gambogate, ethyl gambogate, gambogyl morpholine, gambogyl piperidine, gambogyl (4-methyl pyrazine), 9,10-dihydro-10-morpholinyl gambogic acid, methyl-9,10-dihydro-10- morpholinyl gambogate;

A compound introduced methyl sulfonyloxy at 11-position is selected, independently at each occurrence, from: gambogic acid, methyl gambogate, ethyl gambogate, gambogyl morpholine, gambogyl piperidine, gambogyl (4-methyl pyrazine);

A compound substituted by D-glycosyloxy and L-glycosyloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl 4'-β-amino- 4'-deoxy-4'demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpholinyl gambogyl (benzyl-L-alaninate), 9,10-dihydro-10-morpholinylgambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10-morpholinyl-11-bromo gambogyl (4'-methyl pyrazine), methyl-9,10-dihydro-10-morpholinyl- 11-acetoxy gambogate, methyl-9,10-dihydro-10-morpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methyl pyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromo gambogic acid, ethyl-11-bromo gambogate, ethyl-11-bromo gambogate, 11-bromo gambogyl morpholine, 11-bromo gambogyl piperidine, 11-bromo gambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11- methylsulfonyloxy gambogyl (4'-methylpyrazine), 11-trifluoromethyl sulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxy gambogyl (4'-methylpyrazime);

A compound substituted by 4-O-D-glucosylbenzoyloxy at 6-position is selected, Independently at each occurrence, from: gambogic acid, methyl gambogate, ethylgambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'-demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, ethyl-9,10-dihydro-10-morpholinylgambogate, ethyl-9,10-dihydro-10-morpholinyl gambogate, 9,10-dihydro-10-morpholinyl gambogyl piperidine, 9,10-dihydro-10-morpholinyl gambogyl (benzyl-L-alaninate), 10-dihydro-10-morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10-morpholinyl-11-bromo gambogyl (4'-methyl pyrazine), ethyl-9,10-dihydro-10-morpholinyl-11-acetoxy gambogate, ethyl-9,10-dihydro-10-morpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methylpyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitromethyl gambogate, 9,10-dihydro-10-(1'-aminopiperidinyl) gambogyl (1'-amino) piperidine, 9,10-dihydro-10-(N-methyl-1'-naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid, ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine), 11-trifluoromethyl sulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxy gambogyl (4'-methylpyrazime);

A compound substituted by 4-O-D-glucosylbenzoyl-L-alanyloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'-demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpholinyl gambogyl (benzyl-L-alaninate), 9,10-dihydro-10- morpholinyl gambogyl morpholine, 9,10-di hydro-10-morpholinyl gambogyl (4'-methyl piperazine), methyl-9,10-dihydro-10-morpholinyl-11-bromogambogate, 9,10-dihydro-10-morpholinyl-11-bromogambogylmorpholine, 9,10-dihydro- 10-morpholinyl-11-bromo gambogyl piperidine, 9,10-dihydro-10-morpholinyl-11-bromo gambogyl (4'-methyl pyrazine), ethyl-9,10-dihydro-10-morpholinyl- 11-acetoxy gambogate, ethyl-9,10-dihydro-10-morpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methylpyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11- methylsulfonyloxy gambogyl (4'-methylpyrazine), 11-trifluoromethyl sulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxy gambogyl (4'-methylpyrazime);

A compound substituted by 4-O-D-allosyl benzoyl-L-alanyl oxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'-demethyl podophyllotoxin), diethylene glycol gambogate, triethylene glycol gambogate, o-chlorophenyl alcohol gambogate, diphenyl methylgambogate, 9,10-dihydro-10-morpholinyl gambogic acid, ethyl-9,10-dihydro-10-morpholinyl gambogate, ethyl-9,10-dihydro-10-morpholinyl gambogate, 9,10-dihydro-10-morphofinyl gambogyl piperidine, 9,10-dihydro-10-morpholinyl gambogyl (benzyl-L-alaninate), 9,10-dihydro-10-morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10-morpholinyl-11-bromo gambogyl (4'-methyl pyrazine), ethyl-9,10-dihydro-10-morpholinyl-11-acetoxy gambogate, ethyl-9,10-dihydro-10-morpholinyl-11-benzoyl gambogate, 9,10-dihydro-10-morpholinyl-11- methyl sulfonyloxy gambogic acid, ethyl-9,10-dihydro-10-morpholinyl-11-methylsulfonyl oxygambogate, ethyl-9,10-dihydro-10- morpholinyl-11-methyl sulfonyloxy gambogate, 9,10-dihydro-10-morpholinyl-11-methylsulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methylpyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethylsulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'-naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11- methylsulfonyloxy gambogyl (4'-methylpyrazine), 11-trifluoromethyl sulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxy gambogyl (4'-methylpyrazime);

A compound substituted by phosphoryloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10- morpholinyl gambogyl (benzyl-L-alaninate), 9,10-dihydro-10- morpholinyl gambogyl morpholine, 9,10-dihydro-10-morpholinyl gambogyl (4'-methyl piperazine), methyl-9,10-dihydro-10- morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10- morpholinyl-11-bromo gambogyl (4'-methyl pyrazine), methyl-9,10-dihydro-10-morpholinyl-11-acetoxy gambogate, methyl-9,10-dihydro-10-morpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methyl pyrazine), 9,10-dihydro-10-morpholinyl-11-trifluoromethyl sulfonyloxy gambogic acid , 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'- methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxy gambogyl (4'-methylpyrazime);

A compound substituted by triphosphoryloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpholinyl gambogyl (benzyl-L-alaninate), 9,10-dihydro-10-morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromogambogyl piperidine, 9,10-dihydro-10-morpholinyl-11-bromo gambogyl (4'-methyl pyrazine), methyl-9,10-dihydro-10-morpholinyl-11-acetoxy gambogate, methyl-9,10-dihydro-10-morpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methyl pyrazine), 9,10-dihydro-10-morpholinyl-11-trifluoromethyl sulfonyloxy gambogic acid , 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'-naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11- methylsulfonyloxy gambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxy gambogyl (4'-methyl pyrazime);

A compound substituted at 30-position is: gambogyl dipentylamine, diethylene glycol gambogate, triethylene glycol gambogate, o-chlorophenyl alcohol gambogate, gambogyl (4'-methylthyl pyrazine), gambogyl (4'-β-amino-4'-deoxy-4 'demethyl podophyllotoxin), diphenylmethyl gambogate, gambogyl (benzyl-L-alaninate), N-(2',6'-dioxopiperidin-3-yl) gambogyl, N-(2',6'-dioxopiperidin-3'-yl)-6-D-glucosyl gambogyl, N-(2',6'-dioxopiperidin-3-yl)-6-(4'-O-D-glucosyl) benzoylacyl-L-alaninegam- bogyl, N-(2',6'-dioxopiperid-in-3'-yl)-6-O-phosphate gambogyl, N-(2',6'-dioxo-piperidin-3'- yl)-6-O-triphosphate gambogyl, gambogyl-4'β-amino- 4'-deoxy-4'-demethyl podophyllotoxin), N-(2',6'-dioxopiperidin-3'-yl)-6-D-allosyl gambogyl and / or N-(2',6'-dioxopiperidin-3'-yl)-6- (4'-O-D-allosyl) benzoylacyl-L-alanine gambogyl;

A compound substituted by2,2-dimethylhexahydropyano[3,2-d] [1,3]dioxine6,7,8-triol or 4-(7',8'-dihydroxyl-2',2-dimethyl hexahydropyrano[3,2-d][1,3]dioxin-6'-yloxy) benzoyloxy at 6-position is: 6-(7,8-dihydroxyl-2,2-dimethylhexahydropyrano[3,2-d][1,3] dioxin-6- yloxy) gambogic acid, 6-(4'-(7",8"-dihydroxyl-2',2"-dimethyl hexahydropyrano [3,2-d] [1,3] dioxin6"-yloxy)benzoyloxy) gambogic acid and its glycoside derivatives or analogs.

A compound according to the gambogic acid glycoside derivatives and analogs of formula I, wherein: a process for the manufacture of a compound of formula I comprises:

(a). for the preparation of compounds of formula I and salts thereof in which the reaction of a compound of formula I in X₁, X₂, R₁, R₂, R₃, R₄, R₅, R₆, R₇, R₈, R₉, R₁₀, R₁₁ or/and R₁₂ is a glycosyl, multi-hydroxyl or substituent-oxy with a bond of C-C, C-O, C-S, C-N or C-P under catalysis at -78 °C to 90 °C;

(b). for the preparation of compounds of formula I and salts thereof in which the reaction of a compound of formula I in X₁, X₂, R₁, R₂, R₃, R₄, R₅, R₆, R₇, R₈, R₉, R₁₀, R₁₁ or/and R₁₂ is an amino acid, acyloxy, phosphoric acid, phosphoryloxy, sulfonyloxy, alkoxy, aryloxy, heterocyclicoxy, hydrocarbons, alicyclic, or heterocyclic aryl containing oxygen, sulfur, nitrogen or phosphorus element with a bond of C-C, C-O, C-S, C-N or C-P under catalysis at -78 °C to 90 °C;

(c). for the preparation of compounds of formula I and salts thereof in which the reaction of a compound of formula I in X₁, X₂, R₁, R₂, R₃, R₄, R₅, R₆, R₇, R₈, R₉, R₁₀, R₁₁ or/and R₁₂ is a carboxyl group containing glycosyl, multi-hydroxyl, phosphate, amino acid, alkane, aryl, alicyclic, heterocyclic, or heteroarylcyclic with a bond of C-C, C-O, C-S, C-N or C-P under catalysis at -78 °C to 90 °C;

(d). for the preparation of compounds of formula I and salts thereof in which the reaction of a compound of formula I in X₁, X₂, R₁, R₂, R₃, R₄, R₅, R₆, R₇, R₈, R₉, R₁₀, R₁₁ or/and R₁₂ is a glucosyl, multi-hydroxyl, amino acid or substituent oxy modified by acylation, halogenation with a bond of C-C, C-O, C-S, C-N or C-P under catalysis at -78 °C to 90 °C;

(e). for the preparation of compounds of formula I and salts thereof in which the reaction of a compound of formula I in X₁, X₂, R₁, R₂, R₃, R₄, R₅, R₆, R₇, R₈, R₉, R₁₀, R₁₁ or/and R₁₂ is a carboxyl group containing glucosyl, multi-hydroxyl, phosphate, amino acid, alkane, aryl, alicyclic, heterocyclic or heteroarylcyclic with a bond of C-C, C-O, C-S, C-N or C-P under catalysis at -78 °C to 90 °C;

(f). for the preparation of compounds of formula I and salts thereof in which the reaction of a compound of formula I in X₁, X₂, R₁, R₂, R₃, R₄, R₅, R₆, R₇, R₈, R₉, P₁₀, R₁₁ or/and R₁₂ is an electrophilic substituent at 9-position or nucleophilic substituent at 10-position accompanied by 1,4 addition reaction with a bond of C-C, C-O, C-S, C-N or C-P under catalysis at -78 oC to 90 °C;

(g). for the preparation of compounds of formula I and salts thereof in which the reaction of a compound of formula I in X₁, X₂, R₁, R₂, R₃, R₄, R₅, R₆, R₇, R₈, R₉, R₁₀, R₁₁ or/and R₁₂ is a substituent at allyl of 11-position, 26-position, 31-position or 36-position modified by a bond of C-C, C-O, C-S, C-N or C-P under catalysis at -78 °C to 90 °C;

(h). for the preparation of compounds of formula I and salts thereof in which the reaction of a compound of formula I X₁, X₂, R₁, R₂, R₃, R₄, R₅, R₆, R₇, R₈, R₉, R₁₀, R₁₁ or/ and R₁₂ is a phosphate or multi-phosphate with C-P bond under catalysis at -78 °C to 90 °C.

A compound according to said the process for the manufacture of a compound of formula I, wherein: the compound is selected from the exemplified examples or stereoisomers, tautomers, pharmaceutically acceptable salts, prodrug or solvates thereof in association wit a pharmaceutically acceptable excipient or carrier.

A method for treating a cancer disorder, comprising: administration to a patient in need thereof a therapeutically effected amount of a compound of the gambogic acid glycoside derivatives and analogs of formula I, or stereoisomers, tautomers, pharmaceutically acceptable salts, prodrug or solvates thereof.

A method according to the gambogic acid glycoside derivatives and analogs of formula I, wherein: a compound for treating, preventing or slowing the progression of neoplasia and cancer, and infection diseases by virus, bacterial or fungi.

A compound according to the gambogic acid glycoside derivatives and analogs of formula I, wherein: a method for treating broad spectrum bacterial and fungal diseases, including bacterial infections and fungal infections of the drug application, which comprises administration together with at least one known chemotherapeutic agent selected from the group consisting of antibacterial and antifungal drugs to a patient in need of such treatment.

A method according to the gambogic acid glycoside derivatives and analogs of formula I, wherein: a cancer is selected from the group consisting of Hodgkin's disease, non-Hodgkin's, lymphoma, acute and chronic lymphocytic leukemias, multiple myeloma, neuroblastoma, breast carcinoma, ovarian carcinoma, lung carcinoma, Wilms' tumor, cervical carcinoma, testicular carcinoma, soft tissue sarcoma, chronic lymphocytic leukemia, primary macroglobulinemia, bladder carcinoma, chronic granulocytic leukemia, primary brain carcinoma, malignant melanoma, small cell lung carcinoma, stomach carcinoma, colon carcinoma, malignant pancreatic insulinoma, malignant carcinoid carcinoma, choriocarcinoma, mycosis fungoides, head and neck carcinoma, osteogentic sarcoma, pancreatic carcinoma, acute granulocytic leukemia, hairy cell leukemia, neuroblastoma, rhabdomyosarcoma, Kaposi's sarcoma, genitourinary carcinoma, thyroid carcinoma, esophageal carcinoma, malignant hypercalcemia, cervical hyperplasia, renal cell carcinoma, endometrial carcinoma, polycythemia vera, essential thrombocytosis, adrenal cortical carcinoma, skin cancer and prostatic carcinoma.

The method according to the gambogic acid glycoside derivatives and analogs of formula I, wherein said compounds is administered together with at least one known cancer chemotherapeutic agent selected from the group consisting of busulfan, cisplatin, mitomycin C, carboplatin, colchichine, vinblastine, paclitaxel, docetaxel, camptochecin, topotecan, doxorubicin, etoposide, 5-azacytidine, 5-fluorouracil, methotrexate, 5-fluoro-2'-deoxyuridine, ara-C, hydroxylurea, thioguanine, melphalan, chlorambucil, cyclo phosamide, ifosfamide, vincristine, mitoguazone, epirubicin, aclarubicin, bleomycin, mitoxantrone, elliptinium, fludarabine, octreotide, retinoic acid, tamoxifen, Herceptin®, Rituxan®, arsenic trioxide, gamcitabine, doxazosin,terazosin tamsulosin, CB-64D, CB-184, haloperidol, lovastatin, simvastatin, pravastatin, fluvastatin, atorvastatin, cerivastatin, amprenavir, abavavir, indinavir, nelfinavir, tipranavir, ritonavir, saquinavir, bexarotene, tretinoin, 13-cis-retinoic acid, 9-cis-retinoic acid, difluoromethylornithine, fenretinide, N-4-carboxyphenyl retinamide, lactacystin, genistein, flavopiridol, roscovitine, olomoucine, celecoxib, valecoxib, rofecoxib and alanosine, CGP-73547,CGP-61755,DMP-450, ABT-378, AG1776, BMS232,632, ILX23-7553, MG-132, PS341, Gleevec®, ZD1839,SH268, CEP2563, SU6668, SU11248, EMD121974, R115777, SCH66336, L-778,123, BAL9611,TAN-1813, UCN-01.

A method for treating cancer, comprising: administration to a compound of the gambogic acid glycoside derivatives and analogs of formula I in the range of 0.001 mg/kg-250 mg/kg, a pharmaceutically acceptable salt or prodrug from thereof.

A compound according to the gambogic acid glycoside derivatives and analogs of formula I, and medication applications, wherein:
the administration may be by oral route, parenteral, subcutaneous, intravenous, intramuscular, intra-peritoneal, transdermal, buccal, intrathecal, intracranial, intranasal or topical routes. The invention has the following effects:

The anti-cancer activity and bioavailability of gambogic acid are related with the water solubility of gambogic acid. The introduction of water-soluble substituent, such as glucosyl or multi-hydroxyl to form gambogic acid glycosides or analogs increased of its water-soluble. The modification of gambogic acid at sensitive structural sites of this invention showed that significant improvement of bioavailability and anti-cancer activity, as well as toxicity. According to all patents disclosed, the structural modifications of gambogic acid have made some progress at 10- and 30-positions. However the semi-synthetic modification at 6-position of phenol hydroxyl is still a bottleneck problem. The glycosides of gambogic acid with glycosyl or multi-hydroxyl have not yet reported so far since the glycoside process of gambogic acid made gambogic acid unstable and decomposed. This invention has successfully made of new gambogic acid glycoside derivatives and analogs by introduction of water-soluble substituents, such as glucosyl or multi-hydroxyl to increase water-soluble and potency. The modification of gambogic acid at 6- and 11-positions, sensitive structural sites of this invention raised inhibition rate of tumor respectively, 90-110% and 45-56% compared with anticancer drugs 5-Fu and cyclophosphamide.

### Synthesis and preparation of gambogic acid glycoside analogs and derivatives

Structural modification at 6-positon of gambogic acid by introduction of glycosyl, multi-hydroxyl, hydroxyl, amino acid or substituted acid groups to form gambogic acid glycoside analogs:

(a) a derivative or analog of gambogic acid or gambogic acid in one of the following solvents was reacted with protected or unprotected carboxyl reagent containing glycosyl, multi-hydroxyl, phosphate and amino acids under the catalysis by one of following catalysts at -78°C to 90°C to form C-O, or P-O bond of protected gambogic acid glycoside analogs. And by deprotection the gambogic acid glycosides derivatives and analogs were obtained (Scheme I):

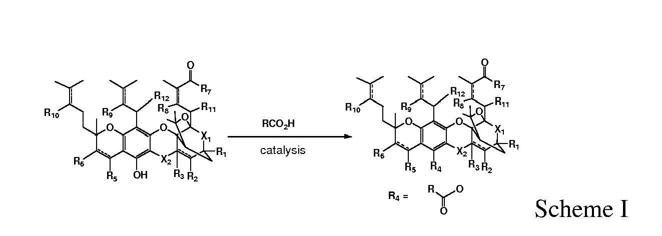

Said solvents are tetrahydrofuran, 1,4-dioxane, acetonitrile, dichloromethane, N,N-dimethylformamide, N,N-dimethyl acetamide, n-hexane, toluene, quinoline, etc; said catalysts are: 1-ethyl-3-(3-dimethyl propyl amine) carbodiimide, ditert-butyl dicarbonate, bis-(2-oxo-3-oxazolalkyl) phosphorus chloride, N,N'-carbonyl pyrrolidine, N,N'-oxo-bis (1,2,4-triazole), 6-chloro-1-hydroxy benzo triazole, N,N'-dicyclohexyl carbodiimide, 4,5-dicyano imidazole, 3-(2-ethoxy phosphoryloxy)-1,2,3-benzotriazine-4-one, diethyl cyano phosphate, N,N'-Diiso propyl carbonate imide, N,N'-diisopropyl ethylamine, 4-dimethyl amino pyridine, 4,4'-dimethoxy-3-phenyl chloromethane, 4-(4,6-dimethoxytriazine)-4-methylmorpholine, N,N'-succinimidyl carbonate, 1-ethyl-(3-dimethyl aminopropyl) carbodiimide, 2-ethoxy-1-ethoxycarbonateacyl-1,2-dihydroquinoline, 2-(7-azobenzotriazole)-N,N,N',N'-tetramethylurea-6-phosphate fluoride, benzo triazole-N,N,N', N'-tetramethyl urea hexafluorophosphate, 6-chloro-3-triazole-1,1,3,3-tetramethylurea hexafluorophosphate, 1-hydroxy-7-azobenzotriazole, 1-hydroxylbenzotriazole, N-hydroxy-5-norbornene-2,3-diimide, 3-hydroxy-1,2,3-benzotriazine-4-(3H) one, N-hydroxysuccinimide, triethyl amine, FMOC, FMOC-OSu.

(b). structural modification at 6-positon of gambogic acid by introduction of C-O or P-O bond to form gambogic acid glycoside analogs: a derivative or analog of gambogic acid or gambogic acid in one of the following solvents was reacted with protected or unprotected halogen reagent containing glycosyl, multi-hydroxyl, phosphate and amino acids under the catalysis by one of following catalysts at -78° C to 90° C to form C-O, or P-O bond of protected halogen gambogic acid glycoside analogs. And by deprotection the gambogic acid glycosides derivatives and analogs were obtained (Scheme II):

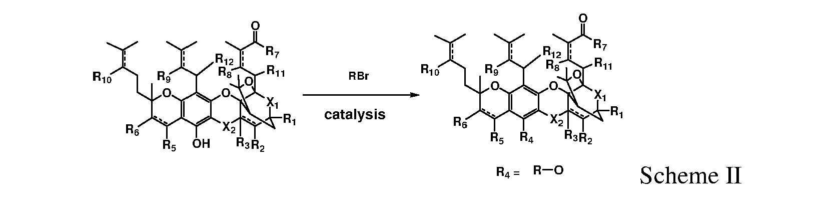

Said solvents are tetrahydrofuran, 1,4-dioxane, acetonitrile, dichloromethane, N, N-dimethylformamide, N,N-dimethylacetamide, n-hexane, toluene, quinoline, etc; said catalysts are : AgCO₃, silver containing catalyst, Lewis acid, perchloric acid, zeolite. Synthesis and preparation of gambogic acid glycoside analogs substituted by ester, anhydride and amide at 30-position:

A derivative or analog of gambogic acid or gambogic acid in one of the following solvents was reacted with protected or unprotected reagent containing hydroxyl, SH, acid, amine, glycosyl, multi-hydroxyl, phosphate and amino acids under the catalysis by one of following catalysts at -78° C to 90° C to form C-O, C-N, C-S or C-P bond of protected gambogic acid glycoside analogs. And by deprotection the gambogic acid glycosides derivatives and analogs were obtained (Scheme III):

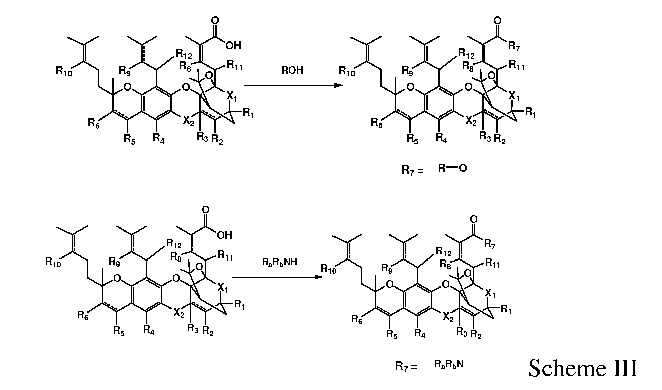

Said solvents are tetrahydrofuran, 1,4-dioxane, acetonitrile, dichloromethane, N-dimethylformamide, N,N-dimethyl acetamide, n-hexane, toluene, quinoline, etc; said catalysts are: 1-ethyl-3-(3-dimethyl propylamine) carbodiimide, ditert-butyl dicarbonate, bis (2-oxo-3-oxazolalkyl) phosphorus chloride, N,N'-carbonyl pyrrolidine, N,N'-oxo-bis(1,2,4-triazole), 6-chloro-1-hydroxy benzo triazole, N,N'-dicyclohexyl carbodiimide, 4,5-dicyanoimidazole, 3-(2-ethoxy phosphoryloxy)-1,2,3-benzotriazine-4-one, diethyl cyano phosphate, N,N'-Diiso-propyl carbonate imide, N,N'-diisopropyl ethylamine, 4-dimethyl amino pyridine, 4,4'-dimethoxy-3-phenyl chloromethane, 4-(4,6-dimethoxytriazine)-4-methyl morpholine, N,N'-succinimidyl carbonate, 1-ethyl-(3-dimethylaminopropyl) carbodiimide, 2-ethoxy-1-ethoxy carbonateacyl-1,2-dihydroquinoline, 2-(7-azobenzotriazole)-N,N,N',N'-tetramethylurea-6-phosphate fluoride, benzo triazole-N,N,N', N'-tetramethyl urea hexafluorophosphate, 6-chloro-3-triazole-1,1,3,3-tetramethyl urea hexafluorophosphate, 1-hydroxy-7-azobenzotriazole, 1-hydroxyl benzotriazole, N-hydroxy-5-norbornene-2,3-diimide, 3-hydroxy-1,2,3-benzotriazine-4 (3H) one, N-hydroxysuccinimide, triethylamine, FMOC, FMOC-OSu.

### Synthesis and preparation of gambogic acid glycoside analogs substituted at 9,10-position

(a) a derivative or analog of gambogic acid or gambogic acid in one of the following solvents was reacted with protected or unprotected nucleophilic reagent containing hydroxyl, SH, amine, glycosyl, multi-hydroxyl, phosphate and amino acids under the catalysis by acid or base catalysts at -78° C to 90° C to form 1,4-addition reaction with a nucleophilic reagent and form C-C, C-O,C-N, C-S or C-P bond of protected gambogic acid glycoside analogs. And by deprotection the gambogic acid glycosides derivatives and analogs were obtained (Scheme IV):

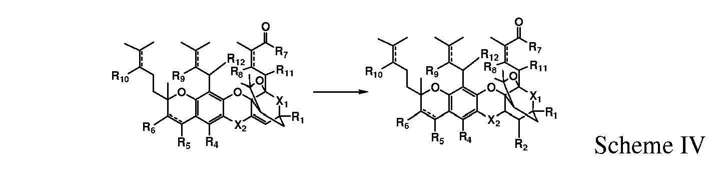

Said solvents are tetrahydrofuran, 1,4-dioxane, acetonitrile, dichloromethane, N,N-dimethylformamide, N,N-dimethylacetamide, n-hexane, toluene, quinoline, etc.

(b) a derivative or analog of gambogic acid or gambogic acid in one of the following solvents was reacted with protected or unprotected two reagents containing aldehyde, ketone, hydroxyl, SH, amine, glycosyl, multi-hydroxyl, phosphate and amino acids under the catalysis by acid or base catalysts at -78° C to 90° C to form 9,10-addition product with a two reagent and form C-C, C-O, C-N, C-S or C-P bond of protected gambogic acid glycoside analogs. And by deprotection the gambogic acid glycosides derivatives and analogs were obtained (Scheme V):

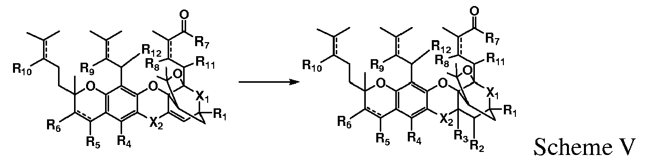

Said solvents are tetrahydrofuran, 1,4- dioxane, acetonitrile, dichloromethane, N-dimethylformamide, N,N-dimethylacetamide, n-hexane, toluene, quinoline, etc.
Synthesis and preparation of gambogic acid glycoside analogs substituted by nucleophilic reagent at 11-position: the structural modification at 11-positon of gambogic acid by bromo-intermediate or introduction of substituent to form gambogic acid glycoside analogs. A derivative and analog of gambogic acid or gambogic acid in one of the following solvents was reacted with NBS, and protected or unprotected nucleophilic reagent at -78° C to 90° C to form C-O, or P-O bond of protected gambogic acid glycoside analogs (Scheme VI):

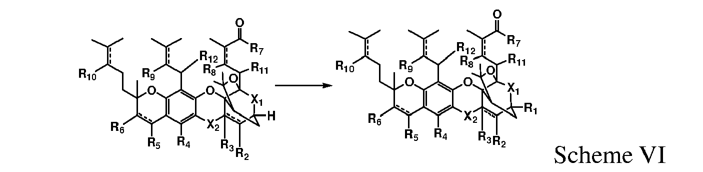

Said solvents are tetrahydrofuran, 1,4-dioxane, acetonitrile, dichloromethane, n-hexane.

(c) Synthesis and preparation of gambogic acid glycoside analogs modified by introduction of epoxide at double bond site: a derivative or analog of gambogic acid or gambogic acid in one of the following solvents was reacted with peroxide reagent at -78° C to 90° C to form epoxided gambogic acid glycoside analogs at 3,4; 9,10; 27,28; 32,33; 37,38 positions (Scheme VII):

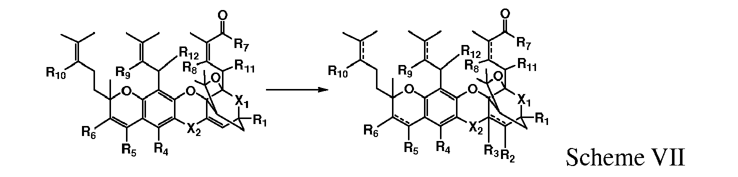

Said solvents are tetrahydrofuran, 1,4-dioxane, acetonitrile, dichloromethane, n-hexane.

### Synthesis and preparation of gambogic acid glycoside analogs substituted by nucleophilic reagent at 8,12-positions

(a) Reduction of ketone to form hydroxyl at 8,12-positons of gambogic acid glycoside analogs: a derivative or analog of gambogic acid or gambogic acid in one of the following solvents was reacted with reduction reagent at room temperature to form hydroxyl gambogic acid glycoside analogs

(b) Reaction with amine to form Schiff base at 8,12-positons of gambogic acid glycoside analogs: a derivative or analog of gambogic acid or gambogic acid in one of the following solvents was reacted respectively with amino reagent at -78° C to 90° C to form Schiff base of gambogic acid glycoside analogs.

(c) Reaction with Wittig reagent to form olefin at 8,12-positons of gambogic acid glycoside analogs: a derivative or analog of gambogic acid or gambogic acid in one of the following solvents was reacted with Wittig reagent containing glycosyl, multi-hydroxyl, phosphate and amino acids under the catalysis by one of following catalysts at -78°C to 90° C to form C=C bond glycoside analogs (Scheme VIII):

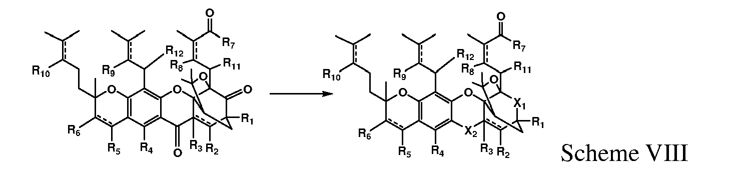

Said solvents are tetrahydrofuran, 1,4-dioxane, acetonitrile, dichloromethane, N-dimethyl formamide, N,N-dimethylacetamide, n-hexane, toluene, quinoline, etc. Synthesis and preparation of gambogic acid glycoside analogs substituted by phosphate reagent at 6-position:

A derivative or analog of gambogic acid or gambogic acid in one of the following solvents was reacted with protected or unprotected phosphate reagent at -78° C to 90° C to form P-O bond of mono- or multi-phosphate gambogic acid glycoside analogs (Scheme IX):

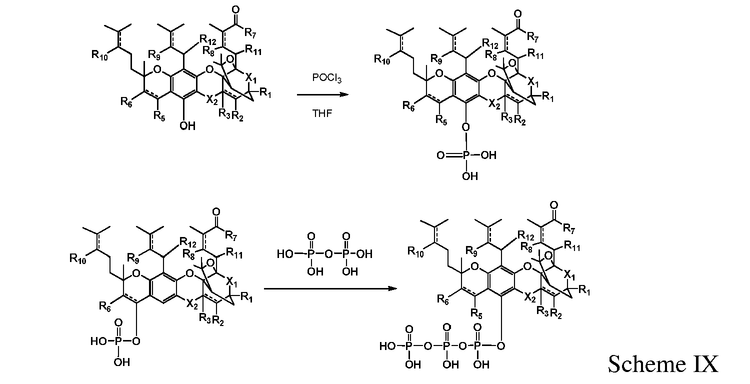

Said solvents are tetrahydrofuran, 1,4-dioxane, acetonitrile, dichloromethane, N, N-dimethyl formamide, N, N-dimethylacetamide, n-hexane and toluene.

A method according to the gambogic acid glycoside derivatives and analogs of formula I and said example compounds, wherein: the compound is selected from the exemplified examples or stereoisomers, tautomers, pharmaceutically acceptable salts, prodrug or solvates thereof in association wit a pharmaceutically acceptable excipient or carrier.

Pharmaceutically acceptable salts can be made by acid or base, such as hydrochloric acid, fumaric acid, maleic acid, succinic acid, acetic acid, citric acid, tartaric acid, carbonic acid, phosphoric acid, oxalic acid,etc or sodium carbonate, sodium hydride, potassium hydroxide, ammonium hydroxide, etc.

Prodrug of this invention can be made to increase its water solubility and molecular size, and reduce its toxicity.

The following examples are illustrative, but not limiting, of the method and composition of the present invention.

### EXAMPLES

### Synthesis and Preparation

The following examples illustrate the present invention. If no mentioned otherwise, the reactions take place at room temperature.

### General Method A (Carboxyl Esterification)

To a mixture of gambogic acid 2.00 mg (3.20 mmol), 1-ethyl-3-(3-dimethylaminopropyl) carbodiimide (EDCI) 1.23 mg ( 6.40 mmol), and 4-dimethylaminopyridine (DMAP) 0.78 g (6.40mmol) in 45 ml THF were added dropwise methanol 1.02 g. The mixture was stirred until the reaction was complete according to thin layer chromatography. Subsequently, the reaction mixture was poured into 100 ml of saline water and extracted with ethyl acetate (3x). The organic phase was evaporated under vacuum and title compound was obtained 1.96 g from the residue by means of flash chromatography (SiO₂).

### General Method B (Carboxyl Amidation)

To a mixture of gambogic acid 360 mg (0.58 mmol), EDCI 221 mg (1.16 mmol), DMAP 141 mg (1.16 mmol) and HOBT 78 mg (0.58 mmol) in 5 ml DMF, were added dropwise n-butyl- amine 73 mg (1.16 mmol). The mixture was stirred until the reaction was complete according to thin layer chromatography. Subsequently, the reaction mixture was poured into 50 ml of saline water and extracted with ethyl acetate (3x). The organic phase was evaporated under vacuum and title compound was obtained 318 mg from the residue by means of flash chromatography (SiO₂).

### General Method C (Glycosylation)

To a mixture of silver carbonate 96.61 mg (0.40 mmol) and methyl gmbogate 225.3 mg (0.35 mmol) in THF 50 ml were added dropwise acetyled bromo allose287.71 mg (0.70 mmol). The mixture was stirred until the reaction was complete according to thin layer chromatography. Subsequently, the reaction mixture was poured into 50 ml of saline water and extracted with ethyl acetate (3x). The organic phase was evaporated under vacuum and title compound was obtained 180 mg from the residue by means of flash chromatography (SiO₂).

### General Method D (Conjugate Addition)

To a mixture of methyl gambogate 450.21 mg (0.70 mmol) in 20 ml THF were added nitromethane 42 mg (0.70 mmol). The mixture was stirred until the reaction was complete according to thin layer chromatography. Subsequently, the reaction mixture was poured into 250 ml of saline water and extracted with ethyl acetate (3x). The organic phase was evaporated under vacuum and title compound was obtained 256 mg. from the residue by means of flash chromatography (SiO₂).

### General Method E (Phenol Esterification)

To a mixture of gambogyl dipentylamine 3.00 g (3.90 mmol) and DMAP 0.60 g (4.9 mmol) in 10 ml dichlomethane were added acetyl protected 4-O-glucosyl benzoyl chloride 3.1 g (7.35 mmol). The mixture was stirred until the reaction was complete according to thin layer chromatography. Subsequently, the reaction mixture was poured into 50 ml of saline water and extracted with ethyl acetate (3x). The organic phase was evaporated under vacuum and title compound was obtained 750 mg from the residue by means of flash chromatography (SiO₂).

### General Method F (Acetylation Deprotection)

To a mixture of Gambogic acid 0.71g (1 mmol) and DMAP 120 mg (1 mmol), triethylamine 150 mg (1.5 mmol) in 10 ml methylene chloride were added 4-O-glucosylbenzoyl chloride 480 mg (1.5 mmol). The mixture was stirred until the reaction was complete according to thin layer chromatography. Subsequently, the reaction mixture was poured into 50 ml of saline water and extracted with ethyl acetate (3x). The organic phase was evaporated under vacuum and title compound was obtained 520 mg from the residue by means of flash chromatography (SiO₂).

### General Method G (Phenol Etherification)

To a mixture of methyl gambogate 900 mg (1.4 mmol) and K₂CO₃ 1.50 g in acetone 20 ml were added dropwise CH₃I 3 ml. The mixture was stirred until the reaction was complete according to thin layer chromato- gramphy. Subsequently, the reaction mixture was poured into 50 ml of saline water and extracted with ethyl acetate (3x). The organic phase was evaporated under vacuum and title compound was obtained 773 mg from the residue by means of flash chromatography (SiO₂).

### Example 1a. Preparation of gambogic acid

The process of gambogic acid reported by S. A. Ahmad is low yield, time consuming and high cost. This invention provided a better new process of gambogic acid with procedues of formation pyridine salt from crude extract, hydrochloric acid replacement, column chromatography, and recrystallization:

(a). Preparation of crude gambogic acid: crushed gamboges was extracted with ethanol and the extract in pyridine was extracted by petroleum ether and water. Crude gambogic acid pyridine salt was obtained by recrystallization.

(b). Preparation of high purity gambogic acid: crude gambogic acid pyridine salt dissolved in dilute hydrochloric acid was extracted by ether and recrystallized to obtain high purity gambogic acid. IR (KBr, cm⁻¹) 3435, 2924, 1737, 1691, 1633, 1594, 1454, 1384, 1175, 1136,1048; ¹H NMR (CDCl₃) δ 12.75 (s, 1H), 7.55 (d, J = 6.9 Hz, 1H), 6.58 (m, 1H), 6.09 (t, J = 7.3 Hz, 1H), 5.37 (d, J = 10.1 Hz, 1H), 5.04 (m, 2H), 3.47 (m, 1H), 3.25 (m, 2H), 2.98 (m, 2H), 2.51 (m, 1H), 2.17 (m, 1H), 2.04 (m, 2H), 1.80-1.45 (m, 22H), 1.36 (s, 3H), 1.28 (s, 3H).

### Example 1. Methyl gambogate

Analogously to method A, starting from gambogic acid. The title compound was obtained and identified. IR (KBr, cm⁻¹) 3448, 2971, 2925, 1737, 1715, 1632, 1594, 1436, 1401, 1382, 1175, 1135; ¹H NMR (CDCl₃) δ 12.86 (s, 1H), 7.54 (d, J = 6.9 Hz, 1H), 6.66 (d, J = 10.1 Hz, 1H), 5.94 (t, J = 7.0 Hz, 1H), 5.45 (d, J = 10.1 Hz, 1H), 5.05 (m, 2H), 3.48 (m, 1H), 3.43 (s, 3H), 3.32 (m, 1H), 3.15 (m, 1H), 2.99 (m, 2H), 2.51 (d, J = 9.3 Hz, 1H), 2.31 (m , 1H), 2.01 (m, 2H), 1.73 (br, 3H), 1.69 (s, 3H), 1.69-1.67 (m, 9H), 1.58 (s, 3H), 1.55 (s, 3H), 1.44 ( s, 3H), 1.28 (s, 3H).

### Example 2. Ethyl gambogate

Analogously to method A, starting from gambogic acid. The title compound was obtained and identified. IR (KBr, cm⁻¹) 3447, 2970, 2926, 1737, 1715, 1635, 1594, 1436, 1405, 1382, 1176, 1136; ¹H NMR (CDCl₃) δ 12.86 (s, 1H), 7.55 (d, J = 6.9 Hz, 1H), 6.67 (d, J = 10.1 Hz, 1H), 5.96 (t, J = 7.0 Hz, 1H), 5.46 (d, J = 10.1 Hz, 1H), 5.06 (m, 2H), 4.13 (m, 2H), 3.48 (m, 1H), 3.34 (m, 1H), 3.19 (m, 1H), 2.96 (m, 2H), 2.53 (d, J = 9.3 Hz, 1H), 2.33 (m , 1H), 2.03 (m, 2H), 1.78 (br, 3H), 1.69 (s, 3H), 1.66-1.67 (m, 9H), 1.54 (s, 3H), 1.55 (s, 3H), 1.46 ( s, 3H), 1.32 (s, 3H), 1.28 (s, 3H).

### Example 3. Ethylene glycol gambogate

Analogously to method A, starting from gambogic acid. The title compound was obtained and identified. IR (KBr cm⁻¹) 3399, 2963, 2920, 1731, 1706, 1644, 1631, 1594, 1456, 1439, 1399, 1378, 1350, 1333, 1307, 1292, 1226, 1178, 1134, 1089; ¹H NMR (CDCl₃) δ 12.86 (s, 1H), 7.55 (d, J = 6.9 Hz, 1H), 6.66 (d, J = 10.2 Hz, 2H), 5.47 (t, J = 15 Hz, 2H), 5.05 (m, 3H), 3.72-3.57 (m, 8H), 3.28-3.18 (m, 3H), 2.06-1.85 (m, 8H), 1.75-1.65 (m, 6H), 1.56 (s, 3H), 1.44 (s, 3H), 1.36 (s, 3H), 1.34 (s, 3H), 1.28 (s, 3H), 1.25 (s, 3H).

### Example 4. Diethylene glycol gambogate

Analogously to method A, starting from gambogic acid. The title compound was obtained and identified. IR (KBr, cm⁻¹) 3399, 2963, 2920, 1731, 1706, 1644, 1631, 1594, 1456, 1439, 1399, 1378, 1350, 1333, 1307, 1292, 1226, 1178, 1134, 1089; ¹H NMR (CDCl₃) δ 12.78 (s, 1H), 7.545 (d, J = 6.6 Hz, 1H), 6.66 (d, J = 9.9 Hz, 1H), 6.32 (m, 1H), 5.46 (d, J = 9.9 Hz , 1H), 5.15-5.05 (m, 2H), 4.25 (m, 2H), 3.72 (m, 1H), 3.59-3.49 (m, 12H), 3.29 (m, 2H), 2.99 (m, 2H), 2.54 (m, 1H), 2.51 (d, J = 9.3 Hz, 1H), 2.06 (s, 2H), 1.74-1.63 (m, 9H), 1.56 (s, 3H), 1.55 (s, 3H), 1.44 (s, 3H), 1.29 (s, 3H), 1.26 (s, 3H).

### Example 5. o-Chlorophenyl alcohol gambogate

Analogously to method A, starting from gambogic acid. The title compound was obtained and identified. IR (KBr, cm⁻¹) 3444, 2976, 2924, 1722, 1651, 1630, 1596, 1438, 1401, 1384, 1366, 1328, 1312, 1297, 1263, 1247, 1229, 1174, 1146, 1091; ¹H NMR (CDCl₃) δ 12.74 (s, 1H), 7.50 (d, J = 6.9 Hz, 1H), 7.35-7.25 (m, 4H), 6.64 (d, J = 9.9 Hz, 1H), 6.43 (m., 1H ), 5.44 (d, J = 10.2 Hz, 1H), 5.30-5.02 (m, 4H), 3.49 (s, 1H), 3.26 (m, 2H), 2.69 (m, 2H), 2.53 (d, J = 9.3 Hz, 1H), 2.31 (m, 1H), 2.03 (m, 3H), 1.8-1.69 (m, 8H), 1.65 (m, 6H), 1.56 (s, 3H), 1.41 (s, 3H), 1.39 (s, 3H), 1.30 (s, 3H).

### Example 6. Diphenyl methanol gambogate

Analogously to method A, starting from gambogic acid. The title compound was obtained and identified. IR (KBr, cm⁻¹) 3457, 2968, 2923, 2853, 1737, 1715, 1632, 1593, 1494, 1455, 1436, 1401, 1383, 1366, 1331, 1239, 1174, 1137, 1090, 1047; ¹H NMR (CDCl₃) δ 12.74 (1H), 7.44 (m, 11H), 7.20 (s, 1H), 6.89 (m, 1H), 6.63 (m, 1H), 5.45 (d, J = 7.2 Hz, 1H), 5.16 (m, 2H), 3.48 (m, 1H), 3.23 (m, 2H), 2.68 (d, J = 8.1Hz, 1H), 2.32 (m, 1H), 2.31 (m, 1H), 2.04 (m, 2H), 1.71-1.64 (m, 13H), 1.55 (s, 3H), 1.40 (s, 6H), 1.32 (s, 3H), 1.27 (s, 3H).

### Example 7. Benzyl alanine gambogyl

Analogously to method B, starting from gambogic acid. The title compound was obtained and identified. IR (KBr, cm⁻¹) 3326, 2972, 2925, 1739, 1630, 1592, 1532, 1498, 1454, 1383, 1297, 1175, 1154; ¹H NMR (CDCl₃) δ 12.90 (1H), 7.55 (d, J = 6.8 Hz, 1H), 7.32 (br, 5H), 6.67 (d, J = 10.1 Hz, 1H), 5.45 (m, 2H), 5.16 (m, 2H), 5.06 (s, 2H), 4.52 (m, 1H), 3.47 (m, 1H), 3.30 (m, 1H), 3.20 (m, 1H), 2.79 (q, 1H), 2.54 (d, J = 9.3 Hz, 1H), 2.42 (m, 1H) , 2.30 (m, 1H), 2.04 (m, 2H), 1.79 (s, 3H), 1.73 (br, 4H), 1.69 (s, 3H), 1.66 (s, 3H), 1.64 (s, 3H), 1.56 (s, 3H), 1.44 (m, 3H), 1.28 (s, 3H), 1.25 (s, 6H).

### Example 8. 6-(4-O-D-Glucosyl) benzoyl gambogyl dipentylamine

(a). Analogously to method B, starting from gambogic acid. The intermediate compound was obtained and identified by thin layer chromatography. (b). Analogously to method E, starting from above intermediate. The intermediate was obtained and identified by thin layer chromatography. (c). Analogously to method F, starting from intermediate obtained from above. The title compound was obtained and identified. IR (KBr, cm⁻¹) 3430, 2959, 2928, 2859, 1740, 1682, 1663, 1603, 1508, 1460, 1430, 1383, 1321, 1244, 1172, 1108, 1082, 1042, 1011, 907, 848, 759, 688, 624, 574, 505; ¹H NMR (CDCl₃) δ 8.15 (d, J = 8.1 Hz, 2H), 7.29 (t, J = 8.1 Hz, 2H), 6.50 (d, J = 10.5Hz, 1H ), 5.82 (d, J = 10.2Hz, 2H), 5.48-5.17 (m, 3H), 4.25 (s, 1H), 4.00-3.70 (m, 10H), 3.49-3.24 (m, 7H), 2.80 ( m, 8 H), 2.60-2.20 (m, 4H), 1.84-0.85 (m, 39H).

### Example 9. 6-(4-O-D-Allosyl) benzoyl gambogyl dipentylamine

(a). Analogously to method B, starting from gambogic acid. The intermediate compound was obtained and identified by thin layer chromatography. (b). Analogously to method E, starting from above intermediate. The title compound was obtained and identified. IR (KBr, cm⁻¹) 3430, 2959, 2928, 2859, 1740, 1682, 1663, 1603, 1508, 1460, 1430, 1383, 1321, 1244, 1172, 1108, 1082, 1042, 1011, 907, 848, 759, 688, 624, 574, 505; ¹H NMR (CDCl₃) δ 8.15 (d, J = 8.1 Hz, 2H), 7.29 (t, J = 8.1 Hz, 2H), 6.50 (d, J = 10.5Hz, 1H), 5.82 (d, J = 10.2Hz, 2H), 5.48-5.17 (m, 3H), 4.25 (s, 1H), 4.00-3.70 (m, 10H), 3.49-3.24 (m, 7H), 2.80 ( m, 8 H), 2.60-2.20 (m, 4H), 1.84-0.85 (m, 39H).

### Example 10. 3-Aminocyclopentyl diimide gambogyl

(a). Analogously to method B, starting from gambogic acid. The intermediate compound was obtained and identified by thin layer chromatography. (b). Analogously to method E, starting from above intermediate. The title compound was obtained and identified. ¹H NMR (CDCl₃) δ 12.86 (1H), 8.75 (br, 2H), 7.69 (d, J = 6.9 Hz, 1H), 6.72 (d, J = 10.1 Hz, 1H), 5.60 (m, 1H), 5.44 (m, 1H), 5.05 (m, 4H), 4.66 (m, 1H), 4.50 (m, 1H), 4.23 (m, 1H), 3.48 (m, 2H), 2.99 (m, 2H), 2.72 ( m, 2H), 2.54 (m, 1H), 2.31 (m, 1H), 2.18 (m, 2H), 2.01 (m, 2H), 1.83-1.22 (m, 24H).

### Example 11. Gambogyl (4-β-amino-4-deoxy-4 'demethyl) podophyllotoxin

Analogously to method B, starting from gambogic acid. The title compound was obtained and identified. IR (KBr, cm⁻¹) 3436, 2967, 2923, 2855, 1773, 1736, 1632, 1593, 1504, 1483, 1457, 1435, 1384, 1331, 1304, 1227, 1175, 1136, 1115, 1039, 1000; ¹H NMR (CDCl₃) δ 12.83 (s, 1H), 7.46 (d, J = 6.9 Hz, 1H), 7.36 (D, J = 7.8 Hz, 1H), 6.82 (m, 1H), 6.78 (d, J = 10.2 Hz, 1H), 6.50 (s, 1H), 6.31 (s, 2H), 6.05 (d, J = 1.5 Hz, 1H), 5.98 (d, J = 1.5 Hz, 1H), 5.47 (m, 1H) , 5.33 (m, 2H), 5.32 (m, 2H), 5.05 (m, 2H), 4.60 (d, J = 4.2 Hz, 1H), 4.42 (m, 1H), 4.12 (m, 2H), 3.88 ( m, 1H), 3.78 (s, 6H), 3.50-3.22 (m, 3H), 3.17 (m, 1H), 2.99 (m, 2H), 2.50 (d, J = 9.6 Hz, 1H), 2.22 (m , 2H), 2.04 (m, 2H), 1.84 (s, 3H), 1.77 (s, 3H), 1.66-1.62 (m, 9H), 1.56 (s, 3H), 140 (s, 3H), 1.29 ( s, 3H).

### Example 12. n-Butyl gambogate

Analogously to method A, starting from gambogic acid. The title compound was obtained and identified. IR (KBr, cm⁻¹) 3353, 2968, 2926, 1738, 1633, 1593, 1534, 1436, 1383, 1332, 1174, 1136, ¹H NMR (CDCl₃) δ 12.86 (s, 1H), 7.56 (d, J = 6.9 Hz, 1H), 6.68 (d, J = 10.2 Hz, 1H), 6.55 (br, 1H), 5.47 (m, 1H), 5.25 (m, 1H), 5.09-5.07 (br, 2H), 3.49 (m, 1H), 3.29 (m, 2H), 3.18 (m, 2H), 2.60 (m, 1H), 2.55 (m, 1H), 2.41 (m, 1H), 2.30 (m, 1H), 2.04 ( m, 2H), 1.79 (s, 3H), 1.76 (m, 2H), 1.73 (s, 3H), 1.69 (br, 6H), 1.56 (s, 3H), 1.49 (br, 2H), 1.45 (s , 3H), 1.41 (m, 3H), 1.35 (br, 2H), 1.29 (s, 3H), 0.92 (m, 3H).

### Example 13. Methyl-9,10-dihydro-10-(nitromethane) gambogate

Analogously to method D, starting from gambogic acid. The title compound was obtained and identified. IR (KBr, cm⁻¹) 3438, 2970, 2926, 1742, 1712, 1625, 1556, 1457, 1398, 1383, 1332, 1292, 1235, 1177, 1133, 1077, 1060, 1037; ¹H NMR (CDCl₃) δ 12.75 (s, 1H), 6.59 (d, J = 10.2 Hz, 1H), 6.10 (m, 1H), 5.41 (d, J = 10.2 Hz, 1H), 5.13 (t, 1H), 5.04 (t, 2H ), 5.02-4.76 (m, 5H, 9-H), 4.56 (t, 1H), 3.55 (s, 3H), 3.25 (m, 1H), 3.12-2.94 (m, 5H), 2.65 (d, J = 8.1 H, 1H), 2.53 (d, J = 4.8Hz, 1H), 2.02 (m, 1H), 1.76 (s, 3H), 1.76-1.22 (m, 21H).

### Example 14. Methyl-6-methoxy gambogate

Analogously to method G, starting from gambogic acid. The title compound was obtained and identified. IR (KBr, cm⁻¹) 3453, 2971, 2926, 2856, 1736, 1715, 1662, 1610, 1588, 1463, 1427, 1382, 1223, 1174, 1136, 1047; ¹H NMR (CDCl₃) δ 7.43 (d, J = 6.9Hz, 1H), 6.66 (d, J = 10.2Hz, 1H), 5.95 (m, 1H), 5.53 (d, J = 10.2 Hz, 1H), 5.05 (m, 2H), 3.81 (s, 3H), 3.48 (m, 1H), 3.43 (s, 3H), 3.38 (m, 1H), 3.26 (br, 1H), 2.97 (m, 2H), 2.49 (d, J = 6.9, 1H), 2.29 (m, 1H), 2.03 (m, 2H), 1.75 (s, 3H), 1.72 (s, 3H), 1.70 (s, 3H), 1.68 (s, 3H), 1.65 (br, 6H), 1.54 ( s, 3H), 1.45 (s, 3H), 1.29 (s, 3H).

### Example 15. 6-Methoxy gambogyl piperidine

(a). Analogously to method B, starting from gambogic acid. The intermediate was obtained and identified by thin layer chromatography. (b). Analogously to method G, starting from above intermediate. The title compound was obtained and identified. IR (KBr, cm⁻¹) 3454, 2923, 2852, 1662, 1611, 1587, 1463, 1427, 1383, 1222, 1172, 1143, 1120; ¹H NM (CDCl₃) δ 7.43 (d, J = 6.9 Hz, 1H ), 6.67 (d, J = 10.2Hz, 1H), 5.56 (d, J = 10.2Hz, 1H), 5.37 (m, 1H), 5.09 (m, 2H), 3.87 (s, 3H), 3.60-3.28 (m, 6H), 3.12 (br, 2H), 2.50 (m, 2H), 2.24 (br, 2H), 2.04 (m, 2H), 1.80-1.25 (m, 32H).

### Example 16. Methyl-11-phenoxylgambogate

To a mixture of NaH 20 mg (0.8 mmol) and phenol 41.0 mg (0.44 mmol) in 5 ml THF were added methyl-11-bromide gambogate 283 mg (0.4 mmol) and the mixture was stirred for 2.5 h at room temperature. The mixture was stirred at room temperature until the reaction was complete according to thin layer chromatography. Subsequently, the reaction mixture was poured into 250 ml of saline water and extracted with ethyl acetate (3x). The organic phase was evaporated under vacuum and title compound was obtained 153 mg from the residue by means of flash chromatography (SiO₂). ¹H NMR (CDCl₃) δ 12.83 (s, 1H), 7.50 (d, J = 6.9 Hz, 1H), 7.29 (d, J = 7.8 Hz, 1H), 7.17 (t, J = 7.4 Hz, 2H), 6.87 (t, J = 7.4 Hz, 1H), 6.82 (d, J = 8.3 Hz, 2H), 6.60 (m, J = 7.0 Hz, 1H), 6.00-5.90 (m, 1H), 5.51 (d, J = 10.5 Hz, 1H), 5.15-5.10 (br, 2H), 5.0-4.92 (m, 3H), 4.61 (m, 2H), 3.42 (s, 3H), 3.30 (m, 1H), 3.22 (m, 2H ), 2.83-2.70 (m, 2H), 2.62-2.50 (m, 2H), 2.25 (m, 1H), 1.80-1.17 (m, 21H).

### Example 17. Methyl-6-benzoyl gambogate

Analogously to method E, starting from methyl gambogate. The title compound was obtained and identified. IR (KBr, cm⁻¹) 3449, 2970, 2926, 1744, 1715, 1663, 1620, 1574, 1462, 1432, 1384, 1365, 1320, 1247, 1176, 1137, 1111, 1064, 1045, 756, 703; ¹H NMR (CDCl₃) δ 8.21 (t, J = 8.7 Hz, 2H), 7.67-7.27 (m, 4H), 6.44 (d, J = 10.2 Hz, 1H), 5.95 (t, J = 8.1 Hz, 1H), 5.57 (d, J = 10.2 Hz, 1H), 5.07 (t, 2H), 3.50 (s, 3H), 3.49 (m, 1H, 3.45 (m, 2H), 3.32 (m, 1H), 3.15 (m , 1H), 2.52 (d, J = 10.3 Hz, 1H), 2.26 (q, 1H), 2.03 (m, 2H), 1.79 (br, 5H), 1.70 (s, 3H), 1.67 (s, 3H) , 1.66 (s, 3H), 1.65 (s, 3H), 1.56 (s, 3H), 1.47 (s, 3H), 1.36 (m, 1H), 1.28 (s, 3H).

### Example 18. Gambogyl dipentylamine

Analogously to method B, starting from gambogic acid. The title compound was obtained and identified. IR (KBr, cm⁻¹) 3448,2961, 2928, 2859, 1739, 1632, 1457, 1436, 1402, 1382, 1331, 1174, 1137, 1048; ¹H NMR (CDCl₃) δ 12.89 (s, 1H), 7.52 (d, J = 6.9 Hz, 1H), 6.68 (d, J = 10.2 Hz, 1H), 5.44 (m, 2H, 3-H), 5.07 (br, 2H), 3.42 (m, 1H), 3.28-2.80 (br, 6H), 2.60-2.00 (br, 5H) ,1.74-1.58 (m, 20H), 1.56-1.12 (m, 20H), 0.985 (m, 6H).

### Example 19. Gambogyl n-butylamine

Analogously to method B, starting from gambogic acid. The title compound was obtained and identified. IR (KBr, cm⁻¹) 3353, 2968, 2926, 1738, 1633, 1593, 1534, 1436, 1383, 1332, 1174, 1136; ¹H NMR (CDCl₃) δ 12.86 (s, 1H), 7.56 (d, J = 6.9 Hz, 1H), 6.68 (d, J = 10.2Hz, 1H), 6.55 (m, 1H), 5.47 (m, 1H), 5.25 (m, 1H), 5.09-5.07 (br, 2H), 3.49 (m, 1H), 3.29 (m, 2H), 3.18 (m, 2H), 2.60 (m, 1H), 2.55 (m, 1H), 2.41 (m, 1H), 2.30 (m, 1H), 2.04 (m, 2H), 1.79-1.25 (m, 31H), 0.92 (t, 3H).

### Example 20. Methyl-9,10-dihydro-10-morpholine gambogate

Analogously to method D, starting from methyl gambogate. The title compound was obtained and identified. IR (KBr, cm⁻¹) 3436, 2969, 2925, 2856, 1739, 1714, 1628, 1585, 1454, 1398, 1383, 1219, 1176, 1122; ¹H NMR (CDCl₃) δ 11.97 (s, 1H), 6.66 (d, J = 10.2 Hz, 1H), 6.61 (t, J = 6.6 Hz, 1H), 5.46 (d, J = 10.2 Hz, 1H), 5.06 (m, 2H), 3.68 (s, 3H), 3.63 (m, 4H), 3.43-3.14 (m, 6H), 2.51 (m, 5H), 2.09-1.99 (m, 4H), 1.95 (s, 3H), 1.74-1.35 (m, 21H), 1.14 (s, 3H).

### Example 21. Methyl-9,10-dihydro-10-piperidine gambogate

Analogously to method D, starting from methyl gambogate. The title compound was obtained and identified. IR (KBr, cm⁻¹) 3450, 2928, 2854, 1739, 1713, 1629, 1586, 1438, 1383, 1175, 1150, 1124; ¹H NMR (CDCl₃) δ 12.01 (s, 1H), 7.24 (br, 1H ), 6.66 (d, J = 10.2 Hz, 1H), 5.46 (d, J = 10.2 Hz, 1H), 5.09 (br, 2H), 3.70 (s, 3H), 3.40-3.05 (br, 4H), 3.00 -2.75 (br, 3H), 2.60-2.20 (br, 5H), 2.20-1.90 (br, 3H), 1.86 (s, 3H), 1.75 (s, 3H), 1.66-1.40 (m, 18H), 1.36 (s, 3H), 1.35 (s, 3H), 1.14 (s, 3H).

### Example 22. 9,10-Dihydro-10-morpholine gambogyl piperidine

(a). Analogously to method B, starting from gambogic acid. The intermediate was obtained and identified by thin layer chromatography. (b). Analogously to method D, starting from above intermediate. The title compound was obtained and identified. IR (KBr, cm⁻¹) 3455, 2967, 2926, 2854, 1738, 1626, 1585, 1439, 1375, 1229, 1122, 1010; ¹H NMR (CDCl₃) δ 12.00 (s, 1H), 6.66 (d, J = 10.5 Hz, 1H), 5.92 (m, 1H), 5.44 (d, J = 10.2 Hz, 1H), 5.07 (m, 2H), 3.72-3.21 (m, 12H), 2.80 (m, 3H), 2.59-2.40 (m, 4H), 2.20-1.84 (m, 6H), 1.76-1.26 (m, 28H), 1.16 (s, 3H).

### Example 23. 9,10-Dihydro-10-(1-aminopiperidinyl) gambogyl-1-aminopiperidinyl

(a). Analogously to method B, starting from gambogic acid. The intermediate was obtained and identified by thin layer chromatography. (b). Analogously to method D, starting from above intermediate. The title compound was obtained and identified. IR (KBr, cm⁻¹) 3446, 2968, 2929, 1745, 1627, 1594, 1436, 1375, 1174, 1144, 1124, 1072, 1037; ¹H NMR (CDCl₃) δ 12.20 (s, 1H), 6.66 (d, J = 10.2 Hz, 1H), 5.81 (m, 1H), 5.50 (d, 1H), .09-5.07 (br, 2H), 4.30 (d, J = 4.5Hz, 1H), 3.75-3.27 (br , 7H), 3.05 (m, 1H), 2.67 (d, J = 6.3 Hz, 2H), 2.53 (d, J = 8.7 Hz, 1H), 2.15-2.00 (m, 3H), 1.88 (s, 3H), 1.80-1.19 (m, 42H).

### Example 24. Methyl-6-O-D-allosyl gmbogate

(a). Analogously to method A, starting from gambogic acid. The intermediate was obtained and identified by thin layer chromatography. (b). Analogously to method C, starting from above intermediate. The intermediate was obtained and identified by thin layer chromatography. (c). Analogously to method F, starting from intermediate obtained from above. The title compound was obtained and identified. IR (KBr, cm⁻¹) 3419, 2967, 2924, 1734, 1710, 1646, 1622, 1455, 1381, 1320, 1230, 1172, 1136, 1072, 888, 802; ¹H NMR (CDCl₃) δ 6.41 (d, J = 10.2 Hz, 1H), 5.56 (d, J = 10.20 Hz, 1H), 5.36 (m, J = 7.2 Hz, 1H), 5.28 (s, 1H), 5.06 (m, 2H), 4.26-4.06 (m, 1H), 3.90-3.13 (m, 16H), 3.13-2.45 (m, 4H), 2.0 (m, 2H), 1.91-1.11 (m, 27H).

### Example 25. 9,10-Dihydro-10-(N-methyl-1-naphthoylamino) gambogyl (N-methyl)-1-naphthoylamine

(a). Analogously to method B, starting from gambogic acid. The intermediate was obtained and identified by thin layer chromatography. (b). Analogously to method D, starting from above intermediate. The title compound was obtained and identified. IR (KBr, cm⁻¹) 3450, 2970, 2923, 1736, 1624, 1585, 1510, 1483, 1454, 1398, 1375, 1324; ¹H NMR (CDCl₃) δ 12.09 (s, 1H), 8.07-7.28 (m, 14H), 6.67 (d, J = 10.2 Hz, 1H), 6.05 (t, J = 7.7 Hz, 1H), 5.45 (m, 1H), 5.09 (m, 2H), 4.30 (d, J = 13.8 Hz , 1H), 4.12 (q, J = 7.2 Hz, 1H), 3.56-2.50 (m, 6H), 2.13-1.16 (m, 40H).

### Example 26. Methyl-6-(4-O-D-allosyl)benzoyl gambogate

(a). Analogously to method A, starting from gambogic acid. The intermediate was obtained and identified by thin layer chromatography. (b). Analogously to method E, starting from above intermediate. The intermediate was obtained and identified by thin layer chromatography. c. Analogously to method F, starting from intermediate obtained from above. The title compound was obtained and identified. IR (KBr, cm⁻¹) 3435, 2925, 285, 1739, 1651, 1606, 1509, 1463, 1384, 1322, 1243, 1174, 1140, 1083, 1043, 850, 759, 618. ¹H NMR (d₆-DMSO) δ 8.12 (d, J = 8.7, 2H), 7.23 (d, J = 9, 2H), 6.49 (d, J = 10.2, 1H), 5.90 (br, 1H), 5.78 (d, J = 10.2, 1H) , 5.41 (d, J = 7.8, 1H), 5.30-5.05 (br, 2H), 4.21 (m, 1H), 3.91 (m, 4H), 3.76-3.60 (m, 5H), 3.56-3.14 (m, 6H), 2.95 (m, 7H), 1.92-1.14 (m, 27H).

### Example 27. 6-(4-O-D-Allosylbenzoyl) gambogyl (N-methyl-1- naphthalene) methylamine

(a). Analogously to method B, starting from gambogic acid. The intermediate was obtained and identified. IR (KBr, cm⁻¹) 3479, 2960, 1755, 1633,1608, 1510, 1373, 1227, 1044. (b). Analogously to method E, starting from above intermediate. The intermediate was obtained and identified by thin layer chromatography. c. Analogously to method F, starting from intermediate obtained from above. The title compound was obtained and identified. IR (KBr, cm⁻¹) 3365, 2925, 1707, 1607, 1512, 1399, 1235, 1066; ¹H NMR (CDCl₃) δ 7.86-7.25 (m, 10H), 6.67 (d, J = 9.9 Hz, 1H), 5.42 (m, 2H), 5.06 (m, 3H), 4.82 (br, 9H), 3.74 (br, 1H), 3.40 (m, 1H), 3.25 (m, 2H), 2.95 (s, 1H), 2.55-1.25 (m, 39H).

### Example 28. Benzyl-L-alaninyl-9,10-dihydro-10-(benzyl-L-alaninyl) gambogate

a. Analogously to method B, starting from gambogic acid. The intermediate was obtained and identified. b. Analogously to method D, starting from above intermediate. The intermediate was obtained and identified by thin layer chromatography. c. Analogously to method F, starting from intermediate obtained from above. The title compound was obtained and identified. IR (KBr, cm⁻¹) 3326, 2972, 2925, 1739, 1630, 1592, 1532, 1498, 1454, 1383, 1297, 1175, 1154; ¹H NMR (CDCl₃) δ 11.98 (s, 1H), 7.39 (m , 10H), 6.67 (d, J = 9.9 Hz, 1H), 5.45 (d, J = 9.9Hz, 1H), 5.18 (m, 4H), 5.02 (br, 2H), 4.56 (t, J = 6.9Hz, 1H), 3.76 (br, 1H), 3.60-3.22 (m, 3H), 3.14 (br, 1H), 2.99 (br, 1H), 2.71 (m, 1H), 2.50 (br, 2H), 2.16-1.12 (m, 39H).

### Example 29. 6-(4-O-D-Glucosylbenzoyl) gambogyl hydroxylethylamine

a. Analogously to method B, starting from gambogic acid. The intermediate was obtained and identified. IR (KBr, cm⁻¹) 3424, 2965, 2927, 2857, 1736, 1713, 1635, 1596, 1507, 1438, 1400, 1385, 1335, 1176, 1138, 1046, 959, 793, 770. b. Analogously to method E, starting from above intermediate. The intermediate was obtained and identified. IR (KBr, cm⁻¹) 3422, 2927, 2857, 1736, 1635, 1595, 1508, 1457, 1436, 1385, 1334, 1177, 1138, 1046, 795, 772, 495. c. Analogously to method F, starting from above intermediate. The title compound was obtained and identified. IR (KBr, cm⁻¹) 3421, 2964 2925, 1739, 1713, 1635, 1606, 1542, 1501, 1456, 1436, 1399, 1326, 1298, 1240, 1176, 1080, 1042, 907, 848, 769; ¹H NMR (CDCl₃) δ 8.22 (d, J = 8.4 Hz, 2H), 7.13 (d, J = 8.4 Hz, 2H), 6.46 (m, 2H), 5.62 (d, J = 9.6 Hz, 1H), 5.38 (d, J = 7.2 Hz, 1H), 5.20 (m, 1H), 5.04 (m, 1H), 4.23 (m, 1H), 3.90 (d, J = 11.4 Hz, 1H), 3.85 (m, 1H), 3.80 (m, 1H), 3.701-3.64 (m, 5H), 3.45-3.36 (m, 3H), 2.66 (m, 2H), 2.52 (d, J = 9.0 Hz, 1H), 2.27 (m, 2H), 2.04 (m, 3H), 1.76 (s, 3H), 1.77 (m , 2H), 1.74 (s, 3H), 1.67-1.58 (m, 11H), 1.56 (s, 3H), 1.46 (m, 5H), 1.35 (s, 3H), 1.28 (s, 3H) .

### Example 30. Aminoethyl-4-O-D-glucosylbenzoyl gambogate

a. Analogously to method A, starting from gambogic acid. The intermediate was obtained and identified. IR (KBr, cm⁻¹) 3430, 2919, 2852, 1733, 1699, 1626, 1604, 1584, 1564, 1504, 1465, 1413, 1386, 1304, 1280, 1227, 1164, 1146, 1096, 1037, 836, 722. b. Analogously to method E, starting from above intermediate. The intermediate was obtained and identified. IR (KBr, cm-1) 3444, 2924, 2856, 1746, 1666, 1634, 1600, 1506, 1457, 1382, 1322, 1225, 1175, 1143, 1087, 1046, 912, 851. c. Analogously to method F, starting from above intermediate. The title compound was obtained and identified. IR (KBr, cm⁻¹) 3344, 2966, 2923, 2855, 1737, 1714, 1665, 1627, 1607, 1520, 1501, 1452, 1374, 1322, 1225, 1178, 1126, 1062, 1047, 957, 909, 832, 747; ¹H NMR (CDCl₃) δ 11.90 (s, 1H), 7.65 (d, J = 8.4 Hz, 2H), 7.54 (d, J = 7.2 Hz, 1H), 7.16 (d, J = 8.4 Hz, 2H), 6.59 (d, J = 10.2 Hz, 1H), 6.48 (t, J = 6.0 Hz, 1H), 5.36 (d, J = 10.2 Hz, 1H), 5.20 (d, J = 7.2 Hz, 1H), 5.00-92 (m, 3H), 4.15-4.19 (m, 3H), 3.74 (m, 3H), 3.60-3.50 (m, 4H), 3.30 (m, 3H), 3.10 (m, 3H), 2.70-2.25 (m, 4H), 2.12-1.98 (m, 4H), 1.97 (m, 3H), 1.70-1.63 (m, 11H), 1.58 (s, 3H), 1.38 (s, 3H), 1.34 (s , 3H), 1.25 (s, 3H).

### Example 31. Methyl-6-(3-acetylamino-4-O-D-glucosyl) benzoylgambogate

a. To a solution of fuming nitric acid 16 ml were added acylated 4-O-D-glucosylbenzoic acid 20.0 g at -20 °C and the mixture was stirred for 1 h. The reaction mixture was poured into 60 ml of water and extracted with ethyl acetate. The organic phase was evaporated to afford the intermediate product 10.2 g from flash chromatography. IR (KBr, cm⁻¹) 3434, 2926, 1753, 1616, 1543, 1701, 1618, 1541, 1502, 1428, 1375, 1231, 1166, 1086, 1065, 1046, 952, 919, 828. b. To a product obtained from above 6.0 g in methanol 20 ml were added 5% palladium car bon 1.2 g and the mixture was hydrogened for 2 h. The organic phase was evaporated to afford the title product 5.3 g from flash chromatography. IR (KBr, cm-1) 3479, 3380, 2964, 1754, 17189, 1622, 1597, 1514, 1447, 1377, 1226, 1157, 1093, 1048, 954. c. To a product obtained from above 4.0 g in THF 15 ml and pyridine 2 ml were added acetic anhydride 2 ml and the mixture was stirred for 4 h. The reaction mixture was poured into 20 ml of water and extracted with ethyl acetate. The organic phase was evaporated to afford the intermediate product 4.10 g from flash chromatography. IR (KBr, cm⁻¹) 3390, 2962, 1757, 1714, 1664, 1597, 1546, 1486, 1444, 1377, 1252, 1229, 1076, 1043, 952, 913, 837. d. To a product obtained from above 4.0 g in dichloromethane 20 ml and DMSO 0.81 ml were added pyridine 0.60 ml and the mixture was stirred for 0.5 h. The organic phase was evaporated and to a mixture of methyl gambogate 3.0 g, DMAP 0.57 g, tri- ethylamine 1.3 ml in methylene chloride 20 ml were added 3-acetylamino-4-O- acetyl glucosyl benzoyl chloride and the mixture was stirred for 0.5 h. at room temperature. The organic phase was evaporated to afford the intermediate product 3.20 g from flash chromatography. e. Analogously to method F, starting from above intermediate. The title compound was obtained and identified. IR (KBr, cm⁻¹) 3427, 2926, 2846, 1742, 1707, 1656, 1635, 1603, 1537, 1456, 1433, 1386, 1263, 1189, 1139, 1047, 805; ¹H NMR (CDCl₃) δ 8.87 (s, 1H), 8.58 (s, 1H), 7.97 (d, J = 7.8 Hz, 1H), 7.36 (d, J = 6.6 Hz, 1H), 7.25 (d, J = 8.4 Hz, 1H), 6.45 ( d, J = 10.8 Hz, 1H), 6.2 (m, J = 6.0 Hz, 1H), 5.56 (d, J = 9.6 Hz, 1H), 5.20-5.05 (m, 4H), 4.05 (m, 1H), 3.80 (m, 1H), 3.78-3.12 (m, 11H), 2.96-2.70 (m, 2 H), 2.66 (m, 2H), 2.45 (d, J = 8.4 Hz, 1H), 2.22 (m, 1H), 2.10 (s, 3H), 2.13 (m, 2H), 1.76 (s, 3H), 1.75 (m, 2H), 1.69 (s, 3H), 1.67 (s, 3H), 1.66 (s, 3H), 1.55 (s, 3H), 1.47 (s, 3H), 1.26 (m, 1H), 1.25 (s, 3H), 1.24 (s, 3H).

### Example 33. 6-(4-O-D-Glucosylbenzoyl) gambogyl (N-methyl-1-naphthoyl- amino) propioylnamine-2

a. Analogously to method B, starting from L-alanine. The intermediate was obtained and identified. IR (KBr, cm⁻¹) 3425, 2978, 2933, 1709, 1648, 1599, 1513, 1487, 1457, 1414, 1387, 1367, 1250, 1167, 1087, 1054, 1020, 866. b. Analogously to method B, starting from the intermediate obtained above. The intermediate was obtained and identified. (KBr, cm⁻¹) 3425, 2960, 2924, 2856, 1740, 1635, 1605, 1577, 1506, 1461, 1436, 1386, 1321, 1246, 1172, 1102, 1082, 1045, 908, 853. c. Analogously to method E, starting from the intermediate obtained above. The intermediate was obtained and identified. IR (KBr, cm⁻¹) 3452, 2965, 2936, 2858, 1750, 1662, 1637, 1605, 1575, 1507, 1482, 1464, 1374, 1324, 1300, 1171, 1142, 1090, 1035, 949, 914. d. Analogously to method F, starting from above intermediate. The title compound was obtained and identified. IR (KBr, cm⁻¹) 3448, 2921, 2851, 1749, 1682, 1633, 1604, 1512, 1462, 1374, 1224, 1166, 1094, 1047, 907; ¹H NMR (CDCl₃) δ 8.14-6.95 (m, 13H), 6.33 (m , 1H), 5.52 (d, J = 10.2 Hz, 1H), 5.33 (m, 1H), 5.25 (m, 1H), 5.15-4.75 (m, 3H), 4.57 (m, 1H), 4.14 (m, 2H), 3.80-3.46 (m, 7H), 3.31 (m, 3H), 3.10 (s, 2H), 2.80-2.75 (m, 4H), 2.43 (d, J = 9.0 Hz, 2H), 2.40-1.97 (m, 7H), 1.78 (s, 3H), 1.77 (m, 3H), 1.69-1.64 (m, 9H), 1.47 (s, 3H), 1.42 (s, 3H), 1.27 (s, 3H), 1.19 (s, 3H).

### Example 34. Methyl-6-O-D-glucosyl gambogate

a. Analogously to method C, starting from methyl gambogate. The intermediate was obtained and identified by thin layer chromatography. b. Analogously to method F, starting from intermediate obtained from above. The title compound was obtained and identified. IR (KBr, cm⁻¹) 3418, 2968, 2926, 1736, 1712, 1648, 1620, 1457, 1383, 1320, 1231, 1174, 1138, 1070, 886, 804; ¹H NMR (CDCl₃) δ 6.43 (d, J = 10.2 Hz, 1H), 5.58 (d, J = 10.20 Hz, 1H), 5.38 (m, J = 7.2 Hz, 1H), 5.30 (s, 1H), 5.09 (m, 2H), 4.28-4.08 ( m, 1H), 3.95-3.15 (m, 16H), 3.15-2.49 (m, 4H), 2.02 (m, 2H), 1.93-1.13 (m, 27 H).

### Example 35. Diphenyl methanol 6-(4-O-D-glucosyl) benzoyl gambogate

a. Analogously to method E, starting from methyl gambogate. The intermediate was obtained and identified by thin layer chromatography. b. Analogously to method F, starting from intermediate obtained from above. The title compound was obtained and identified. IR (KBr, cm⁻¹) 3414, 2966, 2925, 1744, 1667, 1636, 1605, 1509, 1462, 1435, 1386, 1322, 1245, 1173, 1101, 1082, 1044, 908, 846; ¹H NMR (CDCl₃) δ 8.16 (d, J = 7.5, 1H), 7.93 (d, J = 7.5, 1H), 7.20 (s, 10H), 7.02 (d, 1H), 6.86 (d, 1H), 6.42 (d, 1H ), 5.566 (m, 1H), 5.36 (m, 1H), 5.10 (m, 2H), 4.11 (m, 3H), 3.88-3.61 (br, 5H), 3.65-3.15 (br, 3H), 3.04 (s, 3H), 2.82-2.20 (m, 4H), 2.06 (m, 4H), 1.98-1.72 (m, 2H), 1.84-1.23 (m, 25H).

### Example 36. 6-(4-O-D-Glucosyl) benzoylgambogyl butylamine

a. Analogously to method C, starting from gambogyl butylamine. The intermediate was obtained and identified by thin layer chromatography. b. Analogously to method F, starting from intermediate obtained from above. The title compound was obtained and identified. IR(KBr, cm⁻¹) 3414, 2922, 2855, 1742, 1664, 1606, 1507, 1461, 1384, 1325, 1244, 1175, 1144, 1082, 1042, 956; ¹H NMR (CDCl₃) δ 8.19 (d, J = 8.7 Hz, 2H), 7.36 (d, J = 6.3 Hz, 1H), 7.12 (d, J = 8.7 Hz, 2H), 6.41 (d, J = 10.2 Hz, 1H), 5.57 (d, J = 15.3 Hz, 1H), 5.35 (s, 1H), 5.20-5.06 (m, 1H), 4.14 (s, 1H), 4.13 (d, J = 7.5Hz, 1H), 3.80-3.42 (m, 4H), 3.17 (d, J = 4.5 Hz, 2H), 2.60 (s, 6H), 2.08 (d, J = 7.23 Hz, 6H), 1.78-1.24 (m, 34H).

### Example 37. 6-(4-O-D-Glucosyl) benzoyl gambogyl piperidine

a. Analogously to method E, starting from gambogyl piperidine. The intermediate was obtained and identified by thin layer chromatography. b. Analogously to method F, starting from intermediate obtained from above. The title compound was obtained and identified. IR (KBr, cm⁻¹) 3374, 2960, 2918, 2852, 1738, 1666, 1605, 1576, 1542, 1507, 1462, 1436, 1387, 1321, 1246, 1174, 1149, 1086, 1043, 908; ¹H NMR (CDCl₃) δ 8.16 (d, J = 8.4 Hz, 2H), 7.33 (d, J = 7.2Hz, 1H), 7.12 (d, J = 7.8Hz, 2H), 6.44 (t, J = 13.8Hz, 1H ), 5.56 (d, J = 6Hz, 1H), 5.41 (m, 1H), 5.06 (d, J = 7.5Hz, 2H), 4.30 (s, 2H), 4.13 (d, J = 6.9Hz, 2H), 3.90-3.11 (m, 11H), 2.06 (d, J = 14.7Hz, 7H), 1.96 (s, 3H), 1.77-1.24 (m, 32H).

### Example 38. 6-(4-O-D-Glucosyl) benzoyl gambogyl (benzyl)-L-alanine

a. Analogously to method E, starting from gambogyl (benzyl)-L-alanine. The intermediate was obtained and identified by thin layer chromatography. b. Analogously to method F, starting from intermediate obtained from above. The title compound was obtained and identified. IR (KBr, cm⁻¹) 3460, 3066, 2967, 2925, 2856, 1757, 1663, 1607, 1507, 1462, 1435, 1374, 1323, 1226, 1173, 1145, 1088, 1045, 1012, 947, 912; ¹H NMR (d₆-DMSO) δ 8.14 (d, J = 8.1 Hz, 2H), 7.52 (d, J = 6.6 Hz, 1H), 7.35 (d, J = 6 Hz, 2H), 7.23 (d, J = 8.7 Hz , 2H), 6.52 (d, J = 10.8 Hz, 1H), 6.01 (m, 1H), 5.82 (d, J = 10.2 Hz, 1H), 5.60 (s, 1H), 5.42 (d, J = 7.8 Hz , 1H), 5.13 (m, 3H), 4.45 (m, 2H), 4.20 (s, 1H), 4.11 (m, 1H), 4.0 (q, 1H), 3.91 (m, 2H), 3.84 (m, 1H), 3.63 (m, 7H) 3.50-3.20 (m, 6H), 2.14-2.09 (br, 2H), 1.90 (s, 3H), 1.82 (s, 3H), 1.693 (s, 3H), 1.64 ( s, 3H), 1.57 (s, 3H), 2.14-1.147 (m, 16H).

### Example 39. Methyl-6-(4-O-D-glucosyl) benzoyl-9,10-dihydro-10- morpholinylgambogate

a. Analogously to method D, starting from methylgambogate. The intermediate was obtained and identified. b. Analogously to method E, starting from intermediate obtained from above. The intermediate was obtained and identified. c. Analogously to method F, starting from intermediate obtained from above. The title compound was obtained and identified. ¹H NMR (d₆-DMSO) δ 8.13 (d, J = 7.2 Hz, 2H), 7.23 (d, J = 9 Hz, 2H), 6.69 (t, J = 6 Hz, 1H), 6.55 (d, J = 7.2 Hz , 1H), 5.78 (d, J = 10.2 Hz, 1H), 5.42 (d, J = 7.8 Hz, 1H), 5.14 (br, 1H), 4.20 (t, J = 3 Hz, 1H), 3.98-3.80 (m, 2H), 3.66 (s, 6H), 3.63-3.17 (m, 10H), 2.88 (br, 4H), 2.80-2.20 (m, 7H), 1.99-1.12 (m, 30H).

### Example 40. Methyl-6-(4-O-D-glucosyl) benzoyl-9,10-dihydro-10-nitromethylgambogate

a. Analogously to method D, starting from methylgambogate. The intermediate was obtained and identified. b. Analogously to method E, starting from intermediate obtained from above. The intermediate was obtained and identified. c. Analogously to method F, starting from intermediate obtained from above. The title compound was obtained and identified. ¹H NMR (d₆-DMSO) δ 8.14 (d, J = 8.7 Hz, 2H), 7.35 (d, J = 7.5 Hz, 1H), 7.24 (d, J = 8.4 Hz, 2H), 6.47 (d, J = 10.2 Hz, 1H), 5.76 (d, J = 10.2 Hz, 1H), 5.44 (d, J = 5.1 I Hz, 1H), 5.12 (m, 1H), 4.20 (s, 1H) ,3.99-3 .80 (m, 3H), 3.79-3.40 (m, 9H), 3.30 (m, 2H), 2.88 (br, 9H), 2.53 (m, 1H), 2.30 (m, 1H), 2.03 (m, 2H), 1.89-1.23 (m, 26H).

### Example 41. Methyl-6-(4-O-D-glucosyl) benzoyl-L-alaninacyl-9,10-dihydro-10-N-methyl- naphthylamine gambogyl

a. Analogously to method E, starting from 4-glucosylbenzoyl-L-alanine and methyl-9,10-dihydro-10-N-methylamino naphthalene gambogate. The intermediate was obtained and identified. b. Analogously to method F, starting from intermediate obtained from above. The title compound was obtained and identified. IR (KBr, cm⁻¹) 3400, 2956, 1755, 1637, 1607, 1497, 1372, 1229, 1090, 1043; ¹H NMR (CDCl₃) δ 8.73 (d, J = 7.2 Hz, 1H), 8.16-7.46 (m , 13H), 7.24 (d, J = 8.7 Hz, 2H), 5.72 (m, 3H), 5.27-4.89 (m, 7H), 4.53 (m, 2H), 4.25 (m, 6H), 4.01-3.30 ( br, 10H), 3.03 (s, 3H), 2.95 (s, 1H), 2.56 (m, 3H), 2.20 (s, 6H), 2.14-1.33 (m, 21H).

### Example 42. Methyl-6-(4-O-D-glucosyl) benzoyl gambogate

a. Analogously to method E, starting from methylgambogate. The intermediate was obtained and identified. b. Analogously to method F, starting from intermediate obtained from above. The title compound was obtained and identified. IR (KBr, cm⁻¹) 3435, 2925, 285, 1739, 1651, 1606, 1509, 1463, 1384, 1322, 1243, 1174, 1140, 1083, 1043, 850, 759, 618. ¹H NMR (d₆-DMSO) δ 8.12 (d, J = 8.7, 2H), 7.23 (d, J = 9, 2H), 6.49 (d, J = 10.2, 1H), 5.90 (br, 1H), 5.78 (d, J = 10.2, 1H), 5.41 (d, J = 7.8, 1H), 5.30-5.05 (br, 2H), 4.21 (m, 1H), 3.91 (m, 4H), 3.76-3.60 (m, 5H), 3.56-3.14 (m, 6H), 2.95 (m, 7H), 1.92-1.14 (m, 27H).

### Example 43. 6-(4-O-D-Allosyl) benzoyl gambogyl hydroxyethylamine

a. Analogously to method E, starting from gambogyl hydroxyethylamine. The intermediate was obtained and identified. b. Analogously to method F, starting from intermediate obtained from above. The title compound was obtained and identified. IR (KBr, cm⁻¹) 3421, 2968, 2926, 1739, 1711, 1633, 1607, 1544, 1502, 1458, 1438, 1398, 1328, 1299, 1241, 1176, 1082, 1042, 906, 848, 768, 560; ¹H NMR (CDCl₃) δ 8.20 (d, J = 8.4 Hz, 2H), 7.13 (d, J = 8.4 Hz, 2H), 6.44 (m, 2H), 5.60 (d, J = 9.6 Hz, 1H), 5.36 (d, J = 7.2 Hz, 1H), 5.22 (m, 1H), 5.07 (m, 1H), 4.25 (m, 1H), 3.93 (d, J = 11.4 Hz, 1H), 3.86 (m, 1H), 3.81 (m, 1H), 3.71-3.66 (m, 5H), 3.46-3.37 (m, 3H), 2.64 (m, 2H), 2.51 (d, J = 9.0 Hz, 1H), 2.29 (m , 2H), 2.05 (m, 3H), 1.78 (s, 3H), 1.76 (m, 2H), 1.72 (s, 3H), 1.67-1.59 (m, 11H), 1.58 (s, 3H), 1.47 ( m, 5H), 1.36 (s, 3H), 1.29 (s, 3H).

### Example 44. 4-O-D-Allosyl benzoylaminoethyl gambogate

a. Analogously to method C, starting from benzoylaminoethyl gambogate. The intermediate was obtained and identified. b. Analogously to method F, starting from intermediate obtained from above. The title compound was obtained and identified. IR (KBr, cm⁻¹) 3347, 2967, 2924, 2856, 1739, 1716, 1664, 1628, 1609, 1522, 1503, 1453, 1375, 1323, 1224, 1175, 1125, 1060, 1045, 955, 907, 830, 748, 599; ¹H NMR (CDCl₃) δ 11.92 (s, 1H), 7.66 (d, J = 8.4 Hz, 2H), 7.55 (d, J = 7.2 Hz, 1H), 7.13 (d, J = 8.4 Hz, 2H), 6.57 (d, J = 10.2 Hz, 1H), 6.49 (t, J = 6.0 Hz, 1H), 5.38 (d, J = 10.2 Hz, 1H), 5.22 (d, J = 7.2 Hz, 1H), 5.02-92 (m, 3H), 4.25-4.19 (m, 3H), 3.78 (m, 3H), 3.66-3.59 (m, 4H), 3.40 (m, 3H), 3.20 (m, 3H) , 2.70-2.25 (m, 4H), 2.12-1.98 (m, 4H), 1.97 (m, 3H), 1.70-1.63 (m, 11H), 1.58 (s, 3H), 1.38 (s, 3H), 1.34 (s, 3H), 1.25 (s, 3H).

### Example 45. Methyl-6-(3-acetyl-amino-4-O-D-allosyl) benzoyl gambogate

a. Analogously to method C, starting from methylgambogate and 3-acetyl-amino-4-O-D-allose. The intermediate was obtained and identified. b. Analogously to method F, starting from intermediate obtained from above. The title compound was obtained and identified. IR (KBr, cm⁻¹) 3429, 2925, 2848, 1740, 1709, 1655, 1633, 1605, 1539, 1458, 1431, 1384, 1262, 1188, 1138, 1046, 805, 755, 502; ¹H NMR (CDCl₃) δ 8.86 (s, 1H), 8.56 (s, 1H), 7.96 (d, J = 7.8 Hz, 1H), 7.35 (d, J = 6.6 Hz, 1H), 7.24 (d, J = 8.4 Hz, 1H), 6.42 (d, J = 10.8 Hz, 1H), 6.0 (m, J = 6.0 Hz, 1H), 5.58 (d, J = 9.6 Hz, 1H), 5.21-5.02 (m, 4H), 4.07 (m , 1H), 3.90 (m, 1H), 3.80-3.20 (m, 11H), 2.98-2.70 (m, 2 H), 2.64 (m, 2H), 2.47 (d, J = 8.4 Hz, 1H), 2.20 (m, 1H), 2.12 (s, 3H), 2.12 (m, 2H), 1.75 (s, 3H), 1.74 (m, 2H), 1.68 (s, 3H), 1.65 (s, 3H), 1.64 ( s, 3H), 1.54 (s, 3H), 1.45 (s, 3H), 1.28 (m, 1H), 1.25 (s, 3H), 1.23 (s, 3H).

### Example 46. Methyl-6-(4'-((4"R,6"S,7"R,8"S)-7",8"-dihydroxy-2",2"-Dimethylhexahydropyrano [3,2-d][1,3] dioxane-6"-O)) benzoyl gambogate

To a mixture of the product obtained from example 42 2.0 g (2.15 mmol) in 20 ml acetone were added p-toluenesulfonic acid 0.37 g (2.15 mmol) and the mixture was stirred for 12 h. The organic phase was evaporated and the title compound 1.20 g was obtained from the residue by means of flash chromatography (SiO₂). IR (KBr, cm⁻¹) 3410, 2957, 2924, 2854, 1738, 1716, 1663, 1606, 1515, 1463, 1384, 1322,, 1110, 1043, 849, 690, 606; ¹H NMR (CDCl₃) δ 8.0 (d, J = 8.4 Hz, 2H), 7.41 (d, J = 6 Hz, 1H), 6.85 (d, J = 8.4 Hz, 1H), 6.76 (d, J = 8.4 Hz, 2H), 6.45 9d, J = 10.2 Hz, 1H), 5.94 (t, J = 6.0 Hz, 1H), 5.58 (d, J = 10.2 Hz, 1H), 5.11-5.05 (m, 2H), 3.70 (m, 1H), 3.52 ( s, 3H), 3.42 (m, 2H), 3.28-3.25 (m, 2H), 3.00 (m, 2H), 2.53 (d, J = 9.0 Hz, 1H), 2.29 (m, 1H), 2.20 (s , 1H), 2.04 (m, 2H), 1.81-1.56 (m, 27H), 1.48 (s, 3H), 1.39 (m, 2H), 1.29 (s, 3H), 1.26 (s, 3H).

### Example 47. 6-(4'-O-D-Allosyl)benzoyl)-30-(N-(1-(methyl(naphthalene-1-ylmethyl) amino)-1-oxopropan-2-yl)) gambogamide

a. To a mixture of BOC-L-Alanine 1.89g (10 mmol), EDCI 2.304g (12 mmol) and DMAP 0.61g (5 mmol) in 20 ml THF were added N-methyl-naphthalene methylamine 2.05g (12 mmol) and the mixture was stirred for 4 h. The organic phase was evaporated and the intermediate 2.42 g was obtained from the residue by means of flash chromatography (SiO₂). IR (KBr, cm⁻¹) 3426, 2978, 2931, 1709, 1646, 1599, 1511, 1487, 1457, 1414, 1385, 1367, 1250, 1167, 1087, 1051, 1020, 866, 793, 778, 591. b. To a product obtained from above 1.71g (5 mmol) was added solution of 5 ml of trifluoro acetic acid: dichloromethane(1:2) and the mixture was stirred for 2 h. The organic phase was evaporated to afford the title product 2.42 g from flash chromatography and gambogic acid 3.14 g (5 mmol), EDCI 96mg (0.5 mmol), L-alanine-N-methylnaphthalene formamide and DMAP 30.5mg (0.25 mmol) in tetrahydrofuran 5 ml and triethylamine 5 ml were added dropwise and the mixture was stirred for 6 h. The organic phase was evaporated and the intermediate product 2.64 g was obtained from the residue by means of flash chromatography (SiO₂). IR (KBr, cm⁻¹) 3429, 2963, 2925, 2856, 1740, 1639, 1605, 1575, 1508, 1461, 1432, 1384, 1321, 1244, 1172, 1100, 1082, 1043, 908, 850, 793, 760, 688. c. To a product obtained from above of 2.173g (2.5 mmol), DMAP 0.159g (1.3 mmol) and 1 ml triethylamine in 10 ml methylene chloride were added dropwise 4-O-alorglycosyl benzoyl chloride 0.796 g (2.5 mmol) and the mixture was stirred for 30 min. The organic phase was evaporated and the title product 20.52 was obtained from the residue by means of flash chromatography (SiO₂). IR (KBr, cm⁻¹) 3450, 2965, 2926, 2856, 1750, 1662, 1639, 1605, 1575, 1509, 1482, 1462, 1374, 1321, 1300, 1175, 1142, 1090, 1045, 949, 910, 852, 760, 687, 600. d. To a product obtained from above of 1.325 g (1.0 mmol) in anhydrous methanol 10 ml were added dropwise 2-butyltin oxide 0.249 g (1.0 mmol) and the mixture was refluxed for 8 e. The organic phase was evaporated and the title product 0.243 g was obtained from the residue by means of flash chromatography (SiO₂). IR (KBr, cm⁻¹) 3445, 2921, 2851, 1747, 1682, 1631, 1604, 1508, 1460, 1374, 1225, 1166, 1092, 1047, 909, 793, 780, 576; ¹H NMR (CDCl₃) δ 8.15 - 6.95 (m, 13H), 6.34 (m, 1H), 5.50 (d, J = 10.2 Hz, 1H), 5.31 (m, 1H), 5.23 (m, 1H), 5.13-4.75 (m, 3H), 4.59 (m, 1H ), 4.16 (m, 2H), 3.90 - 3.54 (m, 7H), 3.33 (m, 3H), 3.15 (s, 2H), 2.90-2.75 (m, 4H), 2.43 (d, J = 9.0 Hz, 2H), 2.40-1.95 (m, 7H), 1.76 (s, 3H), 1.74 (m, 3H), 1.69-1.64 (m, 9H), 1.49 (s, 3H), 1.42 (s, 3H), 1.27 (s, 3H), 1.19 (s, 3H).

### Example 48. 6-(4-O-D-Glucoslbenzoyl) gambogyl (N-methyl-1-naphthalene) methylamine

a. To a mixture of 0.28 g (1.48 mmol) EDCI, 0.06 g (0.5mmol) DMAP, THF 10 ml, 1g (1mmol) 9,10-dihydro-10-(N-methyl-1-naphthalene methylamine) gambogyl (N-methyl-1- naphthalene methylamine) in THF 10 ml were added 0.59 g (1.48 mmol) acetyled 4-O-glucosylbenzoic acid until the reaction was completeted. The reaction mixture was extracted with ethyl acetate and the organic phase was evaporated and the intermediate 0.24 g was obtained from the residue by means of flash chromatography (SiO₂). IR (KBr, cm⁻¹) 3479, 2960, 1755, 1633,1608, 1510, 1373, 1227, 1044. b. Analogously to method F, starting from intermediate obtained from above. The title compound was obtained and identified. IR (KBr, cm⁻¹) 3367, 2925, 1707, 1607, 1510, 1399, 1238, 1069; ¹H NMR (CDCl₃) δ 7.87-7.27 (m, 10H), 6.68 (d, J = 9.9 Hz, 1H), 5.44 (m, 2H), 5.08 (m, 3H), 4.80 (br, 9H), 3.75 (br, 1H), 3.42 (m, 1H), 3.27 (m, 2H), 2.96 (s, 1H), 2.57-1.25 (m, 39H).

### Example 49. Diphenyl methyl-6-(4-O-D-alorglycosyl)benzoyl gambogate

a. Analogously to method E, starting from methyl gambogate. The intermediate was obtained and identified. b. Analogously to method F, starting from intermediate obtained from above. The title compound was obtained and identified. IR (KBr, cm⁻¹) 3418, 2966, 2925, 1740, 1667, 1636, 1604, 1509, 1460, 1435, 1384, 1322, 1243, 1171, 1101, 1080, 1042, 908, 848, 759, 697, 623, 575, 502; ¹H NMR (CDCl₃) δ 8.18 (d, J = 7.5, 1H), 7.95 (d, J = 7.5, 1H), 7.22 (s, 10H), 7.04 (d, 1H), 6.84 ( d, 1H), 6.40 (d, 1H), 5.58 (m, 1H), 5.38 (m, 1H), 5.12 (m, 2H), 4.13 (m, 3H), 3.87-3.61 (br, 5H), 3.5-3.15 (br, 3H), 3.03 (s, 3H), 2.80-2.20 (m, 4H), 2.04 (m, 4H), 1.96-1.72 (m, 2H), 1.82-1.23 (m, 25H).

### Example 50. 6-(4-O-D-Allosyl) benzoyl gambogyl butylamine

a. Analogously to method E, starting from gambogyl butylamine. The intermediate was obtained and identified. b. Analogously to method F, starting from intermediate obtained from above. The title compound was obtained and identified. IR (KBr, cm⁻¹) 3416, 2920, 2853, 1740, 1662, 1604, 1509, 1461, 1384, 1321, 1244, 1173, 1144, 1080, 1042, 954, 906, 851, 759, 688, 623, 502; ¹H NMR (CDCl₃) δ 8.17 (d, J = 8.7 Hz, 2H), 7.37 (d, J = 6.3 Hz, 1H), 7.13 (d, J = 8.7 Hz, 2H), 6.45 (d, J = 10.2 Hz, 1H), 5.59 (d, J = 15.3 Hz, 1H), 5.36 (s, 1H), 5.22-5.06 (m, 1H), 4.16 (s, 1H), 4.12 (d, J = 7.5Hz, 1H), 3.87-3.42 (m, 4H), 3.19 (d, J = 4.5 Hz, 2H), 2.62 (s, 6H), 2.06 (d, J = 7.23 Hz, 6H), 1.75-1.24 (m, 34H).

### Example 51. 6-(4-O-D-Allosyl) benzoyl gambogyl piperidine

a. Analogously to method E, starting from gambogyl piperidine. The intermediate was obtained and identified. b. Analogously to method F, starting from intermediate obtained from above. The title compound was obtained and identified. IR (KBr, cm⁻¹) 3376, 2960, 2918, 2850, 1738, 1664, 1605, 1574, 1542, 1509, 1462, 1432, 1385, 1321, 1244, 1172, 1149, 1088, 1043, 907, 850, 760, 730, 688, 624, 504, 473; ¹H NMR (CDCl₃) δ 8.18 (d, J = 8.4 Hz, 2H), 7.31 (d, J = 7.2Hz, 1H), 7.14 (d, J = 7.8Hz , 2H), 6.43 (t, J = 13.8Hz, 1H), 5.58 (d, J = 6Hz, 1H), 5.41 (m, 1H), 5.08 (d, J = 7.5Hz, 2H), 4.31 (s, 2H), 4.12 (d, J = 6.9Hz, 2H), 3.90-3.21 (m, 11H), 2.06 (d, J = 14.7Hz, 7H), 1.96 (s, 3H), 1.77-1.24 (m, 32H).

### Example 52. 6-(4-O-D-Allosyl) benzoyl gambogyl benzyl-L-alaninate

a. Analogously to method E, starting from gambogyl benzyl-L-alaninate. The intermediate was obtained and identified. b. Analogously to method F, starting from intermediate obtained from above. The title compound was obtained and identified. IR (KBr, cm⁻¹) 3463, 3064, 2967, 2926, 2856, 1755, 1663, 1606, 1509, 1462, 1433, 1374, 1321, 1227, 1174, 1147, 1088, 1043, 1012, 949, 910, 850, 758, 745, 700, 651, 619, 600; ¹H NMR (d₆-DMSO) δ 8.15 (d, J = 8.1 Hz, 2H), 7.55 (d, J = 6.6 Hz, 1H), 7.37 ( d, J = 6 Hz, 2H), 7.25 (d, J = 8.7 Hz, 2H), 6.54 (d, J = 10.8 Hz, 1H), 6.03 (m, 1H), 5.80 (d, J = 10.2 Hz, 1H), 5.62 (s, 1H), 5.42 (d, J = 7.8 Hz, 1H), 5.15 (m, 3H), 4.45 (m, 2H), 4.21 (s, 1H), 4.15 (m, 1H), 4.05 (q, 1H), 3.91 (m, 2H), 3.87 (m, 1H), 3.67 (m, 7H) 3.52-3.210 (m, 6H), 2.14-2.09 (br, 2H), 1.90 (s, 3H ), 1.82 (s, 3H), 1.693 (s, 3H), 1.64 (s, 3H), 1.57 (s, 3H), 2.14-1.147 (m, 16H).

### Example 53. Methyl-6-(4-O-D-allosyl) benzoyl-9,10-dihydro-10-morpholinyl gambogate

a. Analogously to method D, starting from methyl gambogate. The intermediate was obtained and identified. b. Analogously to method E, starting from intermediate obtained from above. The intermediate was obtained and identified. c. Analogously to method F, starting from intermediate obtained from above. The title compound was obtained and identified. ¹H NMR (d₆-DMSO) δ 8.15 (d, J = 7.2 Hz, 2H), 7.24 (d, J = 9 Hz, 2H), 6.68 (t, J = 6 Hz, 1H), 6.54 (d, J = 7.2 Hz , 1H), 5.79 (d, J = 10.2 Hz, 1H), 5.42 (d, J = 7.8 Hz, 1H), 5.14 (br, 1H), 4.21 (t, J = 3 Hz, 1H), 3.95-3.87 (m, 2H), 3.68 (s, 6H), 3.61-3.17 (m, 10H), 2.88 (br, 4H), 2.80-2.20 (m, 7H), 1.99-1.12 (m, 30H).

### Example 54. Methyl-6-(4-O-D-allosyl) benzoyl-9,10-dihydro-10-nitromethyl gambogate

a. Analogously to method D, starting from methyl gambogate. The intermediate was obtained and identified. b. Analogously to method E, starting from intermediate obtained from above. The intermediate was obtained and identified. c. Analogously to method F, starting from intermediate obtained from above. The title compound was obtained and identified. ¹H NMR (d₆-DMSO) δ 8.12 (d, J = 8.7 Hz, 2H), 7.33 (d, J = 7.5 Hz, 1H), 7.22 (d, J = 8.4 Hz, 2H), 6.49 (d, J = 10.2 Hz, 1H), 5.78 (d, J = 10.2 Hz, 1H), 5.42 (d, J = 5.1 Hz, 1H), 5.13 (m, 1H), 4.21 (s, 1H), 3.96-3 .85 (m, 3H), 3.76-3.40 (m, 9H), 3.30 (m, 2H), 2.86 (br, 9H), 2.51 (m, 1H), 2.31 (m, 1H), 2.01 (m, 2H), 1.87-1.25 (m, 26H).

### Example 55. Methyl-6-((4-(O-D-allosyl) benzoyl-L-alanylacyl)-9,10-dihydro-10-N-methylnaphthylamine gambogate

a. Analogously to method D, starting from methyl gambogate. The intermediate was obtained and identified. b. Analogously to method E, starting from intermediate obtained from above. The intermediate was obtained and identified. c. Analogously to method F, starting from intermediate obtained from above. The title compound was obtained and identified. IR (KBr, cm⁻¹) 3402, 2958, 1755, 1639, 1607, 1499, 1372, 1227, 1092, 1044; ¹H NMR (CDCl₃) δ 8.71 (d, J = 7.2 Hz, 1H), 8.13-7.46 (m , 13H), 7.22 (d, J = 8.7 Hz, 2H), 5.70 (m, 3H), 5.25-4.89 (m, 7H), 4.51 (m, 2H), 4.27 (m, 6H), 4.0-3.40 (br, 10H), 3.05 (s, 3H), 2.97 (s, 1H), 2.58 (m, 3H), 2.23 (s, 6H), 2.10-1.35 (m, 21H).

### Example 56. Methyl-6-phosphatea gambogate

Analogously to method D, starting from methyl gambogate. The title compound was obtained and identified. IR (KBr, cm⁻¹) 3446, 2969, 2855, 1736, 1712, 1652, 1592, 1459, 1384, 1366, 1326, 1235, 2277, 1140, 1051, 992, 913, 809; ¹H NMR (CDCl₃) δ 7.50 (d, J = 6.9 Hz, 1H), 6.72 (d, J = 10.1 Hz, 1H), 5.96 (t, J = 7.3 Hz, 1H), 5.48 (d, J = 10.1 Hz, 1H), 5.08 (m, 2H), 3.52 (m, 1H), 3.43 (s, 3H), 3.32 (m, 1H), 3.15 (m, 1H), 2.99 (m, 2H), 2.51 (d, J = 9.3 Hz, 1H), 2.31 (q, 1H), 2.01 (m, 2H), 1.73 (br, 5H), 1.69 (s, 3H), 1.68-127 (m, 21H).

### Example 57. Methyl-6-triphosphate gambogate

To a mixture of salicyloyl phosphorus chloride11.42 g (7 mmol) and Et₃N 0.97 ml in THF 25 ml were added methyl gambogate 3.0 g (4.6 mmol) and the mixture was stirred for 4 h at -40°C. The reaction mixture was extracted with ethyl acetate. The organic phase was evaporated to afford the intermediate 2.8 g from flash chromatography. To a product obtained from above 2 g (2.5 mmol), pyrophosphate 0.48 g and saturated ammonia solution 5 ml containing 2.54 g of pyridine in dichloromethane 25 ml and the mixture was stirred for 10 h at pH = 3 by HCl. The reaction mixture was extracted with ethyl acetate. The organic phase was evaporated to afford title compound 1.12 g from flash chromatography. ¹H NMR (d₆-DMSO) δ 7.52 (d, J = 6.9 Hz, 1H), 6.72 (d, J = 10.1 Hz, 1H), 5.95 (t, J = 7.3 Hz, 1H), 5.46 (d, J = 10.1 Hz, 1H), 5.06 (m, 2H), 3.50 (m, 1H), 3.43 (s, 3H), 3.30 (m, 1H), 3.13 (m, 1H), 2.99 (m, 2H), 2.50 ( d, J = 9.3 Hz, 1H), 2.31-2.01 (m, 7H), 1.73 (br, 5H), 1.68-127 (m, 24H).

### Example 58. Methyl-11-bromogambogate

Analogously to method A, starting from 11-bromogambogic acid. The title compound was obtained and identified. IR (KBr, cm⁻¹) 3443, 2976, 2929, 1736, 1713 , 1633, 1600, 1431, 1383, 1363, 1233, 1216, 1168, 1137, 1112; ¹H NMR (CDCl₃) δ 13.23 (s, 1H), 7.53 (d, J = 6.9 Hz, 1H), 7.39 (d, J = 7.8 Hz, 1H), 6.64 (m, J = 7.0 Hz, 1H), 6.01-5.92 (m, 1H), 5.56 (d, J = 10.5 Hz, 1H), 5.21-5.17 (br, 2H), 5.06-5.0 (m, 3H), 4.68 (m, 2H), 3.44 (s, 3H), 3.38 (m, 1H), 3.28 (m, 2H), 2.89-2.80 (m, 2H), 2.68-2.53 ( m, 2H), 2.31 (m, 1H), 1.78-1.18 (m, 21H).

**Examples 59-234** in Table 1

**TABLE 1**

| **Example** | **Chemical Structure** | **Formula** | **M. Weight** |
|---|---|---|---|
| **59** | 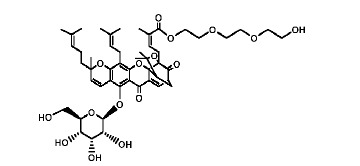 | **C₅₀H₆₆O₁₆** | **923** |
| **60** | 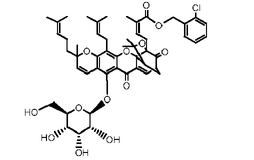 | **C₅₁H₅₉CIO₁₃** | **915** |
| **61** | 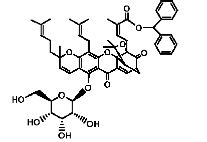 | **C₅₇H₆₄O₁₃** | **957** |
| **62** | 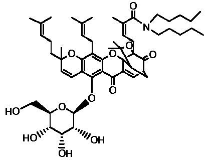 | **C₅₄H₇₅NO₁₂** | **930** |
| **63** | 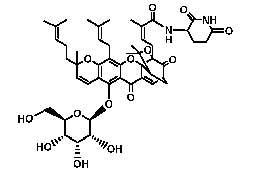 | **C₄₉H₆₀N₂O₁₄** | **901** |
| **64** | 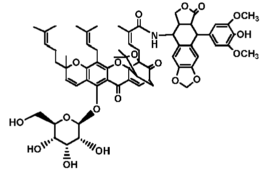 | **C₆₅H₇₃NO₁₉** | **1172** |
| **65** | 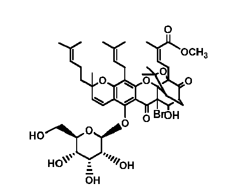 | **C₄₅H₅₇BrO₁₄** | **902** |
| **66** | 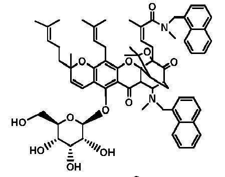 | **C₆₈H₇₈N₂O₁₂** | **1115** |
| **67** | 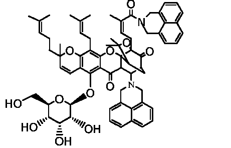 | **C₆₉H₇₆N₂O₁₂** | **1125** |
| **68** | 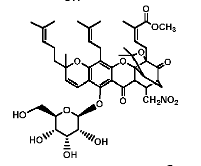 | **C₄₆H₅₉NO₁₅** | **866** |
| **69** | 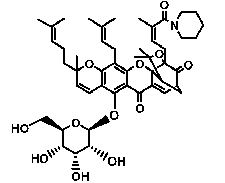 | **C₄₉H₆₃NO₁₂** | **858** |
| **70** | 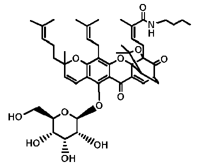 | **C₄₈H₆₃NO₁₂** | **846** |
| **71** | 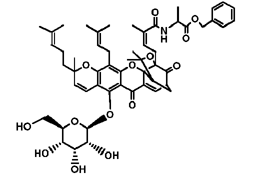 | **C₅₆H₆₃NO₁₄** | **952** |
| **72** | 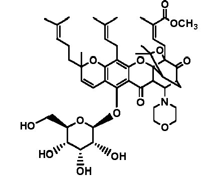 | **C₄₉H₆₃NO₁₄** | **892** |
| **73** | 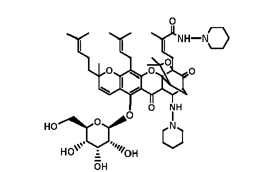 | **C₅₄H₇₆N₄O₁₂** | **973** |
| **74** | 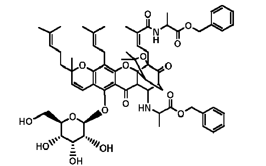 | **C₆₄H₇₈N₂O₁₆** | **1131** |
| **75** | 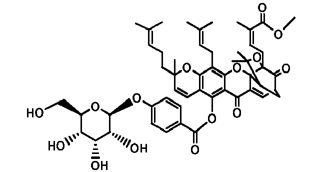 | **C₅₂H₆₀O₁₅** | **925** |
| **76** | 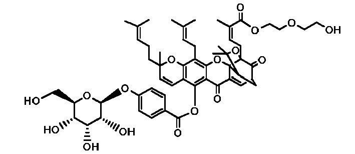 | **C₅₅H₆₆O₁₇** | **999** |
| **77** | 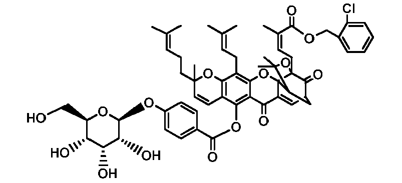 | **C₅₈H₆₃ClO₁₅** | **1036** |
| **78** | 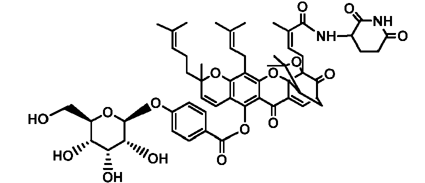 | **C₅₆H₆₄N₂O₁₆** | **1021** |
| **79** | 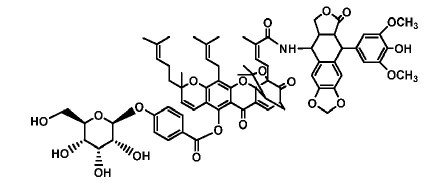 | **C₇₂H₇₇NO₂₁** | **1292** |
| **80** | 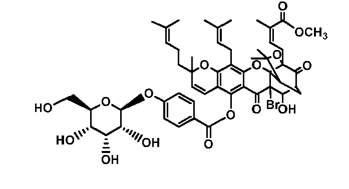 | **Cs₂H₆₁BrO₁₆** | **1021** |
| **81** | 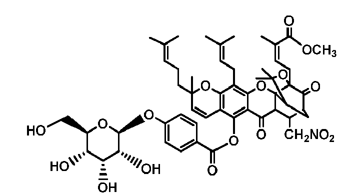 | **C₅₃H₆₃NO₁₇** | **986** |
| **82** | 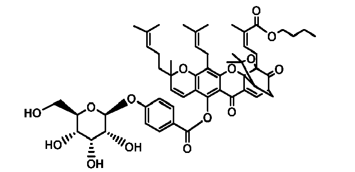 | **C₅₅H₆₆O₁₅** | **967** |
| **83** | 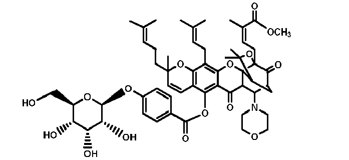 | **C₅₆H₆₉NO₁₆** | **1012** |
| **84** | 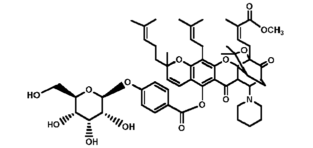 | **C₅₇H₇₁NO₁₅** | **1010** |
| **85** | 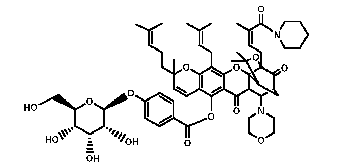 | **C₆₀H₇₆N₂O₁₅** | **1065** |
| **86** | 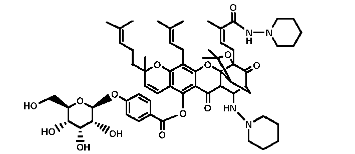 | **C₆₁H₈₀N₄O₁₄** | **1093** |
| **87** | 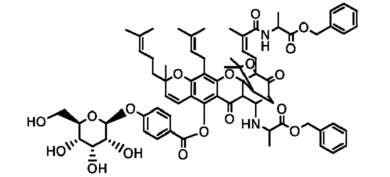 | **C₇₁H₈₂N₂0₁₈** | **1251** |
| **88** | 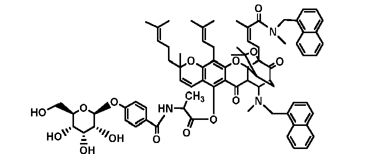 | **C₇₈H₈₇N₃O₁₅** | **1307** |
| **89** | 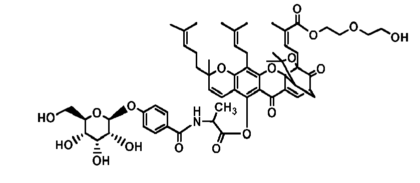 | **C₅₈H₇₁NO₁₈** | **1070** |
| **90** | 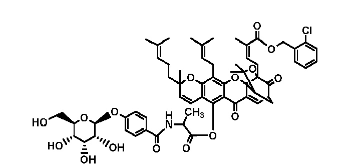 | **C₆₁H₆₈ClNO₁₆** | **1107** |
| **91** | 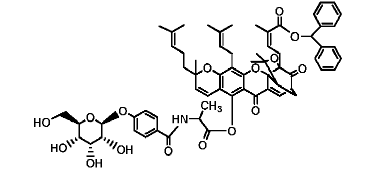 | **C₆₇H₇₃NO₁₆** | **1148** |
| **92** | 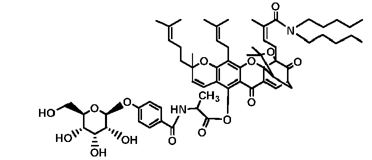 | **C₆₄H₈₄N₂O₁₅** | **1121** |
| **93** | 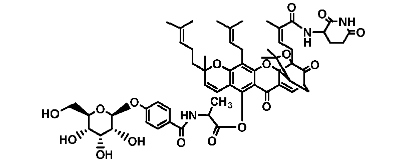 | **C₅₉H₆₉N₃O₁₇** | **1092** |
| **94** | 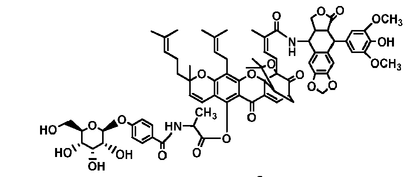 | **C₇₅H₈₂N₂O₂₂** | **1363** |
| **95** | 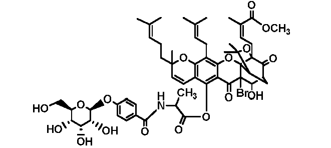 | **C₅₅H₆₆BrNO₁₇** | **1093** |
| **96** | 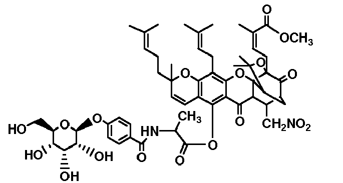 | **C₅₆H₆₈N₂O₁₈** | **1057** |
| **97** | 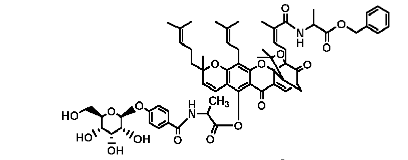 | **C₆₄H₇₄N₂O₁₇** | **1143** |
| **98** | 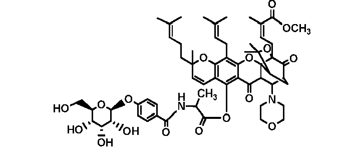 | **C₅₉H₇₄N₂O₁₇** | **1083** |
| **99** | 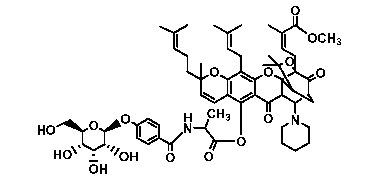 | **C₆₀H₇₆N₂O₁₆** | **1081** |
| **100** | 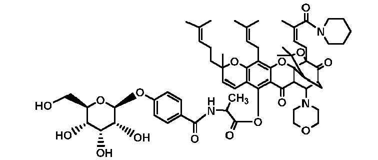 | **C₆₃H₈₁N₃O₁₆** | **1136** |
| **101** | 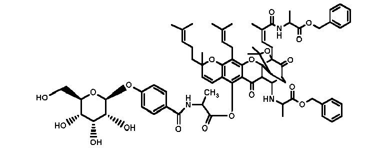 | **C₇₄H₈₇N₃O₁₉** | **1322** |
| **102** | 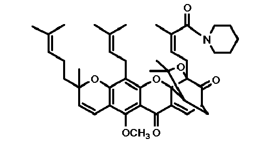 | **C₄₄H₅₅NO₇** | **710** |
| **103** | 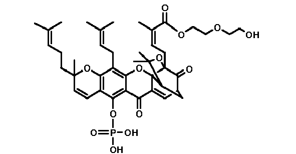 | **C₄₂H₅₃O₁₃P** | **797** |
| **104** | 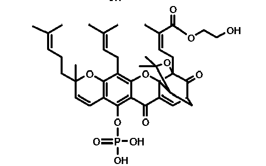 | **C₄₀H₄₉O₁₂P** | **753** |
| **105** | 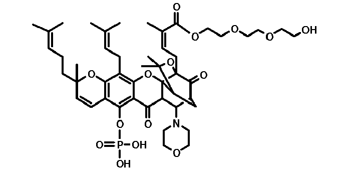 | **C₄₈H₆₆NO₁₅P** | **928** |
| **106** | 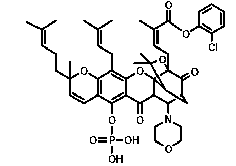 | **C₄₈H₅₇ClNO₁₂P** | **906** |
| **107** | 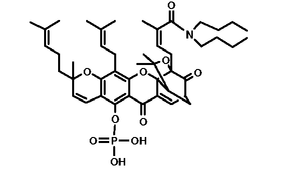 | **C₄₆H₆₂NO₁₀P** | **820** |
| **108** | 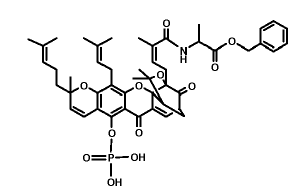 | **C₄₈H₅₆NO₁₂P** | **870** |
| **109** | 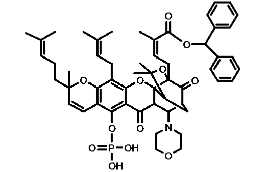 | **C₅₅H₆₄NO₁₂P** | **962** |
| **110** | 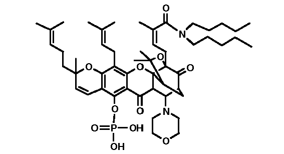 | **C₅₂H₇₅N₂O₁₁P** | **935** |
| **111** | 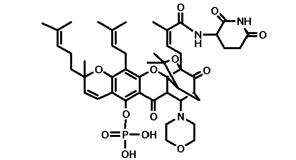 | **C₄₇H₆₀N₃O₁₃P** | **906** |
| **112** | 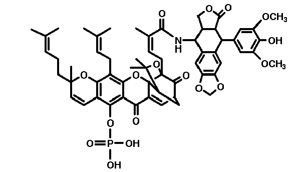 | **C₅₉H₆₄NO₁₇P** | **1090** |
| **113** | 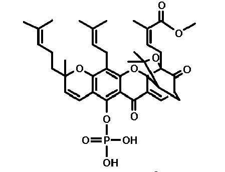 | **C₃₉H₄₇O₁₁P** | **723** |
| **114** | 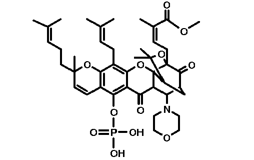 | **C₄₃H₅₆NO₁₂P** | **810** |
| **115** | 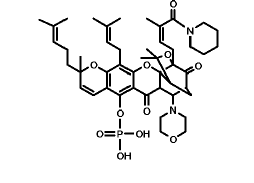 | **C₄₇H₆₃N₂O₁₁P** | **863** |
| **116** | 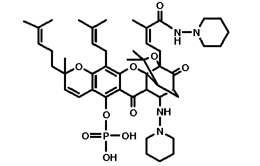 | **C₄₈H₆₇N₄O₁₀P** | **891** |
| **117** | 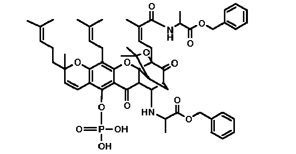 | **C₅₈H₆₉N₂O₁₄P** | **1049** |
| **118** | 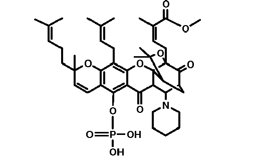 | **C₄₄H₅₈NO₁₁P** | **808** |
| **119** | 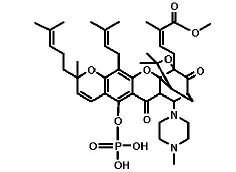 | **C₄₄H₅₉N₂O₁₁P** | **823** |
| **120** | 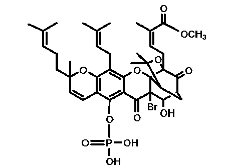 | **C₃₉H₄₈BrO₁₂P** | **820** |
| **121** | 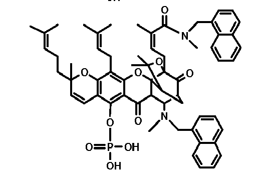 | **C₆₂H₆₉N₂O₁₀P** | **1033** |
| **122** | 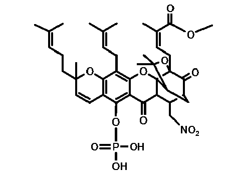 | **C₄₀H₅₀NO₁₃P** | **784** |
| **123** | 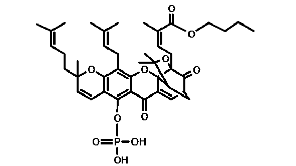 | **C₄₂H₅₃O₁₁P** | **765** |
| **124** | 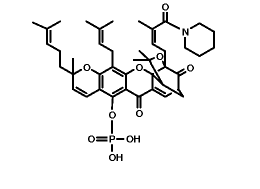 | **C₄₃H₅₄NO₁₀P** | **776** |
| **125** | 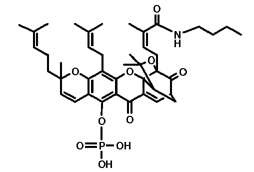 | **C₄₂H₅₄NO₁₀P** | **764** |
| **126** | 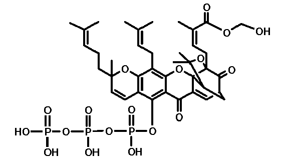 | **C₃₉H₄₉O₁₈P₃** | **899** |
| **127** | 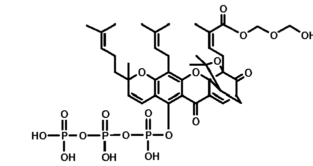 | **C₄oH₅₁O₁₉P₃** | **929** |
| **128** | 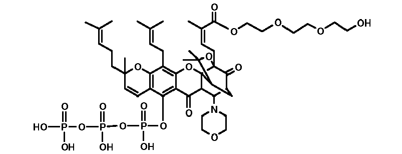 | **C₄₈H₆₈NO₂₁P₃** | **1088** |
| **129** | 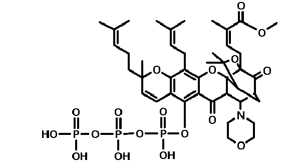 | **C₄₃H₅₈NO₁₈P₃** | **970** |
| **130** | 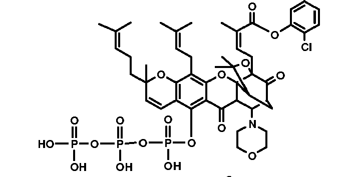 | **C₄₈H₅₉ClNO₁₈P₃** | **1066** |
| **131** | 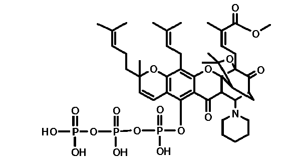 | **C₄₄H₆₀NO₁₇P₃** | **968** |
| **132** | 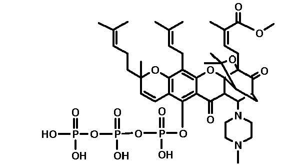 | **C₄₄H₆₁N₂O₁₇P₃** | **983** |
| **133** | 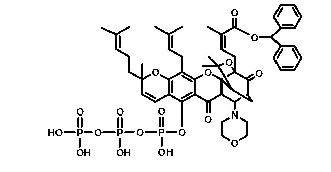 | **C₅₅H₆₆NO₁₈P₃** | **1122** |
| **134** | 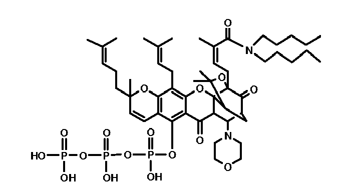 | **C₅₂H₇₇N₂O₁₇P₃** | **1095** |
| **135** | 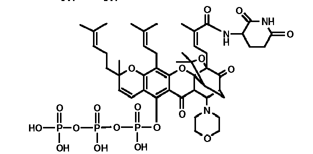 | **C₄₇H₆₂N₃O₁₉P₃** | **1066** |
| **136** | 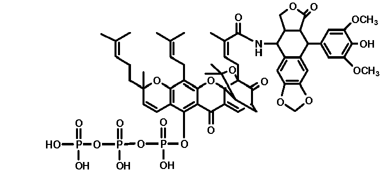 | **C₅₉H₆₆NO₂₃P₃** | **1250** |
| **137** | 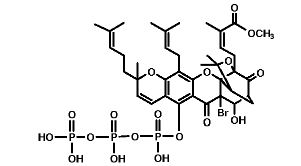 | **C₃₉H₅₀BrO₁₈P₃** | **980** |
| **138** | 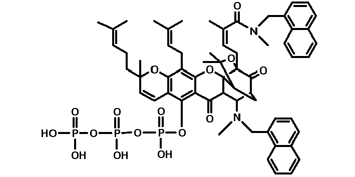 | **C₆₂H₇₁N₂O₁₆P₃** | **1193** |
| **139** | 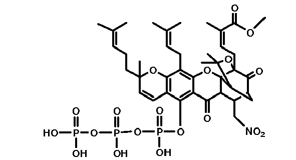 | **C₄₀H₅₂NO₁₉P₃** | **944** |
| **140** | 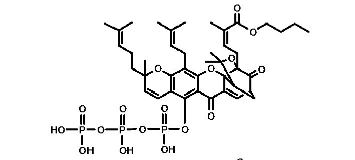 | **C₄₂H₅₅O₁₇P₃** | **925** |
| **141** | 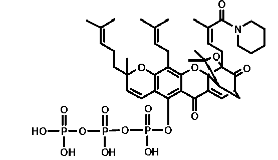 | **C₄₃H₅₆NO₁₆P₃** | **936** |
| **142** | 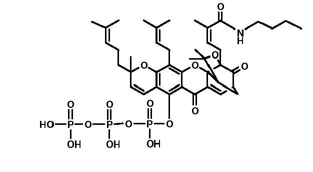 | **C₄₂H₅₆NO₁₆P₃** | **924** |
| **143** | 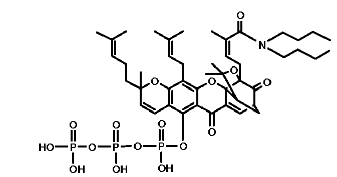 | **C₄₆H₆₄NO₁₆P₃** | **980** |
| **144** | 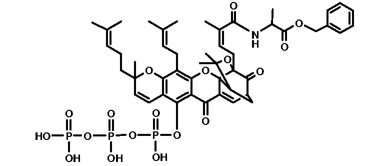 | **C₄₈H₅₈NO₁₈P₃** | **1030** |
| **145** | 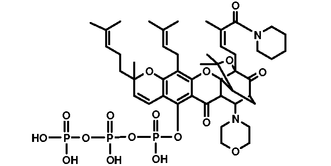 | **C₄₇H₆₅N₂O₁₇P₃** | **1023** |
| **146** | 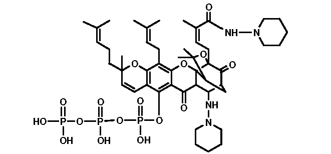 | **C₄₈H₆₉N₄O₁₆P₃** | **1051** |
| **147** | 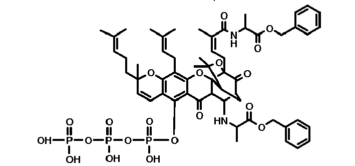 | **C₅₈H₇₁N₂O₂₀P₃** | **1209** |
| **148** | 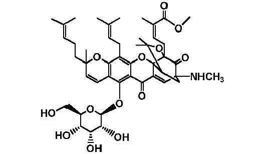 | **C₄₆H₅₉NO₁₃** | **834** |
| **149** | 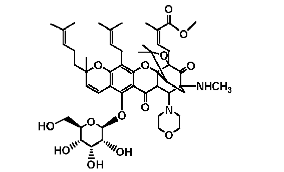 | **C₅₀H₆₈N₂O₁₄** | **921** |
| **150** | 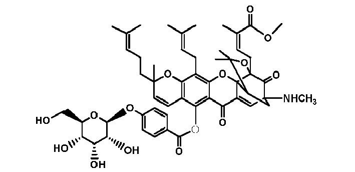 | **C₅₃H₆₃NO₁₅** | **954** |
| **151** | 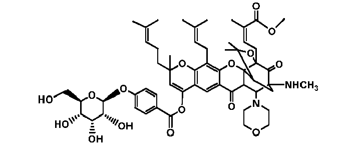 | **C₅₇H₇₂N₂O₁₆** | **1041** |
| **152** | 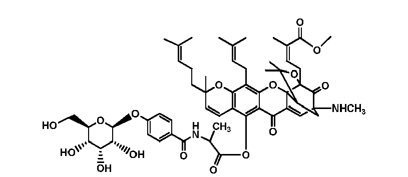 | **C₅₆H₆₈N₂O₁₆** | **1025** |
| **153** | 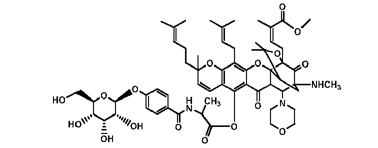 | **C₆₀H₇₇N₃O₁₇** | **1112** |
| **154** | 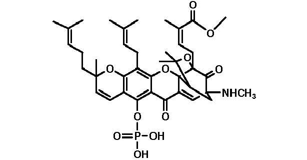 | **C₄₀H₅₀NO₁₁P** | **752** |
| **155** | 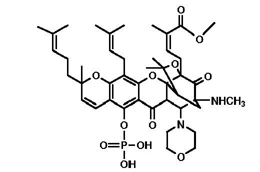 | **C₄₄H₅₉N₂O₁₂P** | **839** |
| **156** | 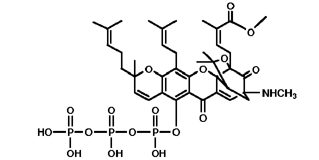 | **C₄₀H₅₂NO₁₇P₃** | **912** |
| **157** | 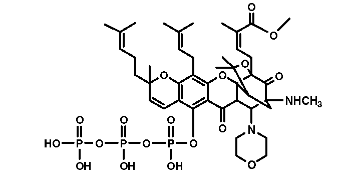 | **C₄₄H₆₁N₂O₁₈P₃** | **999** |
| **158** | 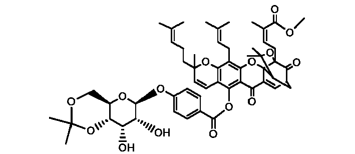 | **C₅₅H₆₄O₁₅** | **965** |
| **159** | 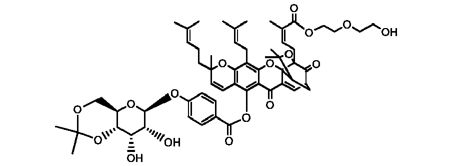 | **C₅₈H₇₀O₁₇** | **1039** |
| **160** | 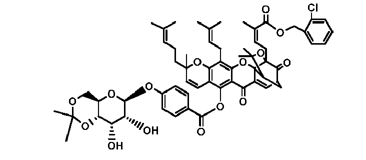 | **C₆₁H₆₇ClO₁₅** | **1076** |
| **161** | 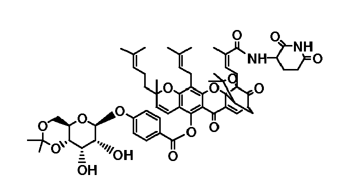 | **C₅₉H₆₈N₂O₁₆** | **1061** |
| **162** | 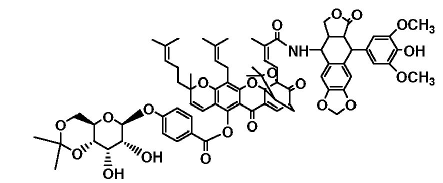 | **C₇₅H₈₁NO₂₁** | **1332** |
| **163** | 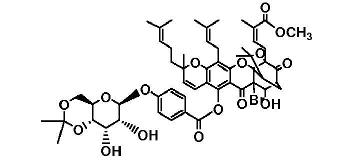 | **C₅₅H₆₅BrO₁₆** | **1062** |
| **164** | 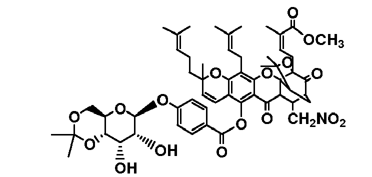 | **C₅₆H₆₇NO₁₇** | **1026** |
| **165** | 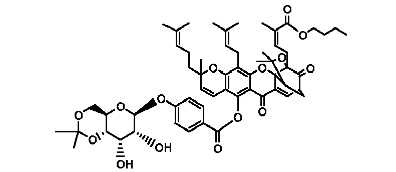 | **C₅₈H₇₀O₁₅** | **1007** |
| **166** | 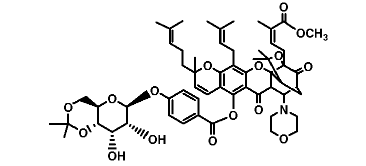 | **C₅₉H₇₃NO₁₆** | **1052** |
| **167** | 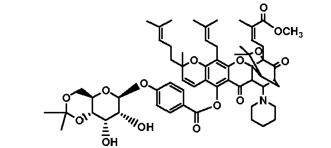 | **C₆₀H₇₅NO₁₅** | **1050** |
| **168** | 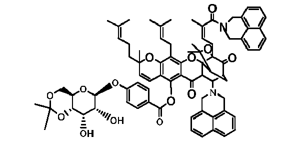 | **C₇₉H₈₄N₂O₁₄** | **1286** |
| **169** | 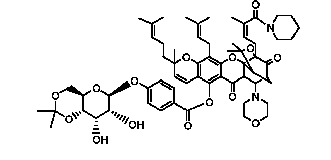 | **C₆₃H₈₀N₂O₁₅** | **1105** |
| **170** | 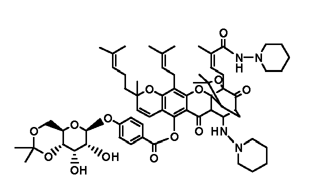 | **C₆₄H₈₄N₄O₁₄** | **1133** |
| **171** | 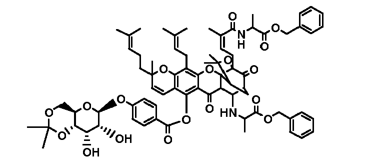 | **C₇₄H₈₆N₂O₁₈** | **1291** |
| **172** | 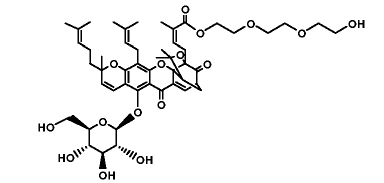 | **C₅₀H₆₆O₁₆** | **923** |
| **173** | 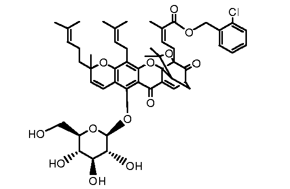 | **C₅₁H₅₉ClO₁₃** | **915** |
| **174** | 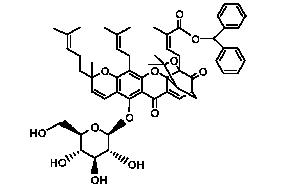 | **C₅₇H₆₄O₁₃** | **957** |
| **175** | 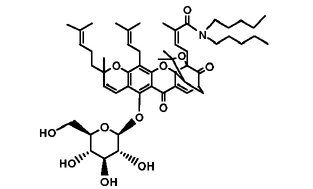 | **C₅₄H₇₅NO₁₂** | **930** |
| **176** | 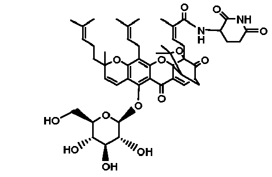 | **C₄₉H₆₀N₂O₁₄** | **901** |
| **177** | 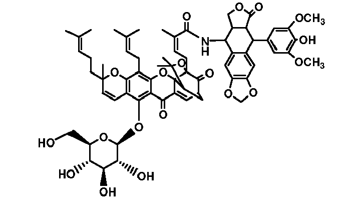 | **C₆₅H₇₃NO₁₉** | **1172** |
| **178** | 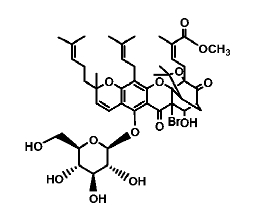 | **C₄₅H₅₇BrO₁₄** | **902** |
| **179** | 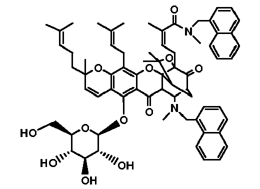 | **C₆₈H₇₈N₂O₁₂** | **1115** |
| **180** | 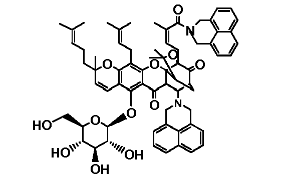 | **C₆₉H₇₆N₂O₁₂** | **1125** |
| **181** | 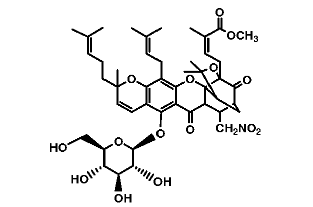 | **C₄₆H₅₉NO₁₅** | **866** |
| **182** | 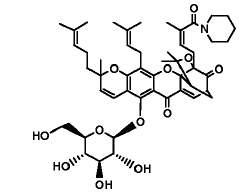 | **C₄₉H₆₃NO₁₂** | **858** |
| **183** | 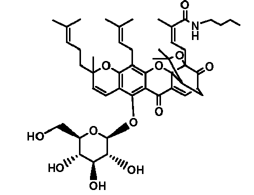 | **C₄₈H₆₃NO₁₂** | **846** |
| **184** | 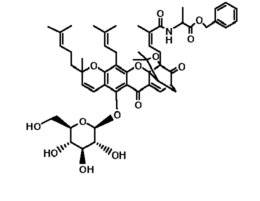 | **C₅₆H₆₃NO₁₄** | **952** |
| **185** | 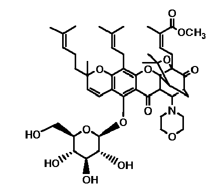 | **C₄₉H₆₃NO₁₄** | **892** |
| **186** | 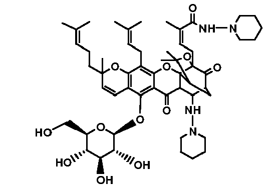 | **C₅₄H₇₆N₄O₁₂** | **973** |
| **187** | 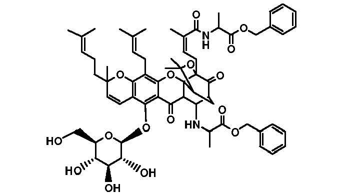 | **C₆₄H₇₈N₂O₁₆** | **1131** |
| **188** | 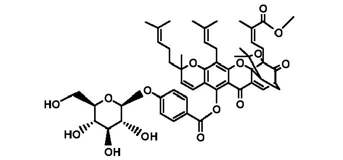 | **C₅₂H₆₀O₁₅** | **925** |
| **189** | 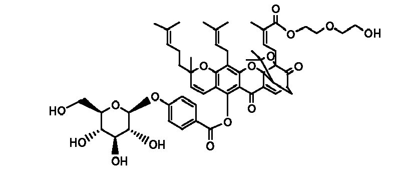 | **C₅₅H₆₆O₁₇** | **999** |
| **190** | 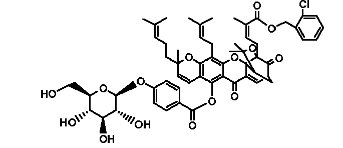 | **C₅₈H₆₃ClO₁₅** | **1036** |
| **191** | 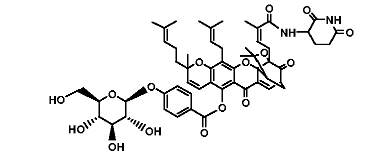 | **C₅₆H₆₄N₂O₁₆** | **1021** |
| **192** | 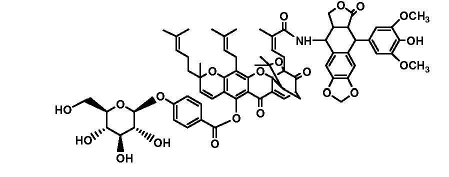 | **C₇₂H₇₇NO₂₁** | **1292** |
| **193** | 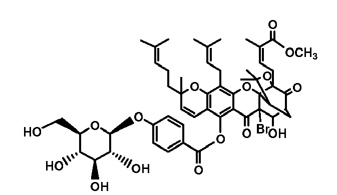 | **Cs₂H₆₁BrO₁₆** | **1021** |
| **194** | 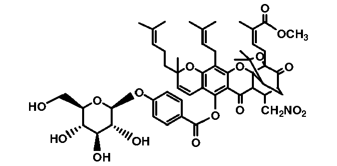 | **C₅₃H₆₃NO₁₇** | **986** |
| **195** | 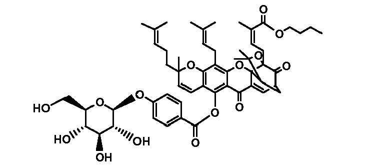 | **C₅₅H₆₆O₁₅** | **967** |
| **196** | 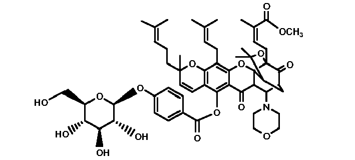 | **C₅₆H₆₉NO₁₆** | **1012** |
| **197** | 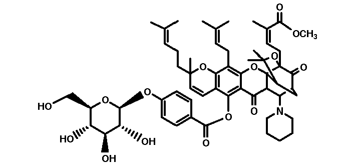 | **C₅₇H₇₁NO₁₅** | **1010** |
| **198** | 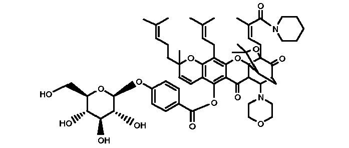 | **C₆₀H₇₆N₂O₁₅** | **1065** |
| **199** | 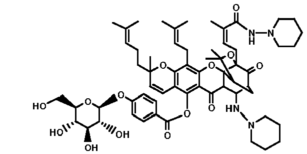 | **C₆₁H₈₀N₄O₁₄** | **1093** |
| **200** | 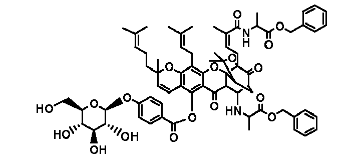 | **C₇₁H₈₂N₂O₁₈** | **1251** |
| **201** | 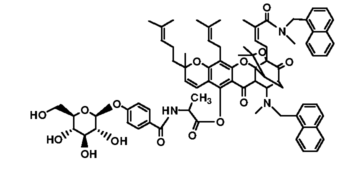 | **C₇₈H₈₇N₃O₁₅** | **1307** |
| **202** | 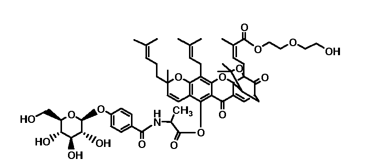 | **C₅₈H₇₁NO₁₈** | **1070** |
| **203** | 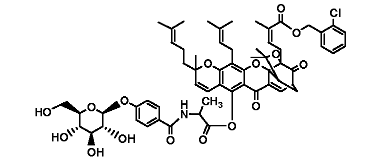 | **C₆₁H₆₈CINO₁₆** | **1107** |
| **204** | 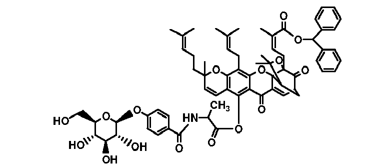 | **C₆₇H₇₃NO₁₆** | **1148** |
| **205** | 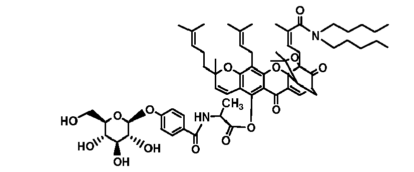 | **C₆₄H₈₄N₂O₁₅** | **1121** |
| **206** | 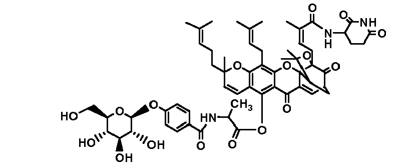 | **C₅₉H₆₉N₃O₁₇** | **1092** |
| **207** | 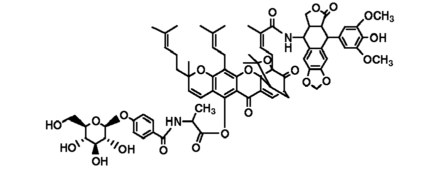 | **C₇₅H₈₂N₂O₂₂** | **1363** |
| **208** | 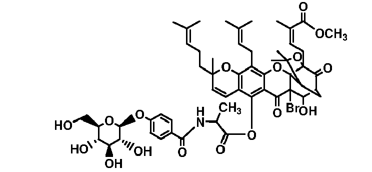 | **C₅₅H₆₆BrNO₁₇** | **1093** |
| **209** | 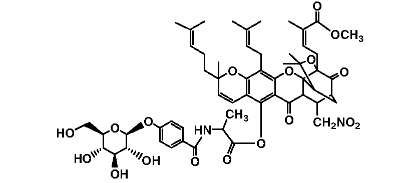 | **C₅₆H₆₈N₂O₁₈** | **1057** |
| **210** | 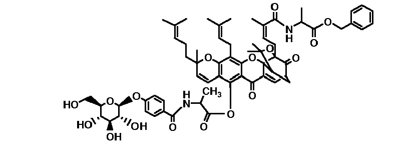 | **C₆₄H₇₄N₂O₁₇** | **1143** |
| **211** | 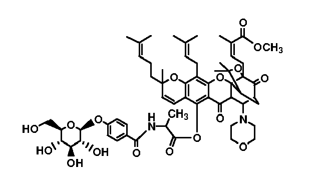 | **C₅₉H₇₄N₂0₁₇** | **1083** |
| **212** | 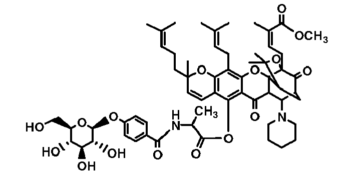 | **C₆₀H₇₆N₂O₁₆** | **1081** |
| **213** | 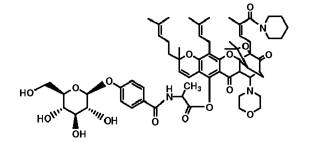 | **C₆₃H₈₁N₃O₁₆** | **1136** |
| **214** | 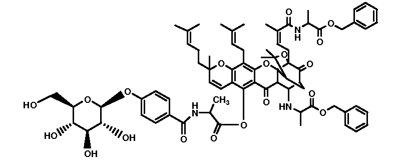 | **C₇₄H₈₇N₃O₁₉** | **1322** |
| **215** | 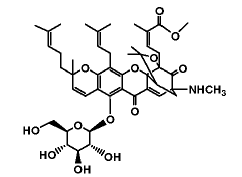 | **C₄₆H₅₉NO₁₃** | **834** |
| **216** | 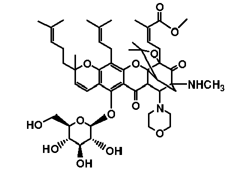 | **C₅₀H₆₈N₂O₁₄** | **921** |
| **217** | 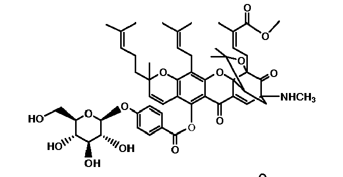 | **C₅₃H₆₃NO₁₅** | **954** |
| **218** | 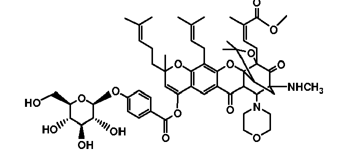 | **C₅₇H₇₂N₂O₁₆** | **1041** |
| **219** | 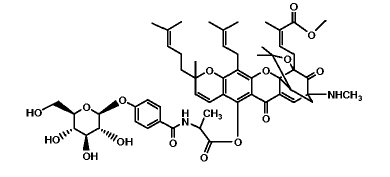 | **C₅₆H₆₈N₂O₁₆** | **1025** |
| **220** | 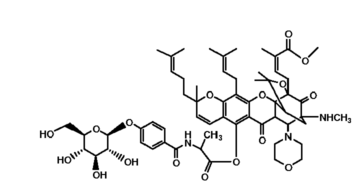 | **C₆₀H₇₇N₃O₁₇** | **1112** |
| **221** | 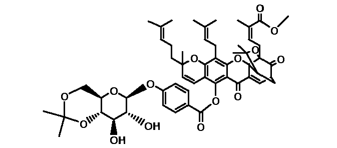 | **C₅₅H₆₄O₁₅** | **965** |
| **222** | 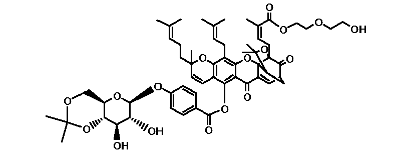 | **C₅₈H₇₀O₁₇** | **1039** |
| **223** | 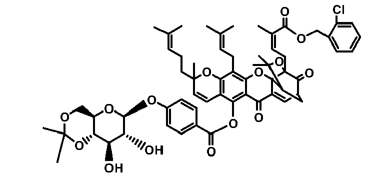 | **C₆₁H₆₇ClO₁₅** | **1076** |
| **224** | 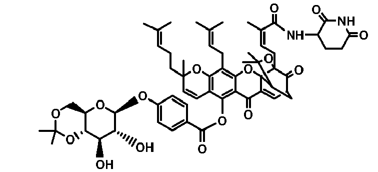 | **C₅₉H₆₈N₂O₁₆** | **1061** |
| **225** | 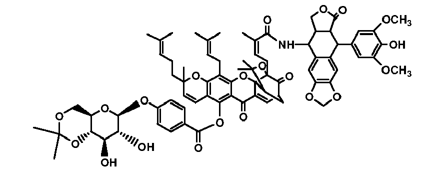 | **C₇₅H₈₁NO₂₁** | **1332** |
| **226** | 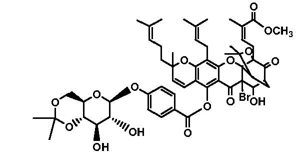 | **C₅₅H₆₅BrO₁₆** | **1062** |
| **227** | 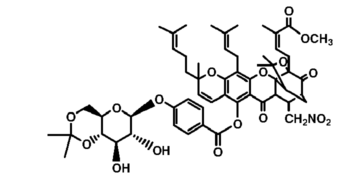 | **C₅₆H₆₇NO₁₇** | **1026** |
| **228** | 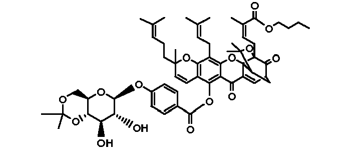 | **C₅₈H₇₀O₁₅** | **1007** |
| **229** | 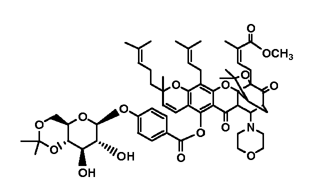 | **C₅₉H₇₃NO₁₆** | **1052** |
| **230** | 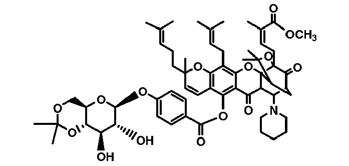 | **C₆₀H₇₅NO₁₅** | **1050** |
| **231** | 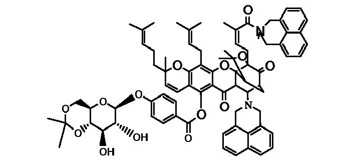 | **C₇₉H₈₄N₂O₁₄** | **1286** |
| **232** | 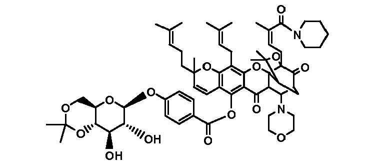 | **C₆₃H₈₀N₂O₁₅** | **1105** |
| **233** | 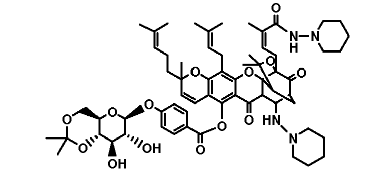 | **C₆₄H₈₄N₄O₁₄** | **1133** |
| **234** | 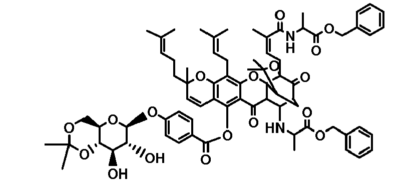 | **C₆₄H₈₄N₄O₁₄** | **1133** |

### Preparation of Injection

### Example 235. Preparation of Injection 1.

Compound 25(example 25) 5.0 g, 1,2-propanediol 1200 ml and Tween 80 100 ml were dissolved and the injection water was added up to total volume of 5000 ml. The solution was filtered with 0.22 µm membrane filter and sterilized for 30 min at 100 °C to obtain 1000 preparation of injection 5 mg / 5 ml.

### Example 236. Preparation of Injection 2.

Compound 26 (example 26) 8.0 g, DMSO 50 ml, 1,2-propanediol 100 ml and Tween 80 100 ml were dissolved and the injection water was added up to total volume of 5000 ml. The solution was filtered with 0.22 µm membrane filter and sterilized 30 min at 100 °C to obtain 1000 preparation of injection 8 mg / 5 ml.

### BIOLOGICAL ACTIVITY

### Example 237. In vitro anti-cancer cell experiment

### Methods

a. Cell lines: Human pancreatic cancer cell line Panc-1, human colorectal cancer cell line HT₂₉ and human lung cancer cell line NCI-H₄₆₀; the medium: s DMEM (Gibco BRL), containing 10% fetal calf serum (Gibco BRL) and 2 mM L-glutamine (Gibco BRL).

b. Test samples: example compounds 3, 18, 25 and 27.
The samples were dissolved in dimethyl sulfoxide (DMSO, Sigma, United States) and medium was added to the final concentration of 0.5%. Cisplatin was as positive control of (CDDP, purity 96%, from Kunming Institute of Precious Metals).

c. Method: cells were digested with trypsin and dispersed into single cells in the medium containing penicillin (25 U/m1) and streptomycin (25 µg/ml). The cells were seeded in 96-well culture plates (Corning Incorporated), at 37 °C, in a humidifield atosphere with 5% CO₂ present for 24 hours. The culture medium was removed, 1-100 µm test compounds were added, cultured for 48 hours. Culture medium was removed and thiophene Wow blue (MTT, USA Sigma products) was added. The result was assayed by SK601-based microplate reader (Japan Seikagaku company's products), 570 nm/630 nm optical density (OD).

Calculation of cell viability: (Experimental group OD / control OD) x 100%; Positive control CDDP was treated in the same way.

### Results

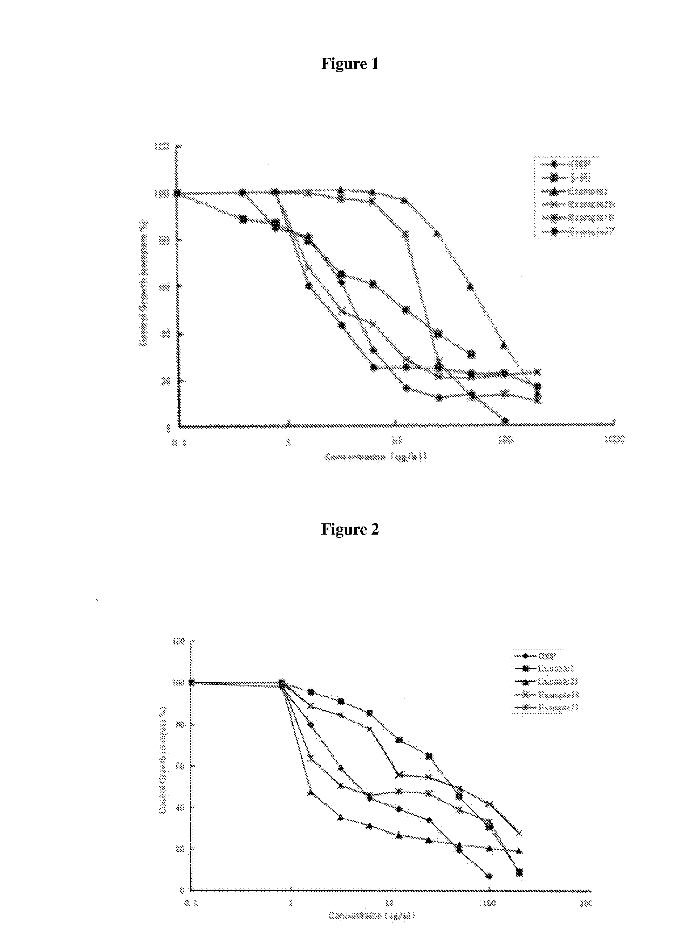

Inhibition of colorectal cancer: as shown in Figure 1, and table 1 four test compounds showed anti-proliferative effect on HT₂₉. Example compounds 27 and 25 showed significant effect of anti-proliferate on HT₂₉ at low IC₅₀ (the compound concentration producing 50 % inhibition of colony formation) values, respectively, 1.19 µg / ml (P < 0.05) and 3.75 µg / ml (P < 0.05) than conventional 5-FU and Cisplatin.

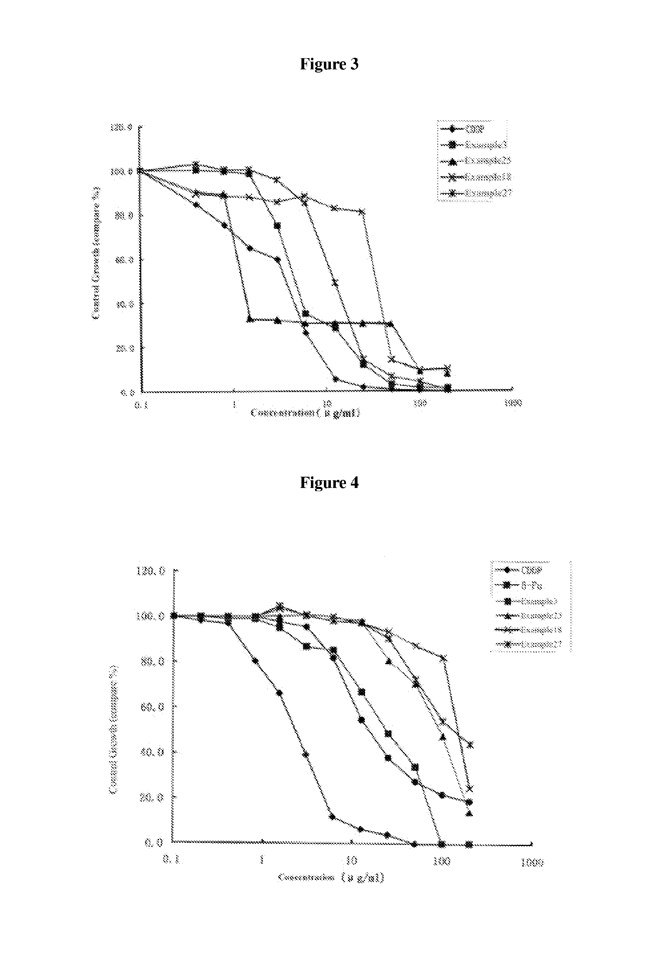

Inhibition of pancreatic cancer: as shown in Figure 3, and table 1 four test compounds showed anti-proliferative effect on Panc-1. Example compounds 3 showed anti-proliferative effect on Panc-1 at IC₅₀ (the compound concentration producing 50% inhibition of colony formation) values 23.4 µg / ml (P < 0.05) close to conventional 5-FU.

Inhibition of lung cancer: as shown in Figure 4, and table 1 four test compounds showed anti-proliferative effect on NCI-H₄₆₀. Example compounds 3 and 18 and 27 showed significant effect of anti-proliferate on NCI-H₄₆₀ at low IC₅₀ (the compound concentration producing 50 % inhibition of colony formation) values, respectively, 6.18 µg / ml (P < 0.05) and 4.73 µg / ml (P < 0.05) than conventional 5-FU.

**Table 1. IC₅₀ (nM)**

| **Example** | **HT₂₉** | **HT₂₉** | **NCl-H₄₆₀** | **Panc-1** |
|---|---|---|---|---|
| 3 | 76.9 | 149.4 | 13.9 | 52.8 |
| 18 | 111.9 | 49.8 | 88.9 | 329.9 |
| 25 | 1.6 | 8.7 | 10.9 | 343.3 |
| 27 | 17.4 | 2.7 | 29.0 | 54.5 |
| CDDP | 22.6 | 4.26 | 8 | 7.2 |
| 5-FU | | 92.3 | | 193.0 |

### Example 238. Efficacy studies of gambogic acid glycoside analogs in mice

Test samples: example compounds 18, 24, 25, 26 and 27

Test animals: Kunming kinds of healthy mice (19-21 g), 10 mice (5 male and 5 female) / group, from Beijing Institute of Military Medical Sciences Animal Center.

Tumor strains: mice sarcoma S₁₈₀ for ascites passaged from Beijing Academy of Military Medical Institute of Pharmacology.

### Methods

Xenografts cultured S₁₈₀ tumor cells were implanted subcutaneously into the flank region of mice and tumors were allowed to grow to the desired average size of 100 mg. The mice were randomized into control and treatment groups with 10 mice per group. The control group was injected with the vehicle used to dissolve the drug. Other groups received the test compounds (example compound 18, 24, 25, 26 and 27) and positive group, cyclophosphamide (CTX) and 5-fluorouracil (5-FU) at the dose and schedule as indicated in Table VI. Injections were I.V. via the tail vein. Tumor measurements were taken every other day 20 % tumor growth inhibition which was not statistically significant.

### Results

The in vivo experimental data showed anti-tumor efficacy of example compounds 24 (one dose), example compound 25 (three doses), example compound 26 (three doses) and example compound 27 (one dose) are statistically significant.

**Table 2. Growth Inhibition of S₁₈₀ sarcoma**

| **Example Compound** | **mg/kg** | **body weight / g** | | | **Tumor weight(g)** | **Inhibition rate(%)** | **P** |
|---|---|---|---|---|---|---|---|
| | | **Before ad.** | **After ad.** | **Without tumor** | | | |
| **Control** | **-** | **23.87±1.70** | **27.05±3.98** | **24.52±3.54** | **2.41±1.22** | **-** | **-** |
| **CTX** | **25** | **22.64±1.28** | **25.44±2.92** | **23.94±2.60** | **1.24±0.50** | **48.6** | **0.0002**** |
| **5-FU** | **15** | **23.07±1.75** | **28.66±2.89** | **27.19±2.88** | **1.37±.033** | **43.2** | **0.0093**** |
| **18** | **10** | **27.32±2.2** | **26.08±2.8** | **25.06±2.09** | **1.14±0.42** | **52.5** | **0.04*** |
| **18** | **5** | **22.80±1.73** | **27.91±4.08** | **26.49±4.62** | **1.44±0.39** | **40.3** | **0.16** |
| **24** | **10** | **21.67±1.26** | **19.77±1.88** | **18.89±1.45** | **0.94±0.26** | **61.0** | **0.0001**** |
| **25** | **4** | **22.38±1.80** | **21.47±3.69** | **20.77±3.37** | **0.84±0.35** | **65.2** | **0.0024**** |
| **25** | **2** | **22.36±1.30** | **22.75±3.51** | **21.69±3.13** | **1.00±0.34** | **58.3** | **0.0012**** |
| **25** | **1** | **22.59±1.36** | **24.75±2.56** | **23.27±2.16** | **1.34±0.47** | **44.6** | **0.01**** |
| **26** | **12** | **25.58±2.48** | **26.71±5.56** | **26.07±5.40** | **0.74±0.11** | **69.3** | **0.0001**** |
| **26** | **8** | **24.41±2.28** | **21.42±0.88** | **20.56±0.54** | **0.96±0.32** | **60.2** | **0.001**** |
| **26** | **4** | **25.89±2.62** | **23.71±3.85** | **22.54±3.42** | **1.12±0.40** | **53.7** | **0.0001**** |
| **27** | **30** | **19.07±1.40** | **20.22±2.11** | **18.67±2.08** | **1.52±0.30** | **37.0** | **0.007**** |
| **27** | **25** | **20.90±1.06** | **20.83±1.36** | **19.00±1.39** | **1.63±0.47** | **32.5** | **0.018*** |

| | | | | | | | |
|---|---|---|---|---|---|---|---|
| Before ad.: before administration; After ad.: after administration | | | | | | | |

* P < 0.01: compared with the control group significantly difference; ** p < 0.001: compared with the control group was very significant difference. Inhibition rate more than 40% of the sample was statistically significant better than control group.

### Example 239 Chronic Toxicity

### Methods

40 mice were randomly divided into four groups, control group and three dose groups (2 mg/kg, 4 mg/kg, and 8 mg/kg) of compound 26. Animals were given an injection in the tail vein with approximately 100 µl volume of compound formulation or vehicle formulation. Mice body weights were measured daily along with daily observation for 30 **days.**

### Results

(1) The general behavior, body weight and organ weight of animal.

(2) Blood test: white blood cells (WB), red blood cells (RB), hemoglobin (Hb), alanine aminotransferase (ALT), aspartate aminotransferase (AST), alkaline phosphatase (ALP), serum uric acid (UA), blood urea (UREA) and creatinine (Cr).

(3) Pathological examination: heart, liver, spleen, lung and kidney.

(a). Heart: there were no nervous of liver capsule, no enlargement, no reduced and no turbid swelling of liver volume and no abnormal of heart tissue of three dose groups (Figure 5-2, 5-3) comparing to control group (Figure 5-1).

Table 1 (b). Liver: there were normal arrangement of liver cells cable, normal morphology and nuclear of liver cell, no cloudy, no swelling, no balloon-like degeneration or non-necrotic change, no periportal cholestasis, no fibrous tissue proliferation and no inflammatory cell infiltration and no abnormal tissue in rat liver slices of three dose groups (Figure 5-5, 5-6) comparing to control group (Figure 5-4).

(c). Spleen: there were no clearly pathological change, nearly the same number with the control of spleen nodules, no clearly proliferation and no back-shape changes, no spleen sinus congestion and no abnormal tissue in rat spleen slices of three dose groups (see 5-8, 5-9) comparing to control group (Figure 5-7).

(d). Lung: there were no red blood cells, no white blood cells, and no fibrin exudation in alveolar, no inflammatory infiltration, no congestion and no fibrous tissue hyperplasia, no inflammatory cell infiltration of bronchial wall, no clearly change of bronchial cavity effusion and no abnormal tissue in rat lung slices of three dose groups (Figure 5-11, 5-12) comparing to control group (Figure 5-10).

(e). Renal: there were no clearly pathological changes in glomerular, no reduce and proliferative changes, no leakage of renal capsule, no granular degeneration and no necrosis of renal tubular epithelial cells, no clear exudation of tubular cavity, no tube and epithelial cells, normal size tubular cavity and no abnormal tissue in rat renal slices of three dose groups (Figure 5-14, 5-15) comparing to control group (Figure 5-13).

### Conclusions

Except a slightly incretion of alanine aminotransferase (ALT) of high dose group and recovery to normal level, above results showed that there were no toxic pathological changes of the heart, liver, spleen, lung and kidney of three dose mice groups. Therefore a continuous administration of the test compound for 30 days with three doses did not cause the organ clear damages.

## Claims

1. A compound of the formula **I**: 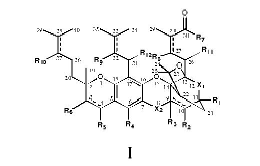 or stereoisomers, tautoers, prodrug, pharmaceutically acceptable salts, complex salts or solvates thereof, wherein:
The dotted lines, are optionally substituted single bonds, optionally substituted double bond or a optionally substituted heterocyclic group containing carbon, oxygen, sulfur or nitrogen element;
R₁, R₂, R₃, R₄, R₅, R₆, R₇, R₈, R₉, R₁₀, R₁₁ or/and R₁₂ is, independently at each occurrence, optionally substituted glycosyl, optionally substituted multi-hydroxyl, optional substituent oxy, optional substituent containing oxygen, sulfur, nitrogen or phosphorus element;
Glycosyl is D- or L-configuration and its glycoside bond is C-C or C-hetero bond connection, including 1-8 optionally substituted glycosyl or optional substituent glycosyl;
Multi-hydroxyl is, independently at each occurrence, 1-10 optionally substituted hydroxyl group of alkyl, aryl, cyclic or heterocyclic, where contains optionally substituted one or combination of amino acid, acyloxy, phosphoric acid oxy, alkoxy, alkyl, alicyclic, aryl ring, aliphatic heterocyclic or aryl heterocyclic;
Substituent oxy is, independently at each occurrence, optionally substituted acyloxy, 1-4 optionally substituted phosphoryloxy, optionally substituted alkoxy, optionally substituted aryloxy or optionally substituted heterocyclic oxy;
Substituent containing oxygen, sulfur, nitrogen or phosphorus element is, independently at each occurrence, optionally substituted saturated, optionally substituted unsaturated C₁₋₁₀ alkyl, 1-4 optionally substituted double bond, optionally substituted triple bond, optional substituent of saturated or unsaturated C₁₋₁₀ alicyclic, arylcyclic and heterocyclic group,
where contains cyclic one or combination of oxygen, sulfur, nitrogen or phosphorus element, saturated or unsaturated 3-10 membered alicyclic, aryl cyclic, multi-cyclic, aliphatic heterocyclic, aryl heterocyclic or fused heterocyclic;
Substituent is, independently at each occurrence, optionally substituted one or combination of suturated or unsaturated C₁₋₁₀ alkyl, 1-4 double bond, 1-4 triple bond, saturated or unsaturated C₁₋₁₀ alicyclic, C₁₋₁₀, aryl and C₁₋₁₀, heterocyclic group;
wherein:
X₁and X₂ are, independently at each occurrence, C=O,C=Rb-Ra, CHOH, CHORb, CHRb or substituent, where Rb contains, independently at each occurrence, C, N or P element; Ra is [], []2, optionally substituted straight-alkyl, optionally substituted branched-alkyl, C₁₋₁₀ optionally substituted saturated alkyl, optionally substituted 1-4 double bond, optionally substituted 1-4 triple bond, optionally substituted unsaturated alkyl, optionally substituted saturated or unsaturated alicyclic, optionally substituted arylcyclic, optionally substituted aryl or optionally substituted heterocyclic, where contains hydroxyl, halogen, oxygen, nitrogen, sulfur or phosphorus element;
wherein:
Substituent is, independently at each occurrence, C₁₋₁₀ optionally substituted saturated or unsaturated alkyl, 1-4 optionally substituted double bond, optionally substituted 1-4 triple bond, optionally substituted saturated or unsaturated C₁₋₁₀ alicyclic, optionally substituted aryl group or optionally substituted heterocyclic, where contains optionally substituted one or combination of oxygen, sulfur, nitrogen, phosphorus element, halogen, saturated or unsaturated 3-10 membered alicyclic, aryl cyclic, multi-cyclic, aliphatic heterocyclic, aryl heterocyclic, fused heterocyclic, 1-8 glycosyl,
substituent glycosyl, amino acid, acyloxy, phosphoryloxy, alkoxy, heterocyclicoxy and multi-hydroxyl of alkyl, ring, aryl or heterocyclic.

2. A compound according to the claim **1**, wherein:
R₁₂ R₁, R₂, R₅, R₆, R₈, R₉, R₁₀, R₁₁ or/and is, independently at each occurrence, H, halogen or XRa; where XRa is unsubstituted or substituted group containing C, O, S, Se, N, and/or P element.

3. A compound according to the claim **1**, wherein:
R₃ is XaRa electrophilic substituent, where Xa is, independently at each occurrence, unsubstituted or substituted group containing C, S, P, and/or Si element.

4. A compound according to the claim **1,** wherein:
R4 is, independently at each occurrence, optional substituent of 1-8 glycosyl, multi-hydroxyl, substituted multi-hydroxyl, 1-5 amino acid, 1-4 phosphate, acyloxy, phosphoric, sulfonyloxy, alkoxy, aryloxy, heterocyclic oxy, alkyl, alicyclic, aryl cyclic, aliphatic heterocyclic or aryl, heterocyclic containing oxygen, sulfur, nitrogen or phosphorus element, where glycosyl is D-and L-configuration with C-C or C-hetero bond of glycoside;
wherein:
Multi-hydroxyl is, independently at each occurrence, optionally substituted one or combination of 1-20 hydroxyl alkyl of cyclic alkyl, aryl, heterocyclic, amino acid, acryloxy, phosphoryloxy, alkoxy or heterocyclic oxy, where contains alkyl, alicyclic, arylcyclic, aliphatic heterocyclic or aryl heterocyclic;
Substituted multi-hydroxyl is, independently at each occurrence, optionally substituted multi-hydroxyl substituted by saturated or unsaturated C₁₋₁₀ alkyl, 1-4 double bond or triple bond, saturated or unsaturated C₁₋₁₀ alicyclic, alkyl and aryl, where contains optionally substituted one or combination of oxygen, sulfur, nitrogen, phosphorus element, halogen, amino acid, acyloxy, phosphoryloxy, alkoxy, saturated or unsaturated 3-10 membered alicyclic, acryl cyclic, multi-cyclic, aliphatic heterocyclic, aryl heterocyclic or fused heterocyclic.

5. A compound according to the claim **1** and **4,** wherein:
1-8 glycosyl is, independently at each occurrence, optionally substituted C₃₋₈ saccharide, optionally substituted monosaccharide, optionally substituted disaccharide, optionally substituted trisaccharide and/or optionally substituted polysaccharide.

6. A compound according to claim **5,** wherein:
C₃₋₈ saccharide, monosaccharide, disaccharide, trisaccharide, and/or polysaccharide is, independently at each occurrence, optionally substituted hydroxyl saccharide, optionally substituted amino saccharide, optionally substituted deoxy saccharide, optionally substituted sulfuric acid saccharide, optionally substituted hetero-element saccharide and/or its glycoside.

7. A compound according to the claim **1,** wherein:
R₇ is H or XbRa; Xb is, independently at each occurrence, optional substituent containing H, C, O or N element.

8. A compound according to the claim **1**, wherein:
When X₁ and/or X₂ is C = O, C = Rb-Ra, CHON, CHORb or CHRb, X₁ and X₂ are the same or different substituents; when Rb is C, N for P element, Ra is, independently at each occurrence, optionally substituted formation of olefin, alkane, halogenated hydrocarbon, alcohol, ether, oxime, hydrazone or substituted said groups.

9. A compound according to the claim **1**, wherein:
A bromo compound at 11-position is selected, independently at each occurrence, from:
gambogic acid, methyl gambogate, ethyl gambogate, gambogyl morpholine, gambogyl piperidine, gambogyl 4-methyl pyrazine, 9,10-dihydro-10-morpholinyl gambogic acid, ethyl-9,10-dihydro-10-morpholinyl gambogate, 9,10-dihydro-10-morpholinyl gambogyl, piperidine, methyl-9,10-dihydro-10-(nitromethane) gambogate, 9,10-dihydro-10-1-aminopiperidinyl gambogyl (1-amino) piperidine, benzyl alanine-9,10-dihydro-10-(benzyl-L-alaninyl) gambogyl, 9,10-dihydro-10-(N-methyl-1-naphthyl amino) gambogyl (N-methyl-1-naphthalene methylamine);
A compound introduced methyl amino at 11-position is selected, independently at each occurrence, from: gambogic acid, methyl gambogate, ethyl gambogale, gambogyl, morpholine, gambogyl piperidine, gambogyl-4-methyl-pyrazine, 9,10-dihydro-10-morpholinyl gambogic acid, methyl-9,10-dihydro-10-morpholinyl gambogate;
A compound introduced benzoyloxy at 11-position is selected, independently at each occurrence, from: gambogic acid, methyl gambogate, ethyl gambogate, gambogyl morpholine, gambogyl piperidine, gambogyl (4-methyl pyrazine), 9,10-dihydro-10-morpholinyl gambogic acid, methyl-9,10-dihydro-10-morpholinyl gambogate;
A compound introduced methyl sulfonyloxy at 11-position is selected, independently at each occurrence, from: gambogic acid, methyl gambogate, ethyl gambogate, gambogyl morpholine, gambogyl piperidine, gambogyl (4-methyl pyrazine);

10. A compound according to the claim **1**, wherein:
A compound substituted by D-glycosyloxy and L-glycosyloxy at 6-position is selected, independently al each occurrence, from: gambogic acid, methyl gambogale, ethyl gambogate, n-butyl gambogale, gambogyl n-butylamine, gambogyl morpholine, gambogyl piperidine, gambogyl cytosine, gambogyl dipentylamine, gambogyl(4'-methylpyrazine),gambogyl (4'-β-amino-4'-deoxy-4'-demethyl podophyllotoxin), diethylene glycol gambogate, triethylene glycol gambogate, o-chloropheyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholimyl gambogate, 9,10-dihydro-10-morpholinyl gambogylpiperidien, 9,10-dihydro-10-morpholinyl gambogyl (benzyl-L-alaninate), 9,10-dihydro-10- morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morphline, 9,10-dihydro-10-morpholinyl-11-bromo gambogyl piperidine, 9,10-dihydro-10-morpholinyl-11-bromo gambogyl, (4'-methyl pyrazine), methyl-9,10-dihydro-10-morpholinyl-11-acetoxy gambogate, methyl-9,10-dihydro-10-morpholinyl-11-benzoyl gambogate, 9,10-dihydro-10-morpholinyl-11-methy sulfonyloxygambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-melhyl pyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'- methyl pyrazine), ethyl-9,10-dihydro-10-piperidinyl gambogate, 9,10-dihydro-10-piperidinyl gambogylpiperidine, 9,10-dihydro-10-piperidinyl gambogyl (4'-methyl piperazine), methyl-9,10-dihydro-10-(nitromethane) gambogate, ethyl-9,10-dihydro-10-nitro-methylgambogate, 9,10-dihydro-10-(1'-mninopiperidinyl) gambogyl (1'-amino) piperidine, 9,10-dihydro-10-(N-methyl-1'-naphthyl amino) gambogyl (N-methyl-1'-naphthalene(methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl, piperidine, methyl-9,10-dihydro-10-benzyloxy gambogate, 9,10-dihydro-10-pyrrolidinyl gambogic acid, ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl, morpholine, 11-methylsulfonyloxy gambogyl piperidine, 11- methysulfonyloxy gambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxygambogyl(4'-methylpyrazime);

11. A compound according to the claim 1, wherein:
A compound substituted by D-allosyloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'-demethyl podophyllotoxin), diethylene glycol gambogate, triethylene glycol gambogate, o-chlorophenyl alcohol gambogate, diphenyl methyl gambogate, 9,10-dihydro10-morpholinyl gambogic acid, methyl-9,10-dihydro-10-morpholinyl gambogate, ethyl-9,10-dihydro-10-morpholinylgambogate, 9,10-dihydro-10-morpholinyl gambogyl, piperidine, 9,10-dihydro-10-morpholinyl gambogyl(benzyl-L-alaninate), 9,10-dihydro-10-morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10-morpholinyl-11-bromo gambogyl (4'-methyl pyrazine), methyl-9,10-dihydro-10-morpholinyl-11-acetoxy gambogate, ethyl-9,10-dihydro-10-morpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methylsulfonyloxy gambogylpiperidine, 9,10-dihydro-10-morpholinyl-11-methyl sulfonyloxy gambogyl (4'-methylpyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl, piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinyl gambogyl piperidine, 9,10-dihydro-10-piperidinyl gambogyl (4'-methyl piperazine), methyl-9,10-dihydro-10-(nitromethane) gambogate, ethyl-9,10-dihydro-10-nitromethyl gambogate, 9,10-dihydro-10-(1'-aminopiperidinyl) gambogyl (1'-amino) piperidine, 9,10-dihydro-10-(N-methyl-1'-naphthyl amino) gambogyl (N-methyl-1'-naphthalene methylamine), 9,10-dihydro-10-(benzyl-L-alaninyl) gambogyl, (benzyl-L-alaninale), ethyl-9,10-dihydro-10-(4'-methyl piperazine triazinyl) gambogate, 9,10-dihydro-10-(4'-methyl piperazinyl) gambogyl piperidine, 9,10-dihydro-10-(4'-methyl piperazinyl) gambogyl (4'-methylpiperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxygambogyl(4'-methylpyrazime);

12. A compound according to the claim 1, wherein:
A compound substituted by 4-O-D-glucosylbenzoyloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'-demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, ethyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpholinyl gambogyl(benzyl-L-alaninate), 9,10-di hydro-10-morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl- 11-bromo gambogyl morpholine, 9,10-dihydro-10-morpholinyl-11-bromo gambogyl piperidine, 9,10-dihydro-10-morpholinyl-11-bromo gambogyl (4'-methyl pyrazine), ethyl-9,10-dihydro-10-morpholinyl-11-acetoxy gambogate, ethyl-9,10-dihy dro-10-morpliolinyl-11-benzoylgambogate, 9,10-dihydro-10-morpholinyl-11-methsulfonyloxygambogic acid, ethyl-9,10-dihydro-10-morpholinyl-11-methylsulfonyloxy gambogate, ethyl-9,10-dihydro-10-morpholinyl-11-methylsulfonyloxy gambogate, 9,10-dihydro-10-morpholinyl-11-methylsulfonyloxy gambogyl morpholine, 9,10-dihydro-10-morpholinyl-11-methylsulfonyloxy gambogylpipendine, 9,10-dihydro-10-morpholinyl-11-methylsulfonyl oxy gambogyl (4'-methylpyrazine), 9,10-dihydro-10-morpholinyl-11-trifluoromethyl sulfonyloxy gambogic acid, 9,10-dihydro-10-morpholinyl-11-methyltrifluoromethylsulfonyloxy gambogate, ethyl-9,10-dihydro-10-morpholinyl-11-trifluoromethylsulfonyloxy gambogate, 9,10-dihydro-10-morpholinyl-11-trifluoromethyl sulfonyloxygambogylmorpholine, 9,10-dihydro-10-morpholinyl-11-trifluoromethyl sulfonyloxy gambogyl piperidine, 9,10-dihydro-10-morpholinyl-11-trilfuoromethyl sulfonyloxy gambogyl (4'-methylpyrazine), ethyl-9,10-dihydro-10-piperidinyl gambogate, 9,10-dihydro-10-piperidinyl gambogyl, piperidine, 9,10-dihydro-10-piperidinyl gambogyl, (4'-methyl piperazine), methyl-9,10-dihydro-10-(nitromethane) gambogate, ethyl-9,10-dihydro-10-nitromethyl gambogate, 9,10-dihydro-10-(1'-aminopiperidinyl) gambogyl (1'-amino) piperidine, 9,10-dihydro-10-(N-methyl-1'-naphthyl amino) gambogyl, (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid, ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethyl sulfonyloxygambogyl(4'-methylpyrazime);
A compound substituted by 4-O-D-glucosylbenzoyl-L-alanyl oxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl, cytosine, gambogyl, dipentylamine, gambogyl, (4'-methyl pyrazine), gambogyl, (4'-β-amino-4'-deoxy-4'-demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpliolinyl gambogyl (benzyl-L-alaninale), 9,10-dihydro-10-morpholinyl gambogyl morpholine, 9,10-dihydro-10-morpholinyl gambogyl, (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10-morpholinyl-11-bromo gambogyl (4'-methyl pyrazine), ethyl-9,10-dihydro-10-morpholinyl- 11-acetoxy gambogate, ethyl-9,10-dihydro-10-moipliolinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-l1-methylsulfonyloxygambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methylpyrazine), 9,10-dihydro-10-moipholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluormethyl sulfonyloxy gambogate, 9,10-dihydro-10-morpholinyl-11-trifluoromethyl sulfonyloxy gambogyl morpholine, 9,10-dihydro-10-morpliolinyl-11-trifuoromethyl sulfonyloxy gambogyl piperidine, 9,10-dihydro-10-morplnolinyl-11-trifluoromethyl sulfonyloxy gambogyl (4'-methylpyrazine), ethyl-9,10-dihydro-10-piperidinyl gambogate, 9,10-dihydro-10-piperidinylgambogyl piperidine, 9,10-dihydro-10-pipetidinyl gambogyl, (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl, piperidine, 9,10-dihydro-10-(4'-melhyl piperazinyl) gambogyl (4'-methyl piperazine), 9,10-dihydro-10-(4'-methyl piperazinyl) gambogyl morpholine, 9,10-dithydro-10-methoxy gambogyl piperidine, methyl-9,10-dihydro-10-benzyloxy gambogate, 9,10-dihydro-10-pyrrolidinyl gambogic acid , ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromo gambogyl piperidine, 11-bromogambogyl (4'-methylpyrazine), methyl-11-acetoxy gambogate, methyl-11-benzoyloxy gambogate, 11-methyl sulfonyloxy gambogic acid, elhyl-l1-melhyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine),11-trifluoromethylsulfonyloxy gambogic acid, methyltrifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogyl piperidine and/or 11-trifluoromethylsulfonyloxy gambogyl(4'-methylpyrazime);
A compound substituted by 4-O-D-allosyl benzoyl-L-alanyl oxy at 6-position is selected,
independently at each occurrence, from: gambogic acid, methyl gambogate, ethyl gambogale, n-bulyl gambogate, gambogyl n-butylamine, gambogyl morpholine, gambogyl piperidine, gambogyl cytosine, gambogyl, dipentylamine, gambogyl, (4'-methyl pyrazine), gambogyl (4°-β-amino-4'-deoxy-4'-demethyl podophylloloxin), diethylene glycol gambogate, triethylene glycol gambogate, o-chlorophenyl alcohol gambogate, diphenyl methyl gambogate, 9,10-dihydro-10-morpholinyl gambogic acid, ethyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpholinyl gambogyl (benzyl-L-alaninate), 9,10-dihydro-10- morpholinyl gambogyl morpholine, 9,10-dihydro-10-morpholinyl gambogyl, (4'-methyl piperazine), methyl-9,10-dihydro-10-morpholinyl-11-bromogambogate, 9,10-dihydro-10-moipholinyl-11-bromo gambogyl morpholine, 9,10-dihydro-10-moipholinyl-11-bromo gambogyl piperidine, 9,10-dihydro-10-mor pholinyl-11-bromo gambogyl (4'-methyl pyrazine), ethyl-9,10-dihydro-10- morpholinyl-11-acetoxy gambogate, ethyl-9,10-dihydro-10-ntorpholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methylpyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogale, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methyl pyrazine), ethyl-9,10-dihydro-10-piperidinyl gambogate, 9,10-dihydro-10-piperidinyl gambogyl, piperidine, 9,10-dihydro-10-piperidinyl gambogyl, (4'-methyl piperazine), methyl-9,10-dihydro-10-(nitromethane) gambogate, ethyl-9,10-dihydro-10-nitro-methyl gambogate, 9,10-dihydro-10-(1'-minopiperidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl)gambogyl piperidine, 9,10-dihydro-10-(4'-methyl piperazinyl) gambogyl, (4'-methylpiperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid, ethyl-9-bromo-10-H-10-hydroxylgambogate, ethyl-9,10-epoxy gambogate, 11-bromogambogic acid, ethyl-11-bromogambogate, ethyl-11-bromogambogate, 11-bromogambogyl morpholine, 11-bromo gambogylpiperidine, 11-bromogambogyl (4'-methylpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogylmorpholine, 11-methylsulfonyloxy gambogyl piperidine, 11- methylsulfonyloxygambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate,11-trifluoromethyl sulfonyloxy gambogyl morpholine, 11-trifluoromethyl sulfonyloxy gambogylpiperidine and/or 11-trifluoromethyl sulfonyloxy gambogyl (4'-methylphrazime);

13. A compound according to the claim **1**, wherein:
A compound substituted by phosphoryloxy at 6-position is selected, independently at each occurrence, from: gambogic acid, methyl gambogate, ethyl gambogate, n-butyl gambogate, gambogyl n-butylamine, gambogyl morpholine, gambogyl piperidine, gambogyl cytosine, gambogyl dipentylamine, gambogyl (4'-methyl pyrazine), gambogyl (4'-β-amino-4'-deoxy-4'-demethyl podophyllotoxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl, piperidine, 9,10-dihydro-10-morpholinyl gambogyl, (benzyl-L-alaninate), 9,10-dihydro-10-morpholinyl gambogyl morpholine, 9,10-dihydro-10-morpholinyl gambogyl (4'-methyl piperazine), methyl-9,10-dihydro-10-morpholinyl-11-bromogambogate, 9,10-dihydro-10-morpholinyl-11-bromo gambogyl morpholine, 9,10-dihydro-10-morpholinyl-11-bromo gambogyl piperidine, 9,10-dihydro-10-morpholinyl-10-bromo gambogyl (4'-methyl pyrazine), methyl-9,10-dihydro-10-morpholinyl-11-acetoxy gambogate, methyl-9,10-dihydro-10-morpholinyl-11-benzoyl gambogate, 9,10-dihydro-10-morpholinyl-11-methysulfonyloxy gambogic acid, ethyl-9,10-dihydro-10-morpholinyl-11-methyl sulfonyloxy gambogate, ethyl-9,10-dihydro-10-morpholinyl-11-methyl sulfonyloxygambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methyl pyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trilluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methylpyrazine), ethyl-9,10-dihydro-10-piperidinyl gambogate, 9,10-dihydro-10-piperidinylgambogyl piperidine, 9,10-dihydro-10-pipetidinyl gambogyl, (4'-methyl piperazine), methyl-9,10-dihydro-10-(nitromethane) gambogate, ethyl-9,10-dihydro-10-nitro-methyl gambogate, 9,10-dihydro-10-(1'-aminopiperidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid, ethyl-9-bromo-10-11-10-hydroxyl gambogate, ethyl-9,10-epoxy gambogate, 11-bromogambogic acid, ethyl-11-bromogambogate, ethyl-11-bomogambogate, 11-bromogambogyl morpholine, 11-bromo gambogyl, piperidine, 11-bromogambogyl (4'-methylpyrazine), methyl-11-acetoxy gambogate,
methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine), 11-trifluoromethylsulfonyloxy gambogic acid, methyl-11-trifluoromethy sulfonyloxy gambogale, ethyl-11-trifluoromethyl sulfonyloxy gambogate,
11-trifluoromethyl sulfonyloxy gambogyl morpholine, 11-trifluoromethyl sulfonyloxy
gambogylpiperidine and/or 11-trifluoromethyl sulfonyloxy gambogyl(4'-netgtkotrazune);
A compound substituted by triphosphoryloxy at 6-position is selected, independently at each
occurrence, from: gambogic acid, methyl gambogate, ethyl gambogate, n-butyl gambogate,
gambogyl n-butylamine, gambogyl morpholine, gambogyl piperidine, gambogyl cytosine,
gambogyl dipentylamine, gambogyl (4'-methyl pyrazine), gambogyl (4'-β-amino-4'-deoxy-4'-demethyl podophylloloxin), diethylene glycol gambogate, triethylene glycol gambogate, o-chlorophenyl alcohol gambogate, diphenyl methyl gambogate, 9,10-dihydro-10-morpholinyl gambogic acid, methyl-9,10-dihydro-10-morpholinyl gambogate, ethyl-9,10-dihydro-10-morpholinyl gambogate, 9,10-dihydro-10-morpholinyl gambogyl piperidine, 9,10-dihydro-10-morpholinyl gambogyl (benzyl-L-alaninate), 9,10-dihydro-10-morpholinyl gambogyl morpholine, 9,10-dihydro-10-morpholinyl gambogyl (4'-methyl pipeiazine), methyl-9,10-dihydro-10-morpholinyl-11-bromogambogate, 9,10-dihydro-10-morpholinyl-11-bromo gambogyl, morpholine, 9,10-dihydro-10-morpholinyl-11-biomo gambogyl piperidine, 9,10-dihydro-10-morpholinyl-11-bromo gamboyl (4'-methyl pyrazine), methyl-9,10-dihydro-10-morpholinyl-11-actoxy gambogate, methyl-9,10-dihydio-10-moipholinyl-11-benzoyl gambogate, 9,10-dihydro-10-morpholinyl-11-methy sulfonyloxy gambogic acid, ethyl-9,10-dihydro-10-morpholinyl-11-methyl sulfonyloxy gambogate, ethyl-9-10-dihydro-10-morpholinyl-11-methyl sulfonyloxy gambogate, 9,10-dihydro-10-morpholinyl-11-methyl sulfonyloxy gambogyl morpholine, 9,10-dihydro-10-morpholinyl-11-methyl sulfonyloxy gambogyl piperidine, 9,10-dihydro-10-morpholinyl-11-methyl sulfonyloxy gambogyl (4'-methyl pyrazine), 9,10-dihydro-10-morpholinyl-11-trifluoromethyl sulfonyloxy gambogic acid, 9,10-dihydro-10-morpholinyl-11-methyl trifluoromethyl sulfonyloxy gambogate, ethyl-9,10-dihydro-10-morpholinyl-11-trifluoromethyl sulfonyloxy gambogate, 9,10-dihydro-10-morpholinyl-11-trifluoromethyl sulfonyloxy gambogyl morpholine, 9,10-dihydro-10-morpholinyl-11-trifluoromethyl sulfonyloxy gambogyl piperidine, 9,10-dihydro-10-morpholinyl-11-trifluoromethyl sulfonyloxy gambogyl (4'-methyl pyrazine), ethyl-9,10-dihydro-10-piperidinyl gambogale, 9,10-dihydro-10-piperidinyl gambogyl piperidine, 9,10-dihydro-10-piperidinyl gambogyl, (4'-methyl piperazine), methyl-9,10-dihydro-10-(nitromethane)gambogate, ethyl-9,10-dihydro-10-nitromethylgambogate, 9,10-dihydro-10-(1'-aminopipelidinyl) gambogyl (1'-amino) piperidine, 9,10-dihydro-10-(N-methyl-1'- naphthyl amino) gambogyl (N-methyl-1'-naphthalene methylamine), 9,10-dihydro-10-(benzyl-L-alaninyl) gambogyl (benzyl-L-alaninate), ethyl-9,10-dihydro-10-(4'-methylpiperazine triazinyl) gambogate, 9,10-dihydro-10-(4'-methyl piperazinyl) gambogyl piperidine, 9,10-dihydro-10-(4'-methyl piperazinyl) gambogyl (4'-methyl piperazine), 9,10-dihydro-10-(4'-methyl piperazinyl) gambogyl morpholine, 9,10-dihydro-10-methoxy gambogyl piperidine, methyl-9,10-dihydro-10-benzyloxy gambogate, 9,10-dihydro-10-pyrrolidinyl gambogic acid, ethyl-9-bromo-10-H-10-hydroxyl gambogate, ethyl-9,10-epoxy gambogate, 11-bromogambogic acid, ethyl-11-bromogambogate, ethyl-11-bromogambogate, 11-bromogambogyl morpholine, 11-bromogambogyl pipendine, 11-bromogambogyl (4'-methpyrazine), methyl-11-acetoxy gambogate, methyl-11-benzoyloxy gambogate, 11-methyl sulfonyloxy gambogic acid, ethyl-11-methyl sulfonyloxy gambogate, ethyl-11-methyl sulfonyloxy gambogate, 11-methylsulfonyloxy gambogyl morpholine, 11-methylsulfonyloxy gambogyl piperidine, 11-methylsulfonyloxy gambogyl (4'-methylpyrazine), 11-trifluoromethylsulfonyloxy gambogic acid, methyl-11-trifluoromethy sulfonyloxy gambogate, ethyl-11-trifluoromethyl sulfonyloxy gambogate, 11-trifluoromethyl sulfonyloxy gambogyl morpholine, 11-trifluoromethyl sulfonyloxy gambogylpiperidine and/or 11-trifluoromethyl sulfonyloxy gamnbogyl(4'-methylpyrazime);
A compound substituted at 30-position is: gambogyl dipentylamine, diethylene glycol gambo- gate, triethylene glycol gambogate, o-chlorophenyl alcohol gambogate, gambogyl (4'-me- thylpyrazine), gambogyl (4'-β-amino-4'-deoxy-4 'demethyl podophyllotoxin), diphenylme-thyl gambogate, gambogyl (benzyl-L-alaninate), N-(2',6'-dioxopipeidin-3-yl) gambogyl, N-(2',6'-dioxopiperidin-3'-yl)-6-D-gluconyl gambogyl, N-(2',6'-dioxopiperidin-3-yl)-6- (4'-O-D-glucosyl)benzoyl acyl-L-alanine gambogyl, N-(2',6'-dioxopiperid-in-3'-yl)-6-O- phosphate gambogyl, N-(2',6'-dioxo-piperidin-3'-yl)-6-O-triphosphate gambogyl, gambogyl-4'β-amino-4'-deoxy-4'-demethyl podophyllotoxin), N-(2',6'-dioxopiperidin-3'-yl)-6-D-alorglycosyl gambogyl and / or N-(2',6'-dioxopiperidin-3'-yl)-6-(4'-O-D-allosyl) benzoylarcyl-L-arlamine gambogyl;
A compound substituted by 2,2-dimethyl hexahydropyano[3,2-d][1,3]dioxine-6,7,8-triol or 4-(7',8'-dihydroxyl-2',2-dimethylhexahydropyrano[3,2-d][1,3]dioxin-6'-yloxy)benzoyloxy at 6-position is: 6-(7,8-dihydioxyl-2,2-dimethyl hexahydropyrano[3,2-d][1,3]dioxin6-yloxy) gambogic acid, 6-(4'-(7",8"-dihydroxyl-2',2"-dimethyl hexahydropyrano [3.2-d][1,3] dioxin6"-yioxy)benzoyloxy) gambogic acid and its glycoside derivatives or analogs.

14. A compound according to the claim **1,** wherein:
A process for the manufacture or a compound or formula **I** comprises:
(a) for the preparation of compounds of formula and salts thereof in which the reaction of a compound of formula **I** in X₁, X₂, R₁, R₂, R₃, R₄, R₅, R₆, R₇, R₈, R₉, R₁₀, R₁₁ or/and R₁₂ is a glycosyl, ntulti-hydroxyl or substituent-oxy with a bond of C-C, C-O, C-S, C-N or C-P under catalysis at -78 °C to 90 °C;
(b) for the preparation of compounds of formula **I** and suits thereof in which the reaction of a compound of formula I in X₁, X₂, R₁, R₂, R₃, R₁, R₅, R₆ R₇, R₈, R₉, R₁₀, R₁₁ or/and R₁₂ is an amino acid, acyloxy, phosphoric acid, phosphoryloxy, sull'onyloxy, alkoxy, aryloxy, heterocyclicoxy, hydrocarbons, alicyclic, or heterocyclic aryl containing oxygen, sulfur, nitrogen or phosphorus element with a bond of C-C, C-O, C-S, C-N or C-P under catalysis at -78 °C to 90 °C;
(c) for the preparation or compound, of formula I and salts thereof in which the reaction or a compound of formula I in X₁, X₂, R₁, R₂, R₃, R₁, R₅, R₆, R₇, R₄, R₉, R₁₀, R₁₁ or/and R₁₂ is a carboxyl group containing glucosyl, multi-hydroxyl, phosphate, amino acid, alkane, aryl, alicyclic, heterocyclic, or heteroarylcyclic with a bond of C-C, C-O, C-S, C-N or C-P under catalysis at -78°C to 90°C;
(d) for the preparation or compound, of formula **I** and salts thereof in which the reaction of a compound of formula I in X₁, X₂, R₁, R₂, R₃, R₄, R₅, R₆, R₇. R₈, R₉, R₁₀, R₁₁ or/and R₁₂ is a glucosyl, multi-hydroxyl, amino acid or substituent oxy modified by acylation, halogenation with a bond of C-C, C-O, C-S, C-N or C-P under catalysis at -78 °C to 90 °C;
(e) for the preparation of compounds of formula **I** and salts thereof in which the reaction of a compound of formula **I** in X₁, X₂, R₁, R₂, R₃, R₁, R₅, R₆, R_{7,} R₈, R₉, R₁₀, R₁₁ or/and R₁₂ is a carboxyl group containing glucosyl, multi-hydroxyl, phosphate, amino acid, alkane, aryl, alicyclic, heterocyclic or heteroarylcyclic with a bond of C-C, C-O, C-S, C-N or C-P under catalysis at -78°C to 90 °C;
(f) for the preparation of compounds of formula and salts thereof in which the reaction of to compound of formula **I** in X₁, X₂, R₁, R₂, R₃, R₄, R₅, R₆, R₇, R₈, R₉, R₁₀, R₁₁ or/and R₁₂ is an electrophilic substituent at 9-position, nucleophilic substituent at 10-position accompanied by 1,4 addition reaction with a bond of C-C, C-O, C-S, C-N or C-P under catalysis at -78 °C to 90°C;
(g) for the preparation of compounds of formula **I** and salts thereof in which the reaction of a compound of formula **I** in X₁, X₂, R₁, R₂, R₃, R₁, R₅ R₆, R_{7,} R₈ R₉, R₁₀, R₁₁ or/and R₁₂ is a substituent at allyl of 11-position, 26-position, 31-position or 36-position modified by a bond of C-C, C-O, C-S, C-N or C-P under catalysis at -78 °C: to 90 °C;
(h) for the preparation of compounds of formula **I** and salts thereof in which the reaction of a compound of formula **I** X₁, X₂, R₁, R₂, R₃, R₁, R₅, R₆, R₇, R₈, R₉, R₁₀, R₁₁ or/and R₁₂ is a phosphate or multi-phosphate with C-P bond under catalysis at -78 °C to 90 °C.

15. A compound according to claim **9**, wherein:
The compound is selected from the exemplified examples or stereoisomers, tautomers, pharmaceutically acceptable salts, prodrug or solvates thereof in association wit a pharmaceutically acceptable excipient or carrier.

16. A method for treating a cancer disorder, comprising: administration to a patient in need thereof a therapeutically effected amount of a compound of the gambogic acid glycoside derivatives and analogs of formula I, or stereoisomers, tautomers, pharmaceutically acceptable salts, prodrug or solvates thereof.

17. A method according to the claim **1,** wherein:
A compound for treating, preventing or slowing the progression of neoplasia and cancer, and infection diseases by virus, bacterial or fungi.

18. A compound according to the claim **1,** wherein:
A method for treating broad-spectrum bacterial and fungal diseases, including bacterial infections and fungal infections of the drug application, which comprises administration together with at least one known chemotherapeutic agent selected from the group consisting of antibacterial and antifungal drugs to a patient in need of such treatment.

19. A method according to the claim **1**, wherein:
A cancer is selected from the group consisting of Hodgkin's disease, non-Hodgkin's, lymphoma, acute and chronic lymphocytic leukemias, multiple myeloma, neuroblastoma, breast carcinoma, ovarian carcinoma, lung carcinoma, Wilms' tumor, cervical carcinoma, testicular carcinoma, soft-tissue sarcoma, chronic lymphocytic leukemia, primary macroglobulinemia, bladder carcinoma, chronic granulocytic leukemia, primary brain carcinoma, malignant melanoma, small-cell lung carcinoma, stomach carcinoma, colon carcinoma, malignant pancreatic insulinoma, malignant carcinoid carcinoma, choriocarcinoma, mycosis fungoides, head and neck carcinoma, osteogentic sarcoma, pancreatic carcinoma, acute granulocytic leukemia, hairy cell leukemia, neuroblastoma, rhabdomyosarcoma, Kaposi's sarcoma, genitourinary carcinoma, thyroid carcinoma, esophageal carcinoma, malignant hypercalcemia, cervical hyperplasia, renal cell carcinoma, endometrial carcinoma, polycythemia vera, essential thrombocytosis, adrenal cortical carcinoma, skin cancer and prostatic carcinoma.

20. The method according to claims **1,** wherein said compounds is administered together with at least one known cancer, chemotherapeutic agent selected from the group consisting of busulfan, cisplatin, mitomycin C, carboplatin, colchichine, vinblastine, paclitaxel, docetaxel, camptochecin, topotecan, doxorubicin, etoposide, 5-azacytidine, 5-fluorouracil, methotrexate, 5-fluoro-2'-deoxy-uridine, ara-C, hydroxylurea, thioguanine, melphalan, chlorambucil, cyclo phosamide, ifosfamide, vincristine, mitoguazone, epirubicin, aclaruhicin, bleomycin, mitoxantrone, elliptinium, fludarabine, octreotide, retinoic acid, tamoxifen, Herceptin®, Rituxan®, arsenic trioxide, gamcitabine, doxazosin,terazosin tamsulosin, CB-64D, CB-184, haloperidol, lovastatin, simvastatin, pravastatin, Huvastatin, atorvastatin, cerivastatin, amprenavir, abavavir, indinavir, nelfinavir, tipranavir, ritonavir, saquinavir, bexarotene, tretinoin, 13-cis-retinoic acid, 9-cis-retinoic acid, difluoromethylornithine, fenretinide, N-4-carboxyphenyl retinamide, lactacystin, genistein, flavopiridol, roscovitine, olomoucine, celecoxib, valecoxib, rofecoxib and alanosine, CGP-73547,CGP-61755,DMP-450, ABT-378. AG1776, BMS232,632. ILX23-7553, MG-132, PS341, Gleevec®, ZD1839,SH268, CEP2563, SU6668, SU11248, EMD121974, R115777, SC1166336, L-778,123, BAL9611,TAN-1813 or/ and UCN-01.

21. A method for treating cancer, comprising: administration to a compound of the claim **1** aNd claim **9,** in The range of 0.001 mg/kg-250 mg/kg, a pharmaceutically acceptable salt or prodrug from thereof.

22. A compound according to the claim **1** and **14,** wherein:
The administration may be by oral route, parenteral, subcutaneous, intravenous, intramuscular, intra-peritoneal, transdermal, buccal, intrathecal, intracranial, intranasal or topical routes.
